# EUROPEAN PATENT APPLICATION

(11) **EP 4 400 514 A1**
(43) Date of publication of application: **17.07.2024**
(21) Application number: 22867793.6
(22) Date of filing: 07.09.2022
(51) Int. Cl.: C07K 16/28, C07K 16/46, C12N 15/13, C12N 15/63, C12P 21/08

(54) **BISPECIFIC ANTIBODY COMPRISING A HETERODIMER BASED ON MHC PROTEINS**

(30) Priority: 08.09.2021 RU 2021126369
(71) Applicant: Joint Stock Company "Biocad", Saint Petersburg, 198515 (RU)
(72) Inventor: LEGOTSKII, Sergei Aleksandrovich, Moscow, 111675 (RU); NAZARENKO, Olga Viktorovna, Leningradskaya obl., 188663 (RU); DANILOV, Maksim Andreevich, Saint Petersburg, 198516 (RU); BARANOVSKAIA, Marianna Dmitrievna, Saint Petersburg, 198510 (RU); POLIAKOV, Dmitrii Nikolaevich, Saint Petersburg, 198510 (RU); VALIAKHMETOVA, Elvira Raisovna, Bashkortostan, 453300 (RU); TOPORKOVA, Kseniia Aleksandrovna, Saint Petersburg, 198335 (RU); MATIUKHINA, Natalia Mikhailovna, Saint Petersburg, 198328 (RU); KRAT, Sergei Mikhailovich, Saint Petersburg, 198206 (RU); GURINA, Natalia Nikolaevna, Saint Petersburg, 192212 (RU); IAKOVLEV, Pavel Andreevich, Saint Petersburg, 190068 (RU); MOROZOV, Dmitry Valentinovich, Saint Petersburg, 190000 (RU)
(74) Representative: Meissner Bolte Partnerschaft mbB
(86) International application number: PCT/RU2022/050281
(87) International publication number: WO 2023/038548

(57) **Abstract**

The present invention relates to the field of biotechnology, specifically to bivalent bispecific chimeric antibodies that include a heterodimer based on the membrane-proximal domains of MHC (major histocompatibility complex) or MHC-like proteins (CD1 (cluster of differentiation 1) or HFE (hemochromatosis protein)), as well as to a technique for producing said bispecific antibodies. The invention further relates to a nucleic acid encoding said bispecific antibody, an expression vector, a host cell for producing said bivalent chimeric bispecific antibody and to a method for producing said cell.

## Description

### Field of the invention

The present invention relates to the field of biotechnology, specifically to bivalent bispecific chimeric antibodies that include a heterodimer based on the membrane-proximal domains of MHC (major histocompatibility complex) or MHC-like proteins (CD1 (cluster of differentiation 1) or HFE (hemochromatosis protein)), as well as to a technique for producing said bispecific antibodies. The invention further relates to a nucleic acid encoding said bispecific antibody, an expression vector, a host cell for producing said bivalent chimeric bispecific antibody and to a method for producing said cell.

### Background of the invention

Monoclonal antibodies in the form of chimeric, humanized or fully human molecules have proven to be useful as effective medicine for treating a number of disorders and diseases.

Naturally occurring human antibody molecules consist of two heavy chain homodimers, each of which forms a heterodimer in partnership with two identical light chain molecules. Conventional monoclonal antibodies in the form of whole molecules consist of bivalent ("two-armed") heterodimers of heavy and light chains.

Diseases are often caused as a result of several pathologies and are accompanied by many concomitant diseases. Bispecific antibodies are capable of binding and thereby neutralizing two or more different antigens per antibody molecule. The potential for a significant improvement in the therapeutic properties (and value) of medicinal products as compared to monoclonal antibodies has made bispecific antibodies an active area of research. Over the past twenty years, the literature has described many solutions regarding engineered versions of bispecific antibodies, as described in Brinkmann, U and RE Kontermann, 2017, The Making of Bispecific Antibodies, MAbs; 209 Feb/Mar; 9(2): 182-212, doi: 10.1080/19420862.2016.1268307.

As mentioned above, there are many approaches to create molecules with combined antigen-binding domains, that is, with antigen-binding domains that differ from each other. However each of these methods has its disadvantages.

Cross-linking by chemical methods is a time-consuming process, since homodimers and other undesirable by-products should be removed from the corresponding portions. In addition, the steps of chemical modification may alter the integrity of proteins, thus impairing stability thereof. Thus, the above method is typically ineffective and may lead to the loss of antibody activity.

A method based on cell fusion (for example, production of hybridomas) is an arbitrary assembly of two heavy and two light chains, resulting in 10 combinations of antibodies. Target heteromultimeric antibodies are only a small part of the antibodies produced in this fashion. Isolation of target heteromultimeric proteins significantly reduces the yield of product and increases production costs.

Recombinant DNA techniques are employed to create various heteromultimeric antibodies, for example, single-chain Fv fragments, diabodies, etc. that are free of an Fc fragment. The main disadvantage of this type of an antibody molecule is an absent Fc domain, which results in antibody failure to trigger an effector function (such as, for example, complement activation, binding to an Fc receptor, etc.). Thus, there is a need for a bispecific antibody comprising a functional Fc domain.

Recombinant DNA techniques are further employed to design bispecific antibodies using the Knob-into-Holes technology. See international applications WO9627011 and WO9850431, as well as Merchant AM ET ALL., An efficient route to human bispecific IgG, Nat Biotechnol. 1998 Jul;16(7):677-81. One factor limiting the use of the above method is the fact that the light chains of the two initial antibodies should be identical to prevent mispairing and formation of undesirable and/or inactive molecules when expressed in a single cell.

The purity of bispecific antibody product depends on two factors as follows:
a) heterodimeric assembly of two distinct heavy chains co-expressed in the cell, and
b) correct pairing of two distinct light chains to the corresponding heavy chains.

The "Knob-into-Holes" technology to design bispecific antibodies solves the problem of correct heterodimeric assembly of two distinct heavy chains co-expressed in the cell. However, the use of the Knob-into-Holes technology to design bispecific antibodies makes it possible to achieve only about 25% yield of a properly assembled bispecific product, as the problem of correct pairing of two distinct light chains to the corresponding heavy chains is still unresolved.

The problem of correct pairing of two distinct light chains to the corresponding heavy chains is solved in various fashions:
1. Use of an (identical) light chain in the first and second antigen-binding portions of antibody (Van Blarcom T ET AL., Productive common light chain libraries yield diverse panels of high affinity bispecific antibodies, MAbs. 2018 Feb/Mar;10(2):256-268. doi: 10.1080/19420862.2017.1406570).
   The disadvantage of the above solution is non-universality thereof, since it may be problematic to select a light chain suitable for the both valences. Furthermore, amino acid substitutions in the light chain to optimize the properties of the antigen-binding fragment may affect the both valences. Further, the binding of the antibody to the second antigen may be disrupted.
2. Use of single-chain formats, i.e. the formats wherein the light and heavy chains of the antigen-binding fragment specific for the first antigen are interconnected via a linker of several amino acids.
   This format has technological disadvantages, since it uses linkers either to fuse the antibody core (IgA, IgD, IgE, IgG or IgM) to a further binding protein (for example, scFv or scFab), or to fuse, for example, light and heavy variable domains (VH and VL) within scFv or a light chain (VL-CK(or CL)) to VH-CH1 within scFab. Linkers may cause problems in therapeutic settings. In fact, these foreign peptides can elicit an immune response against the linker itself or the junction region between the protein and the linker. Furthermore, the flexible nature of these peptides and the mobility thereof make them more prone to proteolytic cleavage, potentially leading to poor antibody stability, aggregation and increased immunogenicity.
3. Modification of CH1-CK domains in a bispecific antibody to allow altering the interaction interface in bispecific antibody expression techniques so as to exclude the incorrect association of light chains. For example, the international application WO2017059551 provides various amino acid substitutions in CH1 and/or CK that facilitate the preferred pairing between the desired heavy chain and the desired light chain.

Despite the above various bispecific antibody expression technologies, there is still a need in the art for improved purity of the bispecific antibody product, as well as for a scalable production solution for producing correctly assembled bispecific antibodies.

### Brief description of the invention

The developed, by the authors of the present invention, novel format of bispecific chimeric antibodies that include a heterodimer based on the membrane-proximal domains of MHC or MHC-like proteins, for example, CD1 (cluster of differentiation 1) or HFE (Human homeostatic iron regulator protein), as well as the technology for producing said bispecific chimeric antibodies surprizinlgy make it possible to produce a high yield of product with the correct heterodimeric assembly of two distinct heavy chains co-expressed in the cell and the correct pairing between two distinct light chains and the corresponding heavy chains.

The developed, by the authors of the present invention, novel format of bispecific chimeric antibodies that include a heterodimer based on membrane-proximal domains of MHC or MHC-like proteins, as well as the technology for producing said bispecific antibodies surprizingly make it possible to produce a correctly assembled bispecific chimeric antibody product with high purity.

Consequently, the above results reduce production costs and lead to a scalable production solution for producing correctly assembled bispecific antibodies.

In one aspect, the present invention relates to a bivalent bispecific chimeric antibody, wherein said antibody comprises:
a) a first light chain and a first heavy chain of antibody specifically binding to a first antigen;
   wherein the first light chain comprises a light chain variable domain and a light chain constant domain;
   wherein the first heavy chain comprises a heavy chain variable domain and heavy chain constant domains of antibody that include a first (CH1) heavy chain constant domain and an Fc fragment monomer comprising second (CH2) and third (CH3) heavy chain constant domains;
b) a second light chain and a second heavy chain of antibody specifically binding to a second antigen,
   wherein the second light chain comprises a light chain variable domain and a constant domain that is selected from the group:
   a first membrane-proximal domain of MHC (major histocompatibility complex) or
   a first membrane-proximal domain of MHC-like protein;
   wherein the second heavy chain comprises a heavy chain variable domain, a constant domain that is selected from the group:
      a second membrane-proximal domain of MHC (major histocompatibility complex) or
      a second membrane-proximal domain of MHC-like protein;
      and an Fc fragment monomer comprising second (CH2) and third (CH3) heavy chain constant domains;
      wherein the first membrane-proximal domain of MHC or MHC-like protein and the second membrane-proximal domain of MHC or MHC-like protein form a heterodimer therebetween, which is stabilized by a disulfide bond;
      wherein the CH3 domain of one heavy chain and the CH3 domain of another heavy chain contact each other with surfaces thereof, which are modified to form a bivalent bispecific chimeric antibody, said modifications in the heavy chain CH3 domains being substitutions to facilitate heterodimerization.

In some embodiments of the invention, the bivalent bispecific chimeric antibody includes a first membrane-proximal domain of MHC or MHC-like protein and a second membrane-proximal domain of MHC or MHC-like protein that form a heterodimer therebetween, which is stabilized by a disulfide bond by means of a mutation or mutations in the first and/or second constant domain to form an S-S bond (disulfide cysteine bridge) between the first and second membrane-proximal domains of MHC or MHC-like protein.

In some embodiments of the invention, the bivalent bispecific chimeric antibody includes a first membrane-proximal domain of MHC or MHC-like protein and a second membrane-proximal domain of MHC or MHC-like protein that form a heterodimer therebetween, which is stabilized by a disulfide bond by means of elongation of the first membrane-proximal domain of MHC or MHC-like protein by one or more (1 to 10) amino acids at the C-terminus and with terminal Cys at the C-terminus to form an S-S bond (disulfide bond, cysteine bridge) between the first membrane-proximal domain of MHC or MHC-like protein and the hinge.

In some embodiments of the invention, the bivalent bispecific chimeric antibody includes an elongation of the first membrane-proximal domain of MHC or MHC-like protein, the elongation is a sequence of three amino acids GSC.

In some embodiments of the invention, the bivalent bispecific chimeric antibody comprises:
a first light chain that comprises a light chain variable domain (VL1) and a light chain constant domain;
a first heavy chain that comprises a heavy chain variable domain (VH1) and heavy chain constant domains of antibody that include a first (CH1) heavy chain constant domain and an Fc fragment monomer comprising second (CH2) and third (CH3) heavy chain constant domains;
a second light chain that comprises a light chain variable domain (VL2) and a first membrane-proximal domain of MHC or a first membrane-proximal domain of MHC-like protein;
a second heavy chain that comprises a heavy chain variable domain (VH2), a second membrane-proximal domain of MHC or a second membrane-proximal domain of MHC-like protein and an Fc fragment monomer comprising second (CH2) and third (CH3) heavy chain constant domains;
wherein the first membrane-proximal domain of MHC or MHC-like protein and the second membrane-proximal domain of MHC or MHC-like protein form a heterodimer therebetween, which is stabilized by a disulfide bond by means of a mutation or mutations in the first and/or second constant domain to form an S-S bond (disulfide cysteine bridge) between the first and second membrane-proximal domains of MHC or MHC-like protein;
wherein between the first Fc variant and the second Fc variant in the CH3 domain there is formed a knob-into-hole structure.

In some embodiments of the invention, the bivalent bispecific chimeric antibody comprises:
a first light chain that comprises a light chain variable domain (VL1) and a light chain constant domain;
a first heavy chain that comprises a heavy chain variable domain (VH1) and heavy chain constant domains of antibody that include a first (CH1) heavy chain constant domain and an Fc fragment monomer comprising second (CH2) and third (CH3) heavy chain constant domains;
a second light chain that comprises a light chain variable domain (VL2) and a first membrane-proximal domain of MHC or a first membrane-proximal domain of MHC-like protein;
a second heavy chain that comprises a heavy chain variable domain (VH2), a second membrane-proximal domain of MHC or a second membrane-proximal domain of MHC-like protein and an Fc fragment monomer comprising second (CH2) and third (CH3) heavy chain constant domains;
wherein between the first Fc variant and the second Fc variant in the CH3 domain there is formed a knob-into-hole structure
wherein the first membrane-proximal domain of MHC or the first membrane-proximal domain of MHC-like protein further comprises an elongation of the first membrane-proximal domain of MHC or MHC-like protein by one or more (from 1 to 10) amino acids at the C-terminus with terminal Cys at the C-terminus to form an S-S bond (disulfide bond, cysteine bridge) between the first membrane-proximal domain of MHC or MHC-like protein and the hinge.

In some embodiments of the invention, the bivalent bispecific chimeric antibody comprises:
a first light chain that comprises a light chain variable domain (VL1) and a light chain constant domain;
a first heavy chain that comprises a heavy chain variable domain (VH1) and heavy chain constant domains of antibody that include a first (CH1) heavy chain constant domain and an Fc fragment monomer comprising second (CH2) and third (CH3) heavy chain constant domains;
a second light chain that comprises a light chain variable domain (VL2) and a first membrane-proximal domain of MHC or a first membrane-proximal domain of MHC-like protein;
a second heavy chain that comprises a heavy chain variable domain (VH2), a second membrane-proximal domain of MHC or a second membrane-proximal domain of MHC-like protein and an Fc fragment monomer comprising second (CH2) and third (CH3) heavy chain constant domains;
wherein between the first Fc variant and the second Fc variant in the CH3 domain there is formed a knob-into-hole structure
wherein the first membrane-proximal domain of MHC or the first membrane-proximal domain of MHC-like protein further comprises an elongation of the first membrane-proximal domain of MHC or MHC-like protein by the amino acid sequence GSC to form an S-S bond (disulfide bond, cysteine bridge) between the first membrane-proximal domain of MHC or MHC-like protein and the hinge.

In some embodiments of the invention, the bivalent bispecific chimeric antibody comprises a first membrane-proximal domain of MHC, which can be selected from a group comprising:
a first membrane-proximal domain of MHC class I (major histocompatibility complex class I),
a first membrane-proximal domain of MHC class II (major histocompatibility complex class II),
a modified variant of a first membrane-proximal domain of MHC class I or
a modified variant of a first membrane-proximal domain of MHC class II.

In some embodiments of the invention, the bivalent bispecific chimeric antibody comprises a second membrane-proximal domain of MHC, which can be selected from a group comprising:
a second membrane-proximal domain of MHC class I (major histocompatibility complex class I),
a second membrane-proximal domain of MHC class II (major histocompatibility complex class II),
a modified variant of a second membrane-proximal domain of MHC class I or
a modified variant of a second membrane-proximal domain of MHC class II.

In some embodiments of the invention, the bivalent bispecific chimeric antibody comprises a membrane-proximal domain of human MHC class I.

In some embodiments of the invention, the bivalent bispecific chimeric antibody comprises a membrane-proximal domain of human MHC class II.

In some embodiments of the invention, the bivalent bispecific chimeric antibody comprises a membrane-proximal domain of MHC class II, which is selected from the group: HLA-DM, HLA-DO, HLA-DP, HLA-DQ or HLA-DR.

In some embodiments of the invention, the bivalent bispecific chimeric antibody comprises a membrane-proximal domain of MHC class I, which is selected from the group: HLA-A, HLA-B, HLA-C, HLA-E, HLA-F or HLA-G.

In some embodiments of the invention, the bivalent bispecific chimeric antibody comprises a membrane-proximal domain of a human MHC-like protein.

In some embodiments of the invention, the bivalent bispecific chimeric antibody comprises a first membrane-proximal domain of MHC-like protein, which may be selected from a group comprising:
a first membrane-proximal domain of CD1 (cluster of differentiation 1),
a first membrane-proximal domain of HFE (hemochromatosis protein),
a modified variant of a first membrane-proximal domain of CD1 or
a modified variant of a first membrane-proximal domain of HFE.

In some embodiments of the invention, the bivalent bispecific chimeric antibody comprises a second membrane-proximal domain of MHC-like protein, which may be selected from a group comprising:
a second membrane-proximal domain of CD1 (cluster of differentiation 1),
a second membrane-proximal domain of HFE (hemochromatosis protein),
a modified variant of a second membrane-proximal domain of CD1 or
a modified variant of a second membrane-proximal domain of HFE.

In some embodiments of the invention, the bivalent bispecific chimeric antibody comprises a membrane-proximal domain of CD1, which is selected from the group: CD1a, CD1b, CD1c, CD1d or CD1e.

In some embodiments of the invention, the bivalent bispecific chimeric antibody includes a variable fragment of the second light chain (VL), which is separated from a first membrane-proximal domain of MHC or MHC-like protein by a linker of 1 to 25 amino acids long, and/or a variable fragment of the second heavy chain (VH), which is separated from a second membrane-proximal domain of MHC or MHC-like protein by a linker of 1 to 25 amino acids long.

In some embodiments of the invention, the bivalent bispecific chimeric antibody comprises:
a) the CH3 domain of one heavy chain, which is modified so that on the surface of the CH3 domain of one heavy chain contacting the surface of the CH3 domain of another heavy chain in the bivalent bispecific antibody, the amino acid residue is substituted for an amino acid residue that has a larger side chain volume, leading to formation of a knob on the surface of the CH3 domain of one heavy chain that can fit into a hole on the surface of the CH3 domain of another heavy chain,
   and
b) the CH3 domain of another heavy chain, which is modified so that on the surface of the CH3 domain of the second heavy chain contacting the surface of the CH3 domain of the first heavy chain in the bivalent bispecific antibody, the amino acid residue is substituted for an amino acid residue that has a smaller side chain volume, leading to formation of a hole on the surface of the CH3 domain of the second heavy chain that can accept a knob on the interface of the CH3 domain of the first heavy chain;

wherein said amino acid residue that has a larger side chain volume is selected from a group including arginine (R), phenylalanine (F), tyrosine (Y), tryptophan (W),
and wherein said amino acid residue that has a smaller side chain volume is selected from a group including alanine (A), serine (S), threonine (T), valine (V).

In some embodiments of the invention, the bivalent bispecific chimeric antibody comprises a constant domain of the first light chain of antibody, which is selected from CK or CL.

In some embodiments of the invention, the bivalent bispecific chimeric antibody comprises CH3 domains of antibody that are further modified by introduction of cysteine (C) as an amino acid into the corresponding positions of each CH3 domain so that a disulfide bridge may form between the CH3 domains.

In some embodiments of the invention, the bivalent bispecific antibody comprises a CH3 domain of one heavy chain, which is modified to form Knob, and a CH3 domain of another heavy chain, which is modified to form Hole, or vice versa.

In some embodiments of the invention, the bivalent bispecific chimeric antibody comprises a CH3 domain of one heavy chain, which has amino acid substitutions S354C/T366W, and a CH3 domain of another heavy chain, which has amino acid substitutions Y349C/T366S/L368A/Y407V.

In some embodiments of the invention, the bivalent bispecific chimeric antibody includes a CH3 domain of one heavy chain, which has amino acid substitutions Y349C/T366S/L368A/Y407, and a CH3 domain of another heavy chain, which has amino acid substitutions S354C/T366W.

In some embodiments of the invention, the bivalent bispecific chimeric antibody includes a first membrane-proximal domain of MHC and a second membrane-proximal domain of MHC that are the α2 domain of MHC I and the β2 domain of MHC II, respectively, and form a heterodimer therebetween.

In some embodiments of the invention, the bivalent bispecific chimeric antibody includes a first membrane-proximal domain of MHC and a second membrane-proximal domain of MHC that are the β2 domain of MHC II and the α2 domain of MHC II, respectively, and form a heterodimer therebetween.

In some embodiments of the invention, the bivalent bispecific chimeric antibody comprises the α2 domain of MHC II that has an amino acid sequence that is selected from the group: SEQ ID NO: 30, SEQ ID NO: 32, SEQ ID NO: 34, SEQ ID NO: 36 or SEQ ID NO: 38, and the β2 domain of MHC II that has the amino acid sequence that is selected from the group: SEQ ID NO: 31, SEQ ID NO: 33, SEQ ID NO: 35, SEQ ID NO: 37 or SEQ ID NO: 39.

In some embodiments of the invention, the bivalent bispecific chimeric antibody includes a first membrane-proximal domain of MHC and a second membrane-proximal domain of MHC that are a modified variant of the α2 domain of MHC II and a modified variant of the β2 domain of MHC II, respectively, and form a heterodimer therebetween.

In some embodiments of the invention, the bivalent bispecific chimeric antibody includes a first membrane-proximal domain of MHC and a second membrane-proximal domain of MHC that are a modified variant of the β2 domain of MHC II and a modified variant of the α2 domain of MHC II, respectively, and form a heterodimer therebetween.

In some embodiments of the invention, the bivalent bispecific chimeric antibody includes a first membrane-proximal domain of MHC and a second membrane-proximal domain of MHC that are the α3 domain of MHC I and β2 microglobulin (β2M), respectively, and form a heterodimer therebetween.

In some embodiments of the invention, the bivalent bispecific chimeric antibody includes a first membrane-proximal domain of MHC and a second membrane-proximal domain of MHC that are β2 microglobulin (β2M) and the α3 domain of MHC I, respectively, and form a heterodimer therebetween.

In some embodiments of the invention, the bivalent bispecific chimeric antibody comprises the α3 domain of MHC I that has an amino acid sequence selected from the group comprising SEQ ID NO: 1 to 29, and β2 microglobulin (β2M) that has the amino acid sequence of SEQ ID NO: 46.

In some embodiments of the invention, the bivalent bispecific chimeric antibody includes a first membrane-proximal domain of MHC and a second membrane-proximal domain of MHC that are a modified variant of the α3 domain of MHC I and a modified variant of β2 microglobulin (β2M), respectively, and form a heterodimer therebetween.

In some embodiments of the invention, the bivalent bispecific chimeric antibody includes a first membrane-proximal domain of MHC and a second membrane-proximal domain of MHC that are a modified variant of β2 microglobulin (β2M) and a modified variant of the α3 domain of MHC I, respectively, and form a heterodimer therebetween.

In some embodiments of the invention, the bivalent bispecific chimeric antibody comprises a modified variant of β2 microglobulin (β2M) that has an amino acid sequence that is selected from SEQ ID NO: 47 or SEQ ID NO: 48.

In some embodiments of the invention, the bivalent bispecific chimeric antibody includes a first membrane-proximal domain of MHC-like protein and a second membrane-proximal domain of MHC-like protein that are the α3 domain of CD1 and β2 microglobulin (β2M), respectively, and form a heterodimer therebetween.

In some embodiments of the invention, the bivalent bispecific chimeric antibody includes a first membrane-proximal domain of MHC-like protein and a second membrane-proximal domain of MHC-like protein that are β2 microglobulin (β2M) and the α3 domain of CD1, respectively, and form a heterodimer therebetween.

In some embodiments of the invention, the bivalent bispecific chimeric antibody comprises the α3 domain of CD1 that has an amino acid sequence selected from the group comprising SEQ ID NO: 40 to 44, and β2 microglobulin (β2M) that has the amino acid sequence of SEQ ID NO: 46.

In some embodiments of the invention, the bivalent bispecific chimeric antibody includes a first membrane-proximal domain of MHC-like protein and a second membrane-proximal domain of MHC-like protein that are a modified variant of the α3 domain of CD1 and a modified variant of β2 microglobulin (β2M), respectively, and form a heterodimer therebetween.

In some embodiments of the invention, the bivalent bispecific chimeric antibody includes a first membrane-proximal domain of MHC-like protein and a second membrane-proximal domain of MHC-like protein that are a modified variant of β2 microglobulin (β2M) and a modified variant of the α3 domain of CD1, respectively, and form a heterodimer therebetween.

In some embodiments of the invention, the bivalent bispecific chimeric antibody comprises a modified variant of the α3 domain of CD1 that has an amino acid sequence selected from the group comprising SEQ ID NO: 49 to 56 and/or SEQ ID NO: 109, and a modified variant of β2 microglobulin (β2M) that has an amino acid sequence selected from SEQ ID NO: 47 or SEQ ID NO: 48.

In some embodiments of the invention, the bivalent bispecific chimeric antibody includes a first membrane-proximal domain of MHC-like protein and a second membrane-proximal domain of MHC-like protein that are the α3 domain of HFE and β2 microglobulin (β2M), respectively, and form a heterodimer therebetween.

In some embodiments of the invention, the bivalent bispecific chimeric antibody includes a first membrane-proximal domain of MHC-like protein and a second membrane-proximal domain of MHC-like protein that are β2 microglobulin (β2M) and the α3 domain of HFE, respectively, and form a heterodimer therebetween.

In some embodiments of the invention, the bivalent bispecific chimeric antibody comprises the α3 domain of HFE that has an amino acid sequence of SEQ ID NO: 45, and β2 microglobulin (β2M) that has the amino acid sequence of SEQ ID NO: 46.

In some embodiments of the invention, the bivalent bispecific chimeric antibody includes a first membrane-proximal domain of MHC-like protein and a second membrane-proximal domain of MHC-like protein that are a modified variant of the α3 domain of HFE and a modified variant of β2 microglobulin (β2M), respectively, and form a heterodimer therebetween.

In some embodiments of the invention, the bivalent bispecific chimeric antibody includes a first membrane-proximal domain of MHC-like protein and a second membrane-proximal domain of MHC-like protein that are a modified variant of β2 microglobulin (β2M) and a modified variant of the α3 domain of HFE, respectively, and form a heterodimer therebetween.

In some embodiments of the invention, the bivalent bispecific chimeric antibody comprises a modified variant of β2 microglobulin (β2M) that has an amino acid sequence that is selected from SEQ ID NO: 47 or SEQ ID NO: 48.

In some embodiments of the invention, the bivalent bispecific chimeric antibody comprises a modified variant of MHC or MHC-like protein, wherein the modified variant refers to a variant comprising substitutions for cysteine (C) to form a disulfide bridge between the chains of heterodimer produced from the first and second membrane-proximal domains of MHC or MHC-like protein.

In some embodiments of the invention, the bivalent bispecific chimeric antibody comprises a modified variant of MHC or MHC-like protein, wherein the modified variant refers to a variant including one or more substitutions at various positions of the membrane-proximal domains of MHC or MHC-like protein, leading to increased thermodynamic stability Tm by more than 1 degree Celsius as compared to wild type membrane-proximal domains of MHC or MHC-like protein, respectively.

In some embodiments of the invention, the bivalent bispecific chimeric antibody comprises a modified variant of MHC or MHC-like protein, wherein the modified variant refers to a variant including one or more substitutions at various positions of the membrane-proximal domains of MHC or MHC-like protein, leading to decreased amount of aggregates by more than 5% at concentration above 10 mg/ml as compared to wild type membrane-proximal domains of MHC or MHC-like protein, respectively.

In some embodiments of the invention, the bivalent bispecific chimeric antibody comprises a modified variant of MHC or MHC-like protein, wherein the modified variant refers to a variant including one or more substitutions at various positions of the membrane-proximal domains of MHC or MHC-like protein, leading to removed glycosylation sites as compared to wild type membrane-proximal domains of MHC or MHC-like protein, respectively.

In some embodiments of the invention, the bivalent bispecific chimeric antibody comprises the variable domain of a first light chain and the variable domain of a second light chain, which are identical.

In some embodiments of the invention, the bivalent bispecific chimeric antibody comprises an Fc fragment that belongs to IgG.

In some embodiments of the invention, the bivalent bispecific chimeric antibody comprises an Fc fragment selected from the group comprising: human IgG1, IgG2, or IgG4.

In some embodiments of the invention, the bivalent bispecific chimeric antibody includes an Fc fragment monomer, wherein substitutions are further introduced, leading to absent ADCC, CDC and/or ADCP properties in the bivalent bispecific antibody.

In some embodiments of the invention, the bivalent bispecific chimeric antibody includes an Fc fragment monomer, wherein LALA substitutions (L234A and L235A) are further introduced.

In some embodiments of the invention, the bivalent bispecific chimeric antibody includes an Fc fragment monomer, wherein substitutions are further introduced, leading to prolonged action of the antibody.

In some embodiments of the invention, the bivalent bispecific chimeric antibody includes an Fc fragment monomer, wherein YTE substitutions (M252Y, S254T and T256E) are further introduced.

In some embodiments of the invention, the bivalent bispecific chimeric antibody includes an Fc fragment monomer, wherein substitutions are further introduced, leading to enhanced ADCC, CDC and/or ADCP properties in the bivalent bispecific antibody.

In some embodiments of the invention, the bivalent bispecific chimeric antibody includes an Fc fragment monomer, wherein the substitution E345R is further introduced. In some embodiments of the invention, the bivalent bispecific chimeric antibody specifically binds to CD20 and CD3.

In some embodiments of the invention, the bivalent bispecific chimeric antibody specifically binds to BCMA and CD3.

In some embodiments of the invention, the bivalent bispecific chimeric antibody specifically binds to PD-L1 and CD47.

In some embodiments of the invention, the bivalent bispecific chimeric antibody specifically binds to coagulation factor 9 (FIX) and coagulation factor 10 (FX).

In some embodiments of the invention, the bivalent bispecific chimeric antibody specifically binds to GD2 and CD3.

In some embodiments of the invention, the bivalent bispecific chimeric antibody specifically binds to AXL and CD3.

In some embodiments of the invention, the bivalent bispecific chimeric antibody specifically binds to PD-L1 and TGF beta.

In one aspect, the present invention relates to an isolated nucleic acid that encodes any of the above bivalent bispecific chimeric antibodies.

In some embodiments of the invention, the nucleic acid is DNA.

In one aspect, the present invention relates to an expression vector comprising any of the above nucleic acids.

In one aspect, the present invention relates to a method for producing a host cell for producing any of the above bivalent bispecific chimeric antibodies and includes transformation of a cell with the above expression vector.

In one aspect, the present invention relates to a host cell for producing any of the above bivalent bispecific chimeric antibodies that comprises any of the above nucleic acids.

In one aspect, the present invention relates to a method for producing any of the above bivalent bispecific chimeric antibodies that includes the steps of:
a) transforming a host cell
   - with expression vectors comprising nucleic acid molecules encoding the first light chain and the first heavy chain of the bispecific chimeric antibody,
   - with expression vectors comprising nucleic acid molecules encoding the second light chain and the second heavy chain of the bispecific chimeric antibody,
b) culturing the host cell under conditions suitable for synthesis of said bivalent bispecific chimeric antibody; and
c) isolating said bivalent bispecific antibody from cell culture.

### Brief description of drawings

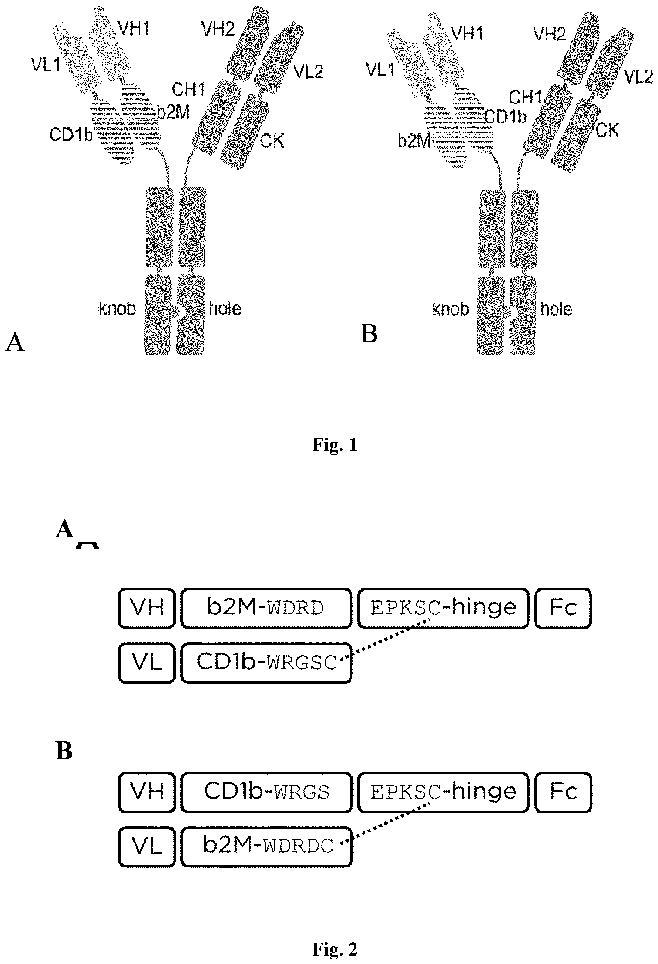

Figure 1 is a schematic representation of the format of the bivalent bispecific chimeric antibody.
   A is the "forward" orientation of the dimerization unit, B is the "reverse" orientation of the dimerization unit
   VH1, VL1 are heavy and light chain variable domains, respectively, responsible for binding to antigen 1;
   VH2, VL2 are variable domains responsible for binding to antigen 2.
   CH1, CK are heavy and light chain constant domains, respectively;
   b2M is β2 microglobulin,
   CD1b is α3 domain of CD1b protein.
   Knob, hole are mutations in CH3 domain of antibody, that enable heterodimerization of heavy chains.
Figure 2 is a schematic representation of the heavy and light chain complex of the chimeric antibody where the CH1 and CK domains were substituted for β2 microglobulin and the membrane-proximal domain α3 of CD1b protein.
   A is "direct" orientation of domains of the dimerization unit;
   B is "reverse" orientation of the dimerization units.
   VH, VL are variable domains of the antibody,
   b2M is β2 microglobulin,
   CD1b is the membrane-proximal domain α3 of CD1b protein,
   hinge is the hinge region of antibody, the first 5 amino acids are shown explicitly,
   Fc is Fc fragment of the antibody;
   amino acids from the C-terminus of the b2M, α3 domains of CD1b and from the N-terminus of the hinge region are shown explicitly;
   SS bond is shown as a dotted line.

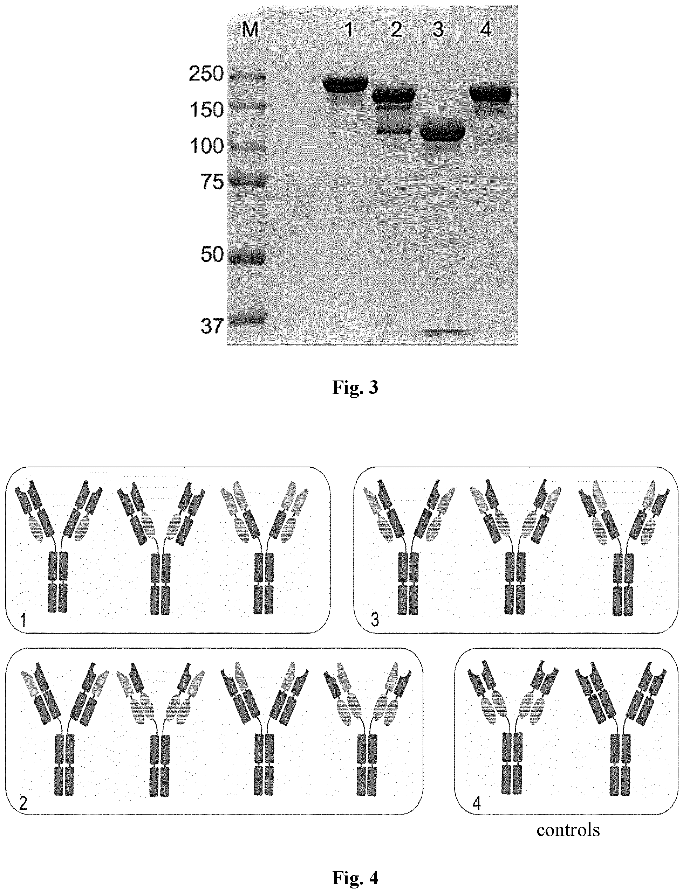

Figure 3 is an electrophoregram of chimeric antibodies comprising and free of additional disulfide bonds between heavy and light chains, 7.5% polyacrylamide gel, non-reducing conditions.
   Lanes:
   M is a standard marker of Precision Plus Protein^{™} Dual Color Standards (BIO-RAD), the molecular weights of the corresponding lanes are indicated as kDa in the column to the left of the gel;
   1- Control Prolgolimab - IgG1;
   2 - Monospecific chimeric antibody based on variable domains of Prolgolimab and domains of MHC-like proteins substituting CH1-CK, with a further disulfide bridge;
   3 - Monospecific chimeric antibody based on variable domains of Prolgolimab and domains of MHC-like proteins substituting CH1-CK, without a further disulfide bridge;
   4 - Monospecific chimeric antibody based on variable domains of Prolgolimab and domains of MHC-like proteins substituting CH1-CK, in the "reverse" orientation with a further disulfide bridge.
Figure 4 is a diagram of an experiment to test the effect of the MHC dimerization unit on the incorrect pairing between heavy and light chains. Ovals indicate domains substituting CH1 and CK from MHC-like proteins.
   Group 1: samples 01-001 - 01-003 are antibodies with a "correct" combination of VH and VL and an "incorrect" pair of constant domains.
   Group 2: samples 01-004 - 01-007 are antibodies with an "incorrect" combination of VH and VL and a "correct" pair of constant domains.
   Group 3: samples 01-008 - 01-010 are antibodies with an "incorrect" combination of VH and VL and an "incorrect" pair of constant domains.
   Group 4: samples 01-011 and 01-012 are control antibodies that are a chimeric antibody wherein constant domains are substituted for MHC dimerization units and a classical antibody of the IgG1 format, respectively.
   The "correct" combination of VH and VL means a pair of VH and VL from a single antibody. An "incorrect" combination of VH and VL means VH and VL from distinct antibodies.
   The "correct" pair of constant domains means either a pair of CH1-CK or a pair of domains from MHC-like proteins (in this case, β2 microglobulin and the α3 domain of the CD1b protein). The "incorrect" pair of constant domains means the CH1-CD1b or β2 microglobulin-CK pairs.

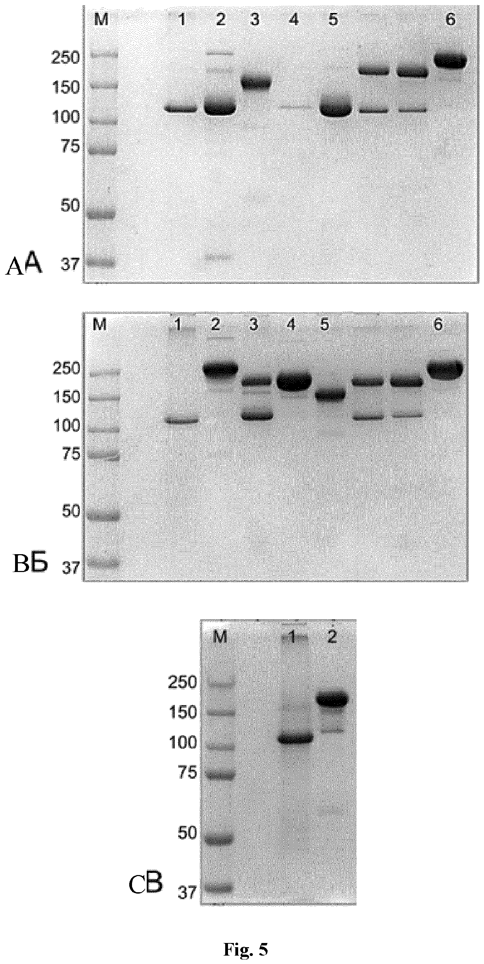

Figure 5 is an electrophoregram of samples produced in an experiment involving incorrect pairing of chains. 7.5% polyacrylamide gel, non-reducing conditions.
   **A** - Lanes:
      M is a standard marker of Precision Plus Protein^{™} Dual Color Standards (BIO-RAD), the molecular weights of the corresponding lanes are indicated as kDa in the column to the left of the gel;
      1 - 01-001;
      2 - 01-002;
      3 - 01-007;
      4 - 01-008;
      5 - 01-009;
      6 - 01-012;
   **B** - Lanes:
      M - Precision Plus Protein^{™} Dual Color Standards (BIO-RAD) standard marker;
      1 - 01-003;
      2 - 01-004;
      3 - 01-005;
      4 - 01-006;
      5 - 01-007,
      6 - 01-012;
   **C** - Lanes:
      M - Precision Plus Protein^{™} Dual Color Standards (BIO-RAD) standard marker;
      1 - 01-010;
      2 - 01-011.

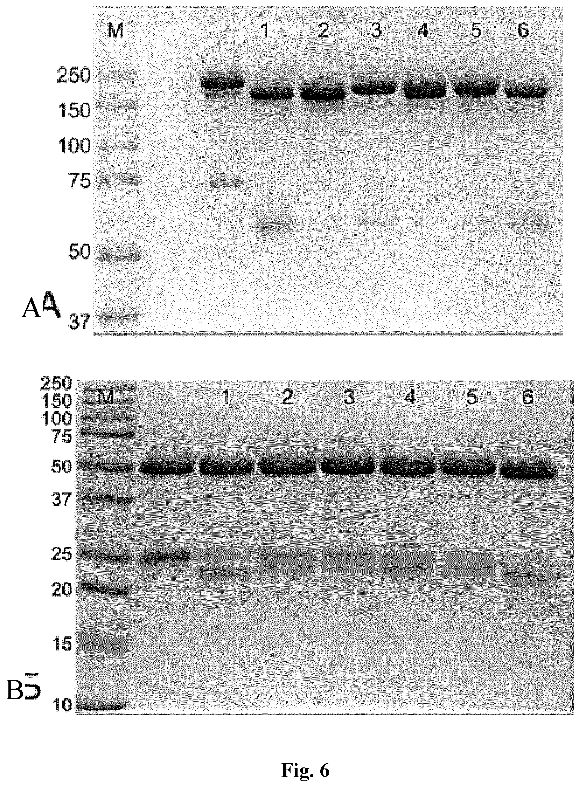

Figure 6 is an electrophoregram of bispecific chimeric antibodies.
   **A** - 7.5% polyacrylamide gel, non-reducing conditions,
   **B** - 12.5% polyacrylamide gel, reducing conditions.
      Lanes:
      M is a standard marker of Precision Plus Protein^{™} Dual Color Standards (BIO-RAD), the molecular weights of the corresponding lanes are indicated as kDa in the column to the left of the gel;
      1 - 02-004,
      2 - 02-005,
      3 - 02-006,
      4 - 02-007,
      5 - 02-008,
      6 - 02-009.
Figure 7 is a sensogram of an experiment involving simultaneous interaction of antibodies 02-006 and 02-007 with two distinct antigens (hPD1ex-H6F and hCSF1R_His). The stages (steps of the experiment) are numbered at the top of the image and separated by vertical lines. Given are 2 sensograms for each antibody that show interaction with hCSF1R His at step 8 (increased signal level is observed) and a reference signal in a 1xKB buffer free of hCSF1R His at step 8 (no increase in signal level).
Figure 8 is the deconvoluted mass spectrum of the total ion current chromatogram for sample 02-004.
   A - peak 4,
   B - peak 5,
   C - peak 6.
Figure 9 is an electrophoregram of SDS gel electrophoresis of bispecific chimeric antibodies following proteolysis by the GingisKHAN protease. 7.5% polyacrylamide gel, non-reducing conditions.
   **A** - lanes:
      M is a standard marker of Precision Plus Protein^{™} Dual Color Standards (BIO-RAD), the molecular weights of the corresponding lanes are indicated as kDa in the column to the left of the gel;
      1 - 02-004 not processed by GingisKHAN,
      2 - 02-009 not processed by GingisKHAN,
      3 - Ocrelizumab not processed by GingisKHAN,
      4 - 01-011 not processed by GingisKHAN,
      5 - Prolgolimab not processed by GingisKHAN,
      6 - 02-004 not processed by GingisKHAN in 100 mM ammonium bicarbonate buffer pH 7.2;
   **B** - lanes:
      M - Precision Plus Protein^{™} Dual Color Standards (BIO-RAD) standard marker;
      1 - 02-004 following proteolysis by GingisKHAN,
      2 - 02-009 following proteolysis by GingisKHAN,
      3 - Ocrelizumab following proteolysis by GingisKHAN,
      4 - 01-011 following proteolysis by GingisKHAN,
      5 - Prolgolimab following proteolysis by GingisKHAN,
      6 - 02-004 following proteolysis by GingisKHAN in 100 mM ammonium bicarbonate buffer pH 7.2,
   **C** - lanes:
      M - Precision Plus Protein^{™} Dual Color Standards (BIO-RAD) standard marker;
      1 - 02-005 not processed by GingisKHAN,
      2 - 02-005 following proteolysis by GingisKHAN,
      3 - 02-006 not processed by GingisKHAN,
      4 - 02-006 following proteolysis by GingisKHAN,
      5 - 02-007 not processed by GingisKHAN,
      6 - 02-007 following proteolysis by GingisKHAN,
   **D** - lanes:
      M - Precision Plus Protein^{™} Dual Color Standards (BIO-RAD) standard marker,
      1 - 02-008 not processed by GingisKHAN,
      2 - 02-008 following proteolysis by GingisKHAN,
      3 - 02-004 not processed by GingisKHAN,
      4 - 02-004 following proteolysis by GingisKHAN.

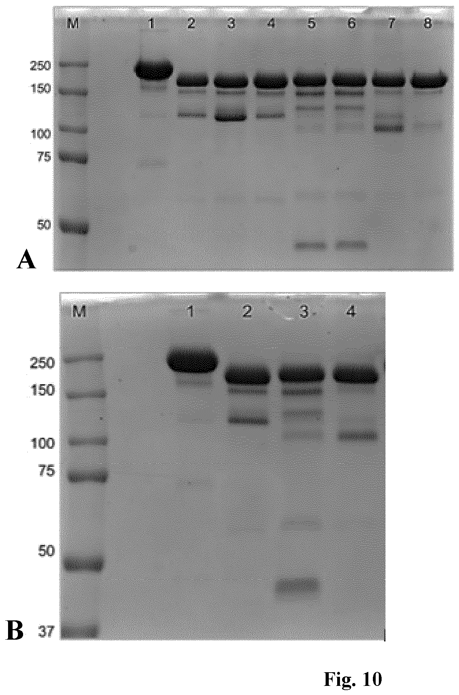

Figure 10 is an electrophoregram of samples with modifications introduced into the dimerization unit based on the membrane-proximal domains of MHC-like protein. 7.5% polyacrylamide gel, non-reducing conditions.
   **A** - Lanes:
      M is a standard marker of Precision Plus Protein^{™} Dual Color Standards (BIO-RAD), the molecular weights of the corresponding lanes are indicated as kDa in the column to the left of the gel;
      1 - Prolgolimab;
      2 - 01-011;
      3 - 03-003;
      4 - 03-004;
      5 - 03-005;
      6 - 03-006;
      7 - 03-007;
      8 - 03-008.
   **B** - Lanes:
      M - Precision Plus Protein^{™} Dual Color Standards (BIO-RAD) standard marker;
      1 - Prolgolimab;
      2 - 01-011;
      3 - 03-001;
      4 - 03-002.

### Description of the invention

### General definitions and general methods

Unless defined otherwise herein, all technical and scientific terms used in connection with the present invention will have the same meaning as is commonly understood by those skilled in the art.

Furthermore, unless otherwise required by context, singular terms shall include plural terms, and the plural terms shall include the singular terms. Typically, the present classification and methods of cell culture, molecular biology, immunology, microbiology, genetics, analytical chemistry, organic synthesis chemistry, medical and pharmaceutical chemistry, as well as hybridization and chemistry of protein and nucleic acids described herein are well known by those skilled and widely used in the art. Enzyme reactions and purification methods are performed according to the manufacturer's guidelines, as is common in the art, or as described herein.

The term "KD" in this description refers to the affinity constant (or equilibrium constant), which is calculated from the ratio of Kd to Ka (i.e. Kd/Ka), and it is expressed as a molar concentration (M).

"Binding affinity" generally refers to the strength of the sum total of noncovalent interactions between a single binding site of a molecule (e.g. an antibody) and its binding partner (e.g. an antigen). Unless indicated otherwise, "binding affinity" refers to intrinsic (characteristic, true) binding affinity which reflects a 1:1 interaction between members of a binding pair (e.g. antibody and antigen). The affinity of a molecule X for its binding partner Y can generally be represented by the affinity constant (KD). The preferred Kd value is about 200 nM, 150 nM, 100 nM, 60 nM, 50 nM, 40 nM, 30 nM, 20 nM, 10 nM, 8 nM, 6 nM, 4 nM, 2 nM, 1 nM, or less. Affinity can be measured by common methods known in the art, including those described in the present description. Low-affinity antibodies generally bind an antigen slowly and tend to dissociate readily, whereas highaffinity antibodies generally bind an antigen faster and tend to remain bound longer. A variety of methods of measuring binding affinity are known in the art, any of which can be used for the purposes of the present invention.

The term "Kd", "koff" or "kdis" refers to the off rate constant of a particular interaction between a binding molecule and antigen. The off rate constant koff can be measured using bio-layer interferometry, for example, using Octet^{™} system.

The term "Ka", "kon" or "on-rate" refers to the association rate constant.

The term "R²" refers to the coefficient of determination.

The term "Response" refers to the antibody-antigen binding signal.

As used in the present description and claims that follow, unless otherwise dictated by the context, the words "include" and "comprise", or variations thereof such as "includes", "including", "comprises", or "comprising", will be understood to imply the inclusion of a stated integer or group of integers but not the exclusion of any other integer or group of integers.

### Detailed description of the invention

### Bivalent bispecific chimeric antibody

The present invention relates to a bivalent bispecific chimeric antibody.

The antibody according to the invention is a monoclonal antibody.

The term "monoclonal antibody" or "mAb" refers to an antibody that is synthesized and isolated by a separate clonal population of cells.

The antibody of the invention is a recombinant antibody.

The term "recombinant antibody" refers to an antibody that is expressed in a cell or cell line comprising nucleotide sequence(s) encoding antibodies, wherein said nucleotide sequence(s) is (are) not associated with the cell in nature.

The bivalent bispecific chimeric antibody according to the invention is an isolated antibody.

The term "isolated" used to describe various antibodies according to this description refers to an antibody which has been identified and separated and/or regenerated from a cell or cell culture, in which the antibody is expressed. Impurities (contaminant components) from natural environment are materials which typically interfere with diagnostic or therapeutic uses of the polypeptide, and may include enzymes, hormones, and other proteinaceous or nonproteinaceous solutes. The isolated polypeptide is typically prepared by at least one purification step.

In one aspect, the present invention relates to a bivalent bispecific chimeric antibody, wherein said antibody comprises:
a) a first light chain and a first heavy chain of antibody specifically binding to a first antigen;
   wherein the first light chain comprises a light chain variable domain and a light chain constant domain;
   wherein the first heavy chain comprises a heavy chain variable domain and heavy chain constant domains of antibody that include a first (CH1) heavy chain constant domain and an Fc fragment monomer comprising second (CH2) and third (CH3) heavy chain constant domains;
b) a second light chain and a second heavy chain of antibody specifically binding to a second antigen,
   wherein the second light chain comprises a light chain variable domain and a constant domain that is selected from the group:
   a first membrane-proximal domain of MHC (major histocompatibility complex) or
   a first membrane-proximal domain of MHC-like protein;
   wherein the second heavy chain comprises a heavy chain variable domain, a constant domain that is selected from the group:
      a second membrane-proximal domain of MHC (major histocompatibility complex) or
      a second membrane-proximal domain of MHC-like protein;
      and an Fc fragment monomer comprising second (CH2) and third (CH3) heavy chain constant domains;
      wherein the first membrane-proximal domain of MHC or MHC-like protein and the second membrane-proximal domain of MHC or MHC-like protein form a heterodimer therebetween, which is stabilized by a disulfide bond;
      wherein the CH3 domain of one heavy chain and the CH3 domain of another heavy chain contact each other with surfaces thereof, which are modified to form a bivalent bispecific chimeric antibody, said modifications in the heavy chain CH3 domains being substitutions to facilitate heterodimerization.

The heterodimer that is formed by the first membrane-proximal domain of MHC or MHC-like protein and the second membrane-proximal domain of MHC or MHC-like protein and stabilized by a disulfide bond means:
1) a heterodimer that includes a mutation or mutations in the first and/or second membrane-proximal domain of MHC or MHC-like protein to form an S-S bond (disulfide bond, cysteine bridge) between the first and second membrane-proximal domain of MHC or MHC-like protein;
   or
2) an elongation of the first membrane-proximal domain of MHC or MHC-like protein by one or more (from 1 to 10) amino acids at the C-terminus and with terminal Cys at the C-terminus to form an S-S bond (disulfide bond, cysteine bridge) between the first membrane-proximal domain of MHC or MHC-like protein and the hinge.

In some embodiments of the invention, the bivalent bispecific chimeric antibody includes a first membrane-proximal domain of MHC or MHC-like protein and a second membrane-proximal domain of MHC or MHC-like protein that form a heterodimer therebetween, which is stabilized by a disulfide bond by means of a mutation or mutations in the first and/or second membrane-proximal domain of MHC or MHC-like protein to form an S-S bond (disulfide cysteine bridge) between the first and second membrane-proximal domain of MHC or MHC-like protein.

In some embodiments of the invention, the bivalent bispecific chimeric antibody includes a first membrane-proximal domain of MHC or MHC-like protein and a second membrane-proximal domain of MHC or MHC-like protein that form a heterodimer therebetween, which is stabilized by a disulfide bond by means of elongation of the first membrane-proximal domain of MHC or MHC-like protein by one or more (1 to 10) amino acids at the C-terminus and with terminal Cys at the C-terminus to form an S-S bond (disulfide bond, cysteine bridge) between the first membrane-proximal domain of MHC or MHC-like protein and the hinge.

In some embodiments of the invention, the bivalent bispecific chimeric antibody includes an elongation of the first membrane-proximal domain of MHC or MHC-like protein, which elongation is a sequence of three amino acids GSC.

In some embodiments of the invention, the bivalent bispecific chimeric antibody comprises:
a first light chain that comprises a light chain variable domain (VL1) and a light chain constant domain;
a first heavy chain that comprises a heavy chain variable domain (VH1) and heavy chain constant domains of antibody that include a first (CH1) heavy chain constant domain and an Fc fragment monomer comprising second (CH2) and third (CH3) heavy chain constant domains;
a second light chain that comprises a light chain variable domain (VL2) and a first membrane-proximal domain of MHC or a first membrane-proximal domain of MHC-like protein;
a second heavy chain that comprises a heavy chain variable domain (VH2), a second membrane-proximal domain of MHC or a second membrane-proximal domain of MHC-like protein and an Fc fragment monomer comprising second (CH2) and third (CH3) heavy chain constant domains;
wherein the first membrane-proximal domain of MHC or MHC-like protein and the second membrane-proximal domain of MHC or MHC-like protein form a heterodimer therebetween, which is stabilized by a disulfide bond by means of a mutation or mutations in the first and/or second membrane-proximal domain of MHC or MHC-like protein to form an S-S bond (disulfide cysteine bridge) between the first and second membrane-proximal domain of MHC or MHC-like protein;
wherein between the first Fc variant and the second Fc variant in the CH3 domain there is formed a knob-into-hole structure.

In some embodiments of the invention, the bivalent bispecific chimeric antibody comprises:
a first light chain that comprises a light chain variable domain (VL1) and a light chain constant domain;
a first heavy chain that comprises a heavy chain variable domain (VH1) and heavy chain constant domains of antibody that include a first (CH1) heavy chain constant domain and an Fc fragment monomer comprising second (CH2) and third (CH3) heavy chain constant domains;
a second light chain that comprises a light chain variable domain (VL2) and a first membrane-proximal domain of MHC or a first membrane-proximal domain of MHC-like protein;
a second heavy chain that comprises a heavy chain variable domain (VH2), a second membrane-proximal domain of MHC or a second membrane-proximal domain of MHC-like protein and an Fc fragment monomer comprising second (CH2) and third (CH3) heavy chain constant domains;
wherein between the first Fc variant and the second Fc variant in the CH3 domain there is formed a knob-into-hole structure,
wherein the first membrane-proximal domain of MHC or the first membrane-proximal domain of MHC-like protein further comprises an elongation of the first membrane-proximal domain of MHC or MHC-like protein by one or more (from 1 to 10) amino acids at the C-terminus and with terminal Cys at the C-terminus to form an S-S bond (disulfide bond, cysteine bridge) between the first membrane-proximal domain of MHC or MHC-like protein and the hinge.

In some embodiments of the invention, the bivalent bispecific chimeric antibody comprises:
a first light chain that comprises a light chain variable domain (VL1) and a light chain constant domain;
a first heavy chain that comprises a heavy chain variable domain (VH1) and heavy chain constant domains of antibody that include a first (CH1) heavy chain constant domain and an Fc fragment monomer comprising second (CH2) and third (CH3) heavy chain constant domains;
a second light chain that comprises a light chain variable domain (VL2) and a first membrane-proximal domain of MHC or a first membrane-proximal domain of MHC-like protein;
a second heavy chain that comprises a heavy chain variable domain (VH2), a second membrane-proximal domain of MHC or a second membrane-proximal domain of MHC-like protein and an Fc fragment monomer comprising second (CH2) and third (CH3) heavy chain constant domains;
wherein between the first Fc variant and the second Fc variant in the CH3 domain there is formed a knob-into-hole structure
wherein the first membrane-proximal domain of MHC or the first membrane-proximal domain of MHC-like protein further comprises an elongation of the first membrane-proximal domain of MHC or MHC-like protein by GSC to form an S-S bond (disulfide bond, cysteine bridge) between the first membrane-proximal domain of MHC or MHC-like protein and the hinge.

In the above bivalent bispecific chimeric antibody, the variable and constant domains are arranged in the heavy and light chains in the following order:
the first light chain:
   1) a light chain variable domain,
   2) a light chain constant domain;
the first heavy chain of antibody:
   1) a heavy chain variable domain,
   2) a first (CH1) heavy chain constant domain,
   3) a second (CH2) heavy chain constant domain and
   4) a third (CH3) heavy chain constant domain;
the second light chain:
   1) a light chain variable domain,
   2) a first membrane-proximal domain of MHC or a first membrane-proximal domain of MHC-like protein;
the second heavy chain of antibody:
   1) a heavy chain variable domain,
   2) a second membrane-proximal domain of MHC or a second membrane-proximal domain of MHC-like protein,
   3) a second (CH2) heavy chain constant domain and
   4) a third (CH3) heavy chain constant domain.

Figure 1 shows a few variants of the format of the above bivalent bispecific chimeric antibody.

The term "antibody" or "immunoglobulin" (Ig), as used in the present description, includes whole antibodies. The term "antibody" refers to a glycoprotein comprising at least two heavy (H) chains and two light (L) chains interconnected by disulfide bonds, or an antigen-binding portion. Each heavy chain comprises a heavy chain variable region (abbreviated referred to in the present description as VH) and a heavy chain constant region. Known are five types of mammalian antibody heavy chains denoted by Greek letters: α, δ, ε, γ and µ. (Janeway C.A., Jr. et al, Immunobiology, 5th ed., publ. by Garland Publishing, 2001). The type of a heavy chain present defines the class of an antibody; these chains are found in IgA, IgD, IgE, IgG, and IgM antibodies, respectively. (Rhoades R.A., Pflanzer R.G., Human Physiology, 4th ed., publ. by Thomson Learning, 2002). Distinct heavy chains differ in size and composition; α and γ contain approximately 450 amino acids, while µ and ε have approximately 550 amino acids. The constant region is identical in all antibodies of the same isotype, but differs in antibodies of different isotypes. Heavy chains γ, α and δ have a constant region composed of three constant domains CH1, CH2 and CH3 (in a line), and a hinge region for added flexibility (Woof J., Burton D., Nat Rev Immunol 4, 2004, cc.89-99); heavy chains µ and ε have a constant region composed of four constant domains CH1, CH2, CH3 and CH4 (Janeway C.A., Jr. et al, Immunobiology, 5th ed., publ. by Garland Publishing, 2001). In mammals, known are only two types of light chains denoted by lambda (λ) and kappa (κ). Each light chain consists of a light chain variable region (abbreviated referred to in the present description as VL) and light chain constant region. The approximate length of a light chain is 211 to 217 amino acids. Preferably the light chain is a kappa (κ) light chain, and the constant domain CL is preferably C kappa (κ).

"Antibodies" according to the invention can be of any class (e.g., IgA, IgD, IgE, IgG, and IgM, preferably IgG), or subclass (e.g., IgG1, IgG2, IgG3, IgG4, IgA1 and IgA2, preferably IgG1).

VL and VH regions may be further subdivided into hyper-variability regions called complementarity determining regions (CDRs), located between regions that are more conserved, termed framework regions (FRs). Each VH and VL is composed of three CDRs and four FRs, arranged from amino-terminus to carboxy-terminus in the following order: FR1, CDR1, FR2, CDR2, FR3, CDR3, FR4. The variable regions of heavy and light chains contain a binding domain that interacts with an antigen. The constant regions of antibodies may mediate the binding of immunoglobulin to host tissues or factors, including various cells of the immune system (e.g. effector cells) and the first component (C1q) of the classical complement system.

The term "antigen-binding portion" of an antibody or "antigen-binding fragment" (or simply "antibody portion" or "antibody fragment"), as used in this description, refers to one or more fragments of an antibody that retain the capability of specific binding to an antigen. An example of a binding fragment incorporated into the term "antigen-binding portion" of antibody includes a Fab fragment, i.e. a monovalent fragment consisting of VL, VH, CL and CH1 domains or a Fab-like fragment, i.e. a monovalent fragment consisting of VL, VH domains, a first membrane-proximal domain of MHC or MHC-like protein and a second membrane-proximal domain of MHC or MHC-like protein.

Preferably, the CDR of an antigen-binding portion, or the whole antigen binding portion of antibodies of the invention is derived from a mouse, lama or human donor library or substantially of human origin with certain amino acid residues altered, e.g. substituted with different amino acid residues so as to optimize specific properties of the antibody, e.g. KD, koff, IC50, EC50, ED50. Preferably, the framework regions of the antibody of the invention are of human origin or substantially of human origin (at least 80, 85, 90, 95, 96, 97, 98 or 99% of human origin).

In other embodiments, the antigen binding portion of the invention may be derived from other non-human species including mouse, lama, rabbit, rat or hamster, but not limited to. Alternatively, the antigen-binding region can be derived from the human species.

The term "variable" refers to the fact that certain portions of the variable domains greatly differ in sequence among antibodies. The V domain mediates antigen binding and determines specificity of each particular antibody for its particular antigen. However, the variability is not evenly distributed across the 110-amino acid span of the variable domains. Instead, the V regions consist of invariant fragments termed framework regions (FRs) of 15-30 amino acids separated by shorter regions of extreme variability termed "hypervariable regions" or CDRs. The variable domains of native heavy and light chains each comprise four FRs, largely adopting a beta-sheet configuration, connected by three hypervariable regions, which form loops connecting, and in some cases forming part of, the beta-sheet structure. The hypervariable regions in each chain are held together in close proximity by FRs and, with the hypervariable regions from the other chain, contribute to the formation of the antigen-binding site of antibodies (see Kabat et al., Sequences of Proteins of Immunological Interest. 5 th Ed. Public Health Service, National Institutes of Health, Bethesda, MD. (1991)). The constant domains are not involved directly in binding of antibody to antigen, but exhibit various effector functions, such as participation of antibody in antibody-dependent cellular cytotoxicity (ADCC).

The term "hypervariable region" according to the present description refers to the amino acid residues of antibody which are responsible for antigen binding. The hypervariable region typically comprises amino acid residues from a "complementarity determining region" or "CDR" and/or those residues from a "hypervariable loop".

"Kabat numbering scheme" or "numbering according to Kabat" as used in this application refers to the system for numbering of amino acid residues that are more variable (i.e. hypervariable) than other amino acid residues in variable regions of heavy and light chains of the antibody (Kabat et al. Ann. N.Y. Acad. Sci., 190:382-93 (1971); Kabat et al. Sequences of Proteins of Immunological Interest, Fifth Edition, U.S. Department of Health and Human Services, NIH Publication No. 91-3242 (1991)).

The antibody of the present invention "which binds" a target antigen refers to an antibody that binds the antigen with sufficient affinity such that the antibody can be used as a diagnostic and/or therapeutic agent targeting a protein or cell or tissue expressing the antigen, and slightly cross-reacts with other proteins. According to analytical methods: fluorescence-activated cell sorting (FACS), radioimmunoassay (RIA) or ELISA, in such embodiments, the degree of antibody binding to a non-target protein is less than 10 % of antibody binding to a specific target protein. With regard to the binding of antibody to a target molecule, the term "specific binding" or "specifically binds to" or "is specific for" a particular polypeptide or an epitope on a particular target polypeptide means binding that is significantly (measurably) different from a non-specific interaction.

Specific binding may be measured, for example, by determining binding of a molecule as compared to binding of a control molecule. For example, specific binding may be determined by competition with another molecule that is similar to the target, for example, an excess of non-labeled target. In this case, specific binding is indicated if the binding of the labeled target to a probe is competitively inhibited by the excess of unlabeled target. As used in the present description, the term "specific binding" or phrases "specifically binds to" or "is specific for" a particular polypeptide or an epitope on a particular target polypeptide may be described by example of a molecule having a Kd for the target of at least about 200 nM, or at least about 150 nM, or at least about 100 nM, or at least about 60 nM, or at least about 50 nM, or at least about 40 nM, or at least about 30 nM, or at least about 20 nM, or at least about 10 nM, or at least about 8 nM, or at least about 6 nM, or at least about 4 nM, or at least about 2 nM, or at least about 1 nM, or greater. In one embodiment, the term "specific binding" refers to binding where a molecule binds to a particular polypeptide or epitope on a particular polypeptide without substantially binding to any other polypeptide or epitope on a polypeptide.

The term "bispecific antibody" refers to an antibody having antigen-binding domains that are capable of specific binding to two distinct epitopes on a single biological molecule or capable of specific binding to epitopes on two distinct biological molecules. The bispecific antibody is also referred to herein as having "dual specificity" or as being a "dual specificity" antibody.

The fragment crystallizable region ("Fc region, Fc") of an immunoglobulin is the "tail" region of an immunoglobulin molecule that interacts with cell surface Fc-receptor, as well as some proteins of the complement system. This property allows antibodies to activate the immune system. In IgG, IgA and IgD isotypes, the Fc region is composed of two identical protein fragments, respectively, from the second and third constant domains of the two heavy chains; in IgM and IgE isotypes, the Fc contains three heavy chain constant domains (CH2, CH3, and CH4 domains) in each polypeptide chain.

"Fc fragment monomer" is understood to mean an Fc region from the second and third constant domains of either one of the two heavy chains (for IgG, IgA and IgD isotypes); for IgM and IgE isotypes, the Fc monomer comprises three constant domains of one of the two heavy chains (CH2, CH3 and CH4 domains).

In some embodiments of the invention, the bivalent bispecific chimeric antibody comprises:
a first light chain that comprises a light chain variable domain (VL1) and a light chain constant domain;
a first heavy chain that comprises a heavy chain variable domain (VH1) and heavy chain constant domains of antibody that include a first (CH1) heavy chain constant domain and an Fc fragment monomer comprising second (CH2) and third (CH3) heavy chain constant domains;
a second light chain that comprises a light chain variable domain (VL2) and a first membrane-proximal domain of MHC or a first membrane-proximal domain of MHC-like protein;
a second heavy chain that comprises a heavy chain variable domain (VH2), a second membrane-proximal domain of MHC or a second membrane-proximal domain of MHC-like protein and an Fc fragment monomer comprising second (CH2) and third (CH3) heavy chain constant domains;
and wherein between the first Fc variant and the second Fc variant in the CH3 domain there is formed a knob-into-hole structure
and between the second membrane-proximal domain of MHC and/or the second membrane-proximal domain of MHC-like protein and the hinge there is inserted a further peptide linker.

The term "peptide linker" as used herein is intended to mean any peptide having the ability to combine domains, with a length which depends on the domains which it binds to each other, and comprising any amino acid sequence. Preferably, the peptide linker has a length of 4 amino acids or more and consists of any set of amino acids selected from G, A, S, P, E, T, D, K.

MHC (major histocompatibility complex) refers to a major histocompatibility complex molecule that is the central component of the immune system of vertebrates present on the surface of all nuclear cells. There are two main forms of MHC, in particular MHC Class I and II.

MHC-like proteins refer to proteins that have a structural similarity to the extracellular portion of MHC class I molecules or MHC class II molecules. In particular, MHC-like proteins mean, for example, CD1 (cluster of differentiation 1) protein or HFE (hereditary hemochromatosis protein) protein.

The general structural similarity between MHC I, II, CD1 and HFE molecules is obvious. This includes the uniformity of the spatial organization of the whole molecule, the number of domains (four), the structural similarity of the membrane-proximal domains of MHC and MHC-like proteins, as well as the antigen-binding site, similar mol. wts.

In some embodiments of the invention, the bivalent bispecific chimeric antibody comprises a first membrane-proximal domain of MHC, which can be selected from a group comprising:
a first membrane-proximal domain of MHC class I (major histocompatibility complex class I),
a first membrane-proximal domain of MHC class II (major histocompatibility complex class II),
a modified variant of a first membrane-proximal domain of MHC class I or
a modified variant of a first membrane-proximal domain of MHC class II.

In humans, the classical MHC I genes are referred to as HLA-A, HLA-B, HLA-C, HLA-E, HLA-F or HLA-G. Class I molecules consist of two polypeptide chains: a polymorphic α-chain (sometimes referred to as heavy chain) and a smaller chain called β2 microglobulin (also known as light chain), which is generally not polymorphic. These two chains form a non-covalent heterodimer on the cell surface. The α-chain contains three domains (α1, α2 and α3). Exon 1 of the α-chain gene encodes the leader sequence, exons 2 and 3 encode the α1 and α2 domains, exon 4 encodes the α3 domain, exon 5 encodes the transmembrane domain, and exons 6 and 7 encode the cytoplasmic tail. The α-chain forms a peptide-binding region involving the α1 and α2 domains.

The α2 domain is followed by the α3 domain located at the C-terminus of the extracellular portion of the α chain of MHC I and forms together with β2 microglobulin a heterodimeric non-covalent complex. Said heterodimeric non-covalent complex composed of the α3 domain of MHC I and β2 microglobulin is referred to in the description of the present invention as a heterodimer based on the membrane-proximal domains of MHC I.

MHC class I molecules are expressed on all nucleated cells, including tumor cells. They are specifically expressed on, inter alia, T- and B-lymphocytes, macrophages, dendritic cells and neutrophils and function to present peptide fragments (typically 8-10 amino acids in length) on the surface of CD8+ to cytotoxic T-lymphocytes (CTL).

In humans, the classical MHC II genes are referred to as HLA-DM, HLA-DO, HLA-DP, HLA-DQ or HLA-DR. MHC Class II molecules are heterodimers composed of non-covalently linked alpha and beta chains. The extracellular portion of each of the chains consists of two domains (α1 (alpha1), α2 (alpha2) and β1 (beta1), β2 (beta2), respectively) and is connected by a short peptide with a transmembrane segment (approximately 30 amino acid residues long). The transmembrane segment is followed by the cytoplasmic domain containing approximately 10-15 residues.

The antigen-binding region of MHC class II molecules is formed by alpha-helical sections of interacting chains similarly to class I molecules except for: the antigen-binding groove of MHC class II molecules is formed not by two domains of a single alpha chain but by two domains of distinct chains, the α1 and β1 domains.

The α1 domain is followed by the α2 domain, which forms a heterodimeric noncovalent complex with the β2 domain. Said heterodimeric non-covalent complex composed of the α2 domain of MHC II and the β2 domain of MHC II is referred to in the description of the present invention as a heterodimer based on the membrane-proximal domains of MHC II.

In the structure of MHC class II molecules, the antigen-binding groove is more open than that of MHC class I molecules and thus receptive of longer peptides.

In some embodiments of the invention, the bivalent bispecific chimeric antibody comprises a second membrane-proximal domain of MHC, which can be selected from a group comprising:
a second membrane-proximal domain of MHC class I (major histocompatibility complex class I),
a second membrane-proximal domain of MHC class II (major histocompatibility complex class II),
a modified variant of a second membrane-proximal domain of MHC class I or
a modified variant of a second membrane-proximal domain of MHC class II.

In some embodiments of the invention, the bivalent bispecific chimeric antibody comprises a membrane-proximal domain of human MHC class I.

In some embodiments of the invention, the bivalent bispecific chimeric antibody comprises a membrane-proximal domain of human MHC class II.

In some embodiments of the invention, the bivalent bispecific chimeric antibody comprises a first membrane-proximal domain of MHC, which can be selected from a group comprising:
a first membrane-proximal domain of MHC class I (major histocompatibility complex class I),
a first membrane-proximal domain of MHC class II (major histocompatibility complex class II),
a modified variant of a first membrane-proximal domain of MHC class I or
a modified variant of a first membrane-proximal domain of MHC class II,
   and
a second membrane-proximal domain of MHC that may be selected from a group including:
   a second membrane-proximal domain of MHC class I (major histocompatibility complex class I),
   a second membrane-proximal domain of MHC class II (major histocompatibility complex class II),
   a modified variant of a second membrane-proximal domain of MHC class I or
   a modified variant of a second membrane-proximal domain of MHC class II.

In some embodiments of the invention, the bivalent bispecific chimeric antibody comprises:
a first membrane-proximal domain of MHC that is a first membrane-proximal domain of MHC class I (major histocompatibility complex class I),
   and
a second membrane-proximal domain of MHC that is a second membrane-proximal domain of MHC class I (major histocompatibility complex class I).

In some embodiments of the invention, the bivalent bispecific chimeric antibody comprises:
a first membrane-proximal domain of MHC that is a first membrane-proximal domain of MHC class II (major histocompatibility complex class II),
   and
a second membrane-proximal domain of MHC that is a second membrane-proximal domain of MHC class II (major histocompatibility complex class II).

In some embodiments of the invention, the bivalent bispecific chimeric antibody comprises:
a first membrane-proximal domain of MHC that is a modified variant of a first membrane-proximal domain of MHC class I,
   and
a second membrane-proximal domain of MHC that is a modified variant of a second membrane-proximal domain of MHC class I.

In some embodiments of the invention, the bivalent bispecific chimeric antibody comprises:
a first membrane-proximal domain of MHC that is a modified variant of a first membrane-proximal domain of MHC class II,
   and
a second membrane-proximal domain of MHC that is a modified variant of a second membrane-proximal domain of MHC class II.

In some embodiments of the invention, the bivalent bispecific chimeric antibody comprises:
a first membrane-proximal domain of MHC that is a first membrane-proximal domain of MHC class I (major histocompatibility complex class I),
   and
a second membrane-proximal domain of MHC that is a modified variant of a second membrane-proximal domain of MHC class I.

In some embodiments of the invention, the bivalent bispecific chimeric antibody comprises:
a first membrane-proximal domain of MHC that is a modified variant of a first membrane-proximal domain of MHC class I,
   and
a second membrane-proximal domain of MHC that is a second membrane-proximal domain of MHC class I (major histocompatibility complex class I).

In some embodiments of the invention, the bivalent bispecific chimeric antibody comprises:
a first membrane-proximal domain of MHC that is a first membrane-proximal domain of MHC class II (major histocompatibility complex class II),
   and
a second membrane-proximal domain of MHC that is a modified variant of a second membrane-proximal domain of MHC class II.

In some embodiments of the invention, the bivalent bispecific chimeric antibody comprises:
a first membrane-proximal domain of MHC that is a modified variant of a first membrane-proximal domain of MHC class II,
   and
a second membrane-proximal domain of MHC that is a second membrane-proximal domain of MHC class II (major histocompatibility complex class II).

In some embodiments of the invention, the bivalent bispecific chimeric antibody comprises a membrane-proximal domain of MHC class II, which is selected from the group: HLA-DM, HLA-DO, HLA-DP, HLA-DQ or HLA-DR.

In some embodiments of the invention, the bivalent bispecific chimeric antibody comprises a membrane-proximal domain of MHC class I, which is selected from the group: HLA-A, HLA-B, HLA-C, HLA-E, HLA-F or HLA-G.

In some embodiments of the invention, the bivalent bispecific chimeric antibody comprises a membrane-proximal domain of a human MHC-like protein.

In some embodiments of the invention, the bivalent bispecific chimeric antibody comprises a first membrane-proximal domain of MHC-like protein, which may be selected from a group comprising:
a first membrane-proximal domain of CD1 (cluster of differentiation 1),
a first membrane-proximal domain of HFE (hemochromatosis protein),
a modified variant of a first membrane-proximal domain of CD1 or
a modified variant of a first membrane-proximal domain of HFE.

CD1 (cluster of differentiation 1) refers to a cluster of differentiation 1 molecule that is a component of the immune system located on the surface of various antigenpresenting cells, such as dendritic cells, macrophages and other cells. In the same fashion as MHC classes I, II, CD1 present antigens for recognition by T cells through interaction with the T cell receptor. Unlike MHC classes I, II, CD1 proteins present lipids and derivatives thereof rather than peptides.

The plurality of CD1 variants found in humans are divided into 5 groups: CD1a, CD1b, CD1c, CD1d, CD1e, differring in the structure of the antigen-binding fragment and, consequently, specificity for lipids of different structures. CD1e group proteins, unlike proteins of other groups, are not expressed on the cell surface, but are soluble and are responsible for lipid transport (Kaczmarek, R., Pasciak, M., Szymczak-Kulus, K., & Czerwinski, M. (2017). CD1: A Singed Cat of the Three Antigen Presentation Systems. Archivum Immunologiae et Therapiae Experimentalis, 65(3), 201-214).

CD1 molecules are similar in structure to MHC class I. In a similar fashion, one CD1 molecule is a non-covalent complex consisting of two polypeptide chains: a polymorphic α-chain (sometimes referred to as heavy chain) and a smaller chain called β2 microglobulin (also known as light chain), which is generally not polymorphic.

The α chain forms an antigen-binding region comprising the α1 and α2 domains. The α2 domain is followed by the α3 domain located at the C-terminus of the extracellular portion of the α chain of CD1 and forms together with β2 microglobulin a heterodimeric non-covalent complex. Said heterodimeric non-covalent complex composed of the α3 domain of CD1 and β2 micro globulin is referred to in the description of the present invention as a heterodimer based on the membrane-proximal domains of MHC-like proteins.

HFE (Hereditary hemochromatosis protein) refers to a hemochromatosis protein molecule. The human HFE gene locus is located on chromosome 6 and consists of 5 exons and 4 introns. Hemochromatosis protein is a membrane protein associated with β2 microglobulin, it is responsible for the regulation of iron absorption via the regulation of transferrin-transferrin receptor interaction. Mutations in the HFE gene lead to hemochromatosis, an iron metabolism-related recessive pathology associated with iron accumulation in various organs. The HFE molecule is identical in structure to MHC I. One HFE molecule is a non-covalent complex consisting of two polypeptide chains: a polymorphic α-chain (sometimes referred to as heavy chain) and a smaller chain called β2 microglobulin (also known as light chain), which is generally not polymorphic.

The α chain includes α1, α2 and α3 domains. The α3 domain follows the α2 domain and is located at the C-terminus of the extracellular portion of the α chain of HFE and forms with β2 microglobulin a heterodimeric non-covalent complex. Said heterodimeric non-covalent complex composed of the α3 domain of HFE and β2 microglobulin is referred to in the description of the present invention as a heterodimer based on the membrane-proximal domains of MHC-like proteins.

In some embodiments of the invention, the bivalent bispecific chimeric antibody comprises a second membrane-proximal domain of MHC-like protein, which may be selected from a group comprising:
a second membrane-proximal domain of CD1 (cluster of differentiation 1),
a second membrane-proximal domain of HFE (hemochromatosis protein),
a modified variant of a second membrane-proximal domain of CD1 or
a modified variant of a second membrane-proximal domain of HFE.

In some embodiments of the invention, the bivalent bispecific chimeric antibody comprises:
a first membrane-proximal domain of MHC-like protein that may be selected from a group comprising:
a first membrane-proximal domain of CD1 (cluster of differentiation 1),
a first membrane-proximal domain of HFE (hemochromatosis protein),
a modified variant of a first membrane-proximal domain of CD1 or
a modified variant of a first membrane-proximal domain of HFE,
   and
a second membrane-proximal domain of MHC-like protein that may be selected from a group comprising:
   a second membrane-proximal domain of CD1 (cluster of differentiation 1),
   a second membrane-proximal domain of HFE (hemochromatosis protein),
   a modified variant of a second membrane-proximal domain of CD1 or
   a modified variant of a second membrane-proximal domain of HFE.

In some embodiments of the invention, the bivalent bispecific chimeric antibody comprises:
a first membrane-proximal domain of MHC-like protein, which is a first membrane-proximal domain of CD1,
   and
a second membrane-proximal domain of MHC-like protein, which is a second membrane-proximal domain of CD1.

In some embodiments of the invention, the bivalent bispecific chimeric antibody comprises:
a first membrane-proximal domain of MHC-like protein, which is a first membrane-proximal domain of HFE,
   and
a second membrane-proximal domain of MHC-like protein, which is a second membrane-proximal domain of HFE,

In some embodiments of the invention, the bivalent bispecific chimeric antibody comprises:
a first membrane-proximal domain of MHC-like protein, which is a modified variant of a first membrane-proximal domain of CD1,
   and
a second membrane-proximal domain of MHC-like protein, which is a modified variant of a second membrane-proximal domain of CD1.

In some embodiments of the invention, the bivalent bispecific chimeric antibody comprises:
a first membrane-proximal domain of MHC-like protein, which is a modified variant of a first membrane-proximal domain of HFE,
   and
a second membrane-proximal domain of MHC-like protein, which is a modified variant of a second membrane-proximal domain of HFE.

In some embodiments of the invention, the bivalent bispecific chimeric antibody comprises:
a first membrane-proximal domain of MHC-like protein, which is a first membrane-proximal domain of CD1,
   and
a second membrane-proximal domain of MHC-like protein, which is a modified variant of a second membrane-proximal domain of CD1.

In some embodiments of the invention, the bivalent bispecific chimeric antibody comprises:
a first membrane-proximal domain of MHC-like protein, which is a modified variant of a first membrane-proximal domain of CD1,
   and
a second membrane-proximal domain of MHC-like protein, which is a second membrane-proximal domain of CD1.

In some embodiments of the invention, the bivalent bispecific chimeric antibody comprises:
a first membrane-proximal domain of MHC-like protein, which is a first membrane-proximal domain of HFE,
   and
a second membrane-proximal domain of MHC-like protein, which is a modified variant of a second membrane-proximal domain of HFE.

In some embodiments of the invention, the bivalent bispecific chimeric antibody comprises:
a first membrane-proximal domain of MHC-like protein, which is a modified variant of a first membrane-proximal domain of HFE,
   and
a second membrane-proximal domain of MHC-like protein, which is a second membrane-proximal domain of HFE.

In some embodiments of the invention, the bivalent bispecific chimeric antibody comprises a membrane-proximal domain of CD1, which is selected from the group: CD1a, CD1b, CD1c, CD1d or CD1e.

In some embodiments of the invention, the bivalent bispecific chimeric antibody includes a variable fragment of the second light chain (VL), which is separated from a first membrane-proximal domain of MHC or MHC-like protein by a linker of 1 to 25 amino acids long, and/or a variable fragment of the second heavy chain (VH), which is separated from a second membrane-proximal domain of MHC or MHC-like protein by a linker of 1 to 25 amino acids long.

In some embodiments of the invention, the bivalent bispecific chimeric antibody comprises:
a) the CH3 domain of one heavy chain, which is modified so that on the surface of the CH3 domain of one heavy chain contacting the surface of the CH3 domain of another heavy chain in the bivalent bispecific antibody, the amino acid residue is substituted for an amino acid residue that has a larger side chain volume, leading to formation of a knob on the surface of the CH3 domain of one heavy chain that can fit into a hole on the surface of the CH3 domain of another heavy chain,
   and
b) the CH3 domain of another heavy chain, which is modified so that on the surface of the CH3 domain of the second heavy chain contacting the surface of the CH3 domain of the first heavy chain in the bivalent bispecific antibody, the amino acid residue is substituted for an amino acid residue that has a smaller side chain volume, leading to formation of a hole on the surface of the CH3 domain of the second heavy chain that can accept a knob on the interface of the CH3 domain of the first heavy chain;
   wherein said amino acid residue that has a larger side chain volume is selected from a group including arginine (R), phenylalanine (F), tyrosine (Y), tryptophan (W),
   and wherein said amino acid residue that has a smaller side chain volume is selected from a group including alanine (A), serine (S), threonine (T), valine (V).

In some embodiments of the invention, the bivalent bispecific chimeric antibody comprises a constant domain of the first light chain of antibody, which is selected from CK or CL.

In mammals, known are only two types of light chains denoted by lambda (λ) and kappa (κ). The constant domain of the lambda light chain is designated CL, and that of the kappa light chain is designated CK.

In some embodiments of the invention, the bivalent bispecific chimeric antibody comprises CH3 domains of antibody that are further modified by introduction of cysteine (C) as an amino acid into the corresponding positions of each CH3 domain so that a disulfide bridge may form between the both CH3 domains.

In some embodiments of the invention, the bivalent bispecific antibody comprises a CH3 domain of one heavy chain, which is modified to form Knob, and a CH3 domain of another heavy chain, which is modified to form Hole, or vice versa.

"knobs-into-holes" (interactions of the "knobs -into-holes" type) is an approach that enables to circumvent the problem associated with mispaired byproducts. This approach aims at forcing the pairing of two different antibody heavy chains by introducing mutations into the CH3 domains to modify the contact interfaces. On one chain, bulky amino acids were replaced by amino acids with short side chains to create a "hole". Conversely, amino acids with large side chains were introduced into the other CH3 domain to create a "knob". Co-expression of these two heavy chains produced a high yield of the heterodimer formation ("knob-hole") relative to the homodimer formation ("hole-hole" or "knob-knob") (WO9627011 and WO9850431, as well as Merchant AM ET ALL., An efficient route to human bispecific IgG, Nat Biotechnol. 1998 Jul;16(7):677-81).

In some embodiments of the invention, the bivalent bispecific chimeric antibody comprises a CH3 domain of one heavy chain, which has amino acid substitutions S354C/T366W, and a CH3 domain of another heavy chain, which has amino acid substitutions Y349C/T366S/L368A/Y407V.

In some embodiments of the invention, the bivalent bispecific chimeric antibody includes a CH3 domain of one heavy chain, which has amino acid substitutions Y349C/T366S/L368A/Y407, and a CH3 domain of another heavy chain, which has amino acid substitutions S354C/T366W.

In some embodiments of the invention, the bivalent bispecific chimeric antibody includes a first membrane-proximal domain of MHC and a second membrane-proximal domain of MHC that are the α2 domain of MHC I and the β2 domain of MHC II, respectively, and form a heterodimer therebetween.

In some embodiments of the invention, the bivalent bispecific chimeric antibody includes a first membrane-proximal domain of MHC and a second membrane-proximal domain of MHC that are the β2 domain of MHC II and the α2 domain of MHC II, respectively, and form a heterodimer therebetween.

In some embodiments of the invention, the bivalent bispecific chimeric antibody comprises the α2 domain of MHC II that has an amino acid sequence that is selected from the group: SEQ ID NO: 30, SEQ ID NO: 32, SEQ ID NO: 34, SEQ ID NO: 36 or SEQ ID NO: 38, and the β2 domain of MHC II that has the amino acid sequence that is selected from the group: SEQ ID NO: 31, SEQ ID NO: 33, SEQ ID NO: 35, SEQ ID NO: 37 or SEQ ID NO: 39.

SEQ ID NO: 30 is an amino acid sequence of the α2 membrane-proximal domain of MHC class II HLA-DM (HLA-DMA*01:01:01:01).

SEQ ID NO: 31 is an amino acid sequence of the β2 membrane-proximal domain of MHC class II HLA-DM (HLA-DMB*01:01:01:01).

SEQ ID NO: 32 is an amino acid sequence of the α2 membrane-proximal domain of MHC class II HLA-DO (HLA-DOA*01:01:01).

SEQ ID NO: 33 is an amino acid sequence of the β2 membrane-proximal domain of MHC class II HLA-DO (HLA-DOB*01:01:01:01).

SEQ ID NO: 34 is an amino acid sequence of the α2 membrane-proximal domain of MHC class II HLA-DP (HLA-DPA1*01:03:01:01).

SEQ ID NO: 35 is an amino acid sequence of the β2 membrane-proximal domain of MHC class II HLA-DP (HLA-DPB1*01:01:01:01).

SEQ ID NO: 36 is an amino acid sequence of the α2 membrane-proximal domain of MHC class II HLA-DQ (HLA-DQA1*01:01:01:01).

SEQ ID NO: 37 is an amino acid sequence of the β2 membrane-proximal domain of MHC class II HLA-DQ (HLA-DQB1*05:01:01:01).

SEQ ID NO: 38 is an amino acid sequence of the α2 membrane-proximal domain of MHC 2 class II HLA-DR (HLA-DRA*01:01:01:01).

SEQ ID NO: 39 is an amino acid sequence of the β2 membrane-proximal domain of MHC class HLA-DR (HLA-DRB1*01:01:01:01).

In some embodiments of the invention, the bivalent bispecific chimeric antibody includes a first membrane-proximal domain of MHC and a second membrane-proximal domain of MHC that are a modified variant of the α2 domain of MHC II and a modified variant of the β2 domain of MHC II, respectively, and form a heterodimer therebetween.

In some embodiments of the invention, the bivalent bispecific chimeric antibody includes a first membrane-proximal domain of MHC and a second membrane-proximal domain of MHC that are a modified variant of the β2 domain of MHC II and a modified variant of the α2 domain of MHC II, respectively, and form a heterodimer therebetween.

In some embodiments of the invention, the bivalent bispecific chimeric antibody includes a first membrane-proximal domain of MHC and a second membrane-proximal domain of MHC that are the α3 domain of MHC I and β2 microglobulin (β2M), respectively, and form a heterodimer therebetween.

In some embodiments of the invention, the bivalent bispecific chimeric antibody includes a first membrane-proximal domain of MHC and a second membrane-proximal domain of MHC that are β2 microglobulin (β2M) and the α3 domain of MHC I, respectively, and form a heterodimer therebetween.

β2 microglobulin is a non-glycosylated protein of 12 kDa; one of functions thereof is to stabilize the α chain of MHC class I, as well as CD1 and HFE proteins. The human β2 microglobulin within the above proteins has a virtually identical structure and, therefore, in the description of the present invention, the human β2 microglobulin is mentioned without indicating the protein that it is part of.

Unlike the α chain, β2 microglobulin does not pass through the membrane. The locus of the human β2 microglobulin is located on chromosome 15. The β2 microglobulin gene consists of 4 exons and 3 introns.

In some embodiments of the invention, the bivalent bispecific chimeric antibody comprises the α3 domain of MHC I that has an amino acid sequence selected from the group comprising SEQ ID NO: 1 to 29, and β2 microglobulin (β2M) that has the amino acid sequence of SEQ ID NO: 46.

SEQ ID NO: 1 is an amino acid sequence of the α3 membrane-proximal domain of MHC class I HLA-A (allele A*01:01:01:01).

SEQ ID NO: 2 is an amino acid sequence of the α3 membrane-proximal domain of MHC class I HLA-A (A*02:01:01:01).

SEQ ID NO: 3 is an amino acid sequence of the α3 membrane-proximal domain of MHC class I HLA-A (A*03:01:01:01).

SEQ ID NO: 4 is an amino acid sequence of the α3 membrane-proximal domain of MHC class I HLA-A (A* 11:01:01:01).

SEQ ID NO: 5 is an amino acid sequence of the α3 membrane-proximal domain of MHC class I HLA-A (A*23:01:01:01).

SEQ ID NO: 6 is an amino acid sequence of the α3 membrane-proximal domain of MHC class I HLA-B (B*07:02:01:01).

SEQ ID NO: 7 is an amino acid sequence of the α3 membrane-proximal domain of MHC class I HLA-B (B*08:01:01:01).

SEQ ID NO: 8 is an amino acid sequence of the α3 membrane-proximal domain of MHC class I HLA-B (B*13:01:01:01).

SEQ ID NO: 9 is an amino acid sequence of the α3 membrane-proximal domain of MHC class I HLA-B (B*14:01:01:01).

SEQ ID NO: 10 is an amino acid sequence of the α3 membrane-proximal domain of MHC class I HLA-B (B*15:01:01:01).

SEQ ID NO: 11 is an amino acid sequence of the α3 membrane-proximal domain of MHC class I HLA-C (C*01:02:01:01).

SEQ ID NO: 12 is an amino acid sequence of the α3 membrane-proximal domain of MHC class I HLA-C (C*03:02:01).

SEQ ID NO: 13 is an amino acid sequence of the α3 membrane-proximal domain of MHC class I HLA-C (C*04:01:01:01).

SEQ ID NO: 14 is an amino acid sequence of the α3 membrane-proximal domain of MHC class I HLA-C (C*05:01:01:01).

SEQ ID NO: 15 is an amino acid sequence of the α3 membrane-proximal domain of MHC class I HLA-C (C*06:02:01:01).

SEQ ID NO: 16 is an amino acid sequence of the α3 membrane-proximal domain of MHC class I HLA-E (E*01:01:01:01).

SEQ ID NO: 17 is an amino acid sequence of the α3 membrane-proximal domain of MHC class I HLA-E (E*01:03:01:01).

SEQ ID NO: 18 is an amino acid sequence of the α3 membrane-proximal domain of MHC class I HLA-E (E*01:05).

SEQ ID NO: 19 is an amino acid sequence of the α3 membrane-proximal domain of MHC class I HLA-E (E*01:06).

SEQ ID NO: 20 is an amino acid sequence of the α3 membrane-proximal domain of MHC class I HLA-E (E*01:09).

SEQ ID NO: 21 is an amino acid sequence of the α3 membrane-proximal domain of MHC class I HLA-F (F*01:01:01:01).

SEQ ID NO: 22 is an amino acid sequence of the α3 membrane-proximal domain of MHC class I HLA-F (F*01:02).

SEQ ID NO: 23 is an amino acid sequence of the α3 membrane-proximal domain of MHC class I HLA-F (F*01:03:01:01).

SEQ ID NO: 24 is an amino acid sequence of the α3 membrane-proximal domain of MHC class I HLA-F (F*01:04:01:01).

SEQ ID NO: 25 is an amino acid sequence of the α3 membrane-proximal domain of MHC class I HLA-F (F*01:05).

SEQ ID NO: 26 is an amino acid sequence of the α3 membrane-proximal domain of MHC class I HLA-G (G*01:01:01:01).

SEQ ID NO: 27 is an amino acid sequence of the α3 membrane-proximal domain of MHC class I HLA-G (G*01:03:01:01).

SEQ ID NO: 28 is an amino acid sequence of the α3 membrane-proximal domain of MHC class I HLA-G (G*01:04:01:01).

SEQ ID NO: 29 is an amino acid sequence of the α3 membrane-proximal domain of MHC class I HLA-G (G*01:06).

SEQ ID NO: 46 is an amino acid sequence of the universal β2 microglobulin (β2M) of MHC class I or MHC-like proteins (CD1, HFE).

In some embodiments of the invention, the bivalent bispecific chimeric antibody includes a first membrane-proximal domain of MHC and a second membrane-proximal domain of MHC that are a modified variant of the α3 domain of MHC I and a modified variant of β2 microglobulin (β2M), respectively, and form a heterodimer therebetween.

In some embodiments of the invention, the bivalent bispecific chimeric antibody includes a first membrane-proximal domain of MHC and a second membrane-proximal domain of MHC that are a modified variant of β2 microglobulin (β2M) and a modified variant of the α3 domain of MHC I, respectively, and form a heterodimer therebetween.

In some embodiments of the invention, the bivalent bispecific chimeric antibody comprises a modified variant of β2 microglobulin (β2M) that has an amino acid sequence that is selected from SEQ ID NO: 47 or SEQ ID NO: 48.

SEQ ID NO:47 is an amino acid sequence of the universal β2 microglobulin (β2M) of MHC class I or MHC-like proteins (CD1, HFE) with the mutation R12C (SS bridge is inside the unit).

SEQ ID NO: 48 is an amino acid sequence of the universal β2 microglobulin (β2M) of MHC class I or MHC-like proteins (CD1, HFE) with the mutation R12C (SS bridge is inside the unit) + hinge with the mutation C220A (SS bridge has been removed from the C-terminus of the unit and transferred inside the unit).

In some embodiments of the invention, the bivalent bispecific chimeric antibody includes a first membrane-proximal domain of MHC-like protein and a second membrane-proximal domain of MHC-like protein that are the α3 domain of CD1 and β2 microglobulin (β2M), respectively, and form a heterodimer therebetween.

In some embodiments of the invention, the bivalent bispecific chimeric antibody includes a first membrane-proximal domain of MHC-like protein and a second membrane-proximal domain of MHC-like protein that are β2 microglobulin (β2M) and the α3 domain of CD1, respectively, and form a heterodimer therebetween.

In some embodiments of the invention, the bivalent bispecific chimeric antibody comprises the α3 domain of CD1 that has an amino acid sequence selected from the group comprising SEQ ID NO: 40 to 44, and β2 microglobulin (β2M) that has the amino acid sequence of SEQ ID NO: 46.

SEQ ID NO: 40 is an amino acid sequence of the α3 membrane-proximal domain of CD1a.

SEQ ID NO: 41 is an amino acid sequence of the α3 membrane-proximal domain of CD1b.

SEQ ID NO: 42 is an amino acid sequence of the α3 membrane-proximal domain of CD1c.

SEQ ID NO: 43 is an amino acid sequence of the α3 membrane-proximal domain of CD1d.

SEQ ID NO: 44 is an amino acid sequence of the α3 membrane-proximal domain of CD1e.

In some embodiments of the invention, the bivalent bispecific chimeric antibody includes a first membrane-proximal domain of MHC-like protein and a second membrane-proximal domain of MHC-like protein that are a modified variant of the α3 domain of CD1 and a modified variant of β2 microglobulin (β2M), respectively, and form a heterodimer therebetween.

In some embodiments of the invention, the bivalent bispecific chimeric antibody includes a first membrane-proximal domain of MHC-like protein and a second membrane-proximal domain of MHC-like protein that are a modified variant of β2 microglobulin (β2M) and a modified variant of the α3 domain of CD1, respectively, and form a heterodimer therebetween.

In some embodiments of the invention, the bivalent bispecific chimeric antibody comprises a modified variant of the α3 domain of CD1 that has an amino acid sequence selected from the group comprising SEQ ID NO: 49 to 56 or SEQ ID NO: 109, and a modified variant of β2 microglobulin (β2M) that has an amino acid sequence selected from SEQ ID NO: 47 or SEQ ID NO: 48.

SEQ ID NO: 49 is an amino acid sequence of the α3 membrane-proximal domain of CD1b with the mutation N57C (an additional SS bridge inside the unit) and an elongation by 3 amino acids GSC at the C terminus.

SEQ ID NO: 50 is an amino acid sequence of the α3 membrane-proximal domain of CD1b with the mutation N57C (the SS bridge is inside the unit) and an elongation by 2 amino acids GS at the C terminus (the SS bridge has been removed from the C terminus of the unit and transferred inside the unit).

SEQ ID NO: 51 is an amino acid sequence of the α3 membrane-proximal domain of CD1b with the mutation N59A (substitution at the predicted N-glycosylation site) and an elongation by 3 amino acids GSC at the C terminus.

SEQ ID NO: 52 is an amino acid sequence of the α3 membrane-proximal domain of CD1b with the mutation N59D (substitution at the predicted N-glycosylation site) and an elongation by 3 amino acids GSC at the C terminus.

SEQ ID NO: 53 is an amino acid sequence of the α3 membrane-proximal domain of CD1b with the mutations N57C, N59A and an elongation by 3 amino acids GSC at the C terminus (the additional SS-bridge is inside the unit + the predicted N-glycosylation site has been removed).

SEQ ID NO: 54 is an amino acid sequence of the α3 membrane-proximal domain of CD1b with the mutations N57C, N59D and an elongation by 3 amino acids GSC at the C terminus (the additional SS-bridge is inside the unit + the predicted N-glycosylation site has been removed).

SEQ ID NO: 55 is an amino acid sequence of the α3 membrane-proximal domain of CD1b with the mutations N57C, N59A and an elongation by 2 amino acids GS at the C terminus (the SS-bridge has been transferred inside the unit + the predicted N-glycosylation site has been removed).

SEQ ID NO: 56 is an amino acid sequence of the α3 membrane-proximal domain of CD1b with the mutations N57C, N59D and an elongation by 2 amino acids GS at the C terminus (the SS-bridge has been transferred inside the unit + the predicted N-glycosylation site has been removed).

SEQ ID NO: 109 is an amino acid sequence of the α3 membrane-proximal domain of CD1b with an elongation by 3 amino acids GSC at the C terminus.

In some embodiments of the invention, the bivalent bispecific chimeric antibody includes a first membrane-proximal domain of MHC-like protein and a second membrane-proximal domain of MHC-like protein that are the α3 domain of HFE and β2 microglobulin (β2M), respectively, and form a heterodimer therebetween.

In some embodiments of the invention, the bivalent bispecific chimeric antibody includes a first membrane-proximal domain of MHC-like protein and a second membrane-proximal domain of MHC-like protein that are β2 microglobulin (β2M) and the α3 domain of HFE, respectively, and form a heterodimer therebetween.

In some embodiments of the invention, the bivalent bispecific chimeric antibody comprises the α3 domain of HFE that has an amino acid sequence of SEQ ID NO: 45, and β2 microglobulin (β2M) that has the amino acid sequence of SEQ ID NO: 46.

SEQ ID NO: 45 is an amino acid sequence of the α3 membrane-proximal domain of HFE.

In some embodiments of the invention, the bivalent bispecific chimeric antibody includes a first membrane-proximal domain of MHC-like protein and a second membrane-proximal domain of MHC-like protein that are a modified variant of the α3 domain of HFE and a modified variant of β2 microglobulin (β2M), respectively, and form a heterodimer therebetween.

In some embodiments of the invention, the bivalent bispecific chimeric antibody includes a first membrane-proximal domain of MHC-like protein and a second membrane-proximal domain of MHC-like protein that are a modified variant of β2 microglobulin (β2M) and a modified variant of the α3 domain of HFE, respectively, and form a heterodimer therebetween.

In some embodiments of the invention, the bivalent bispecific chimeric antibody comprises a modified variant of β2 microglobulin (β2M) that has an amino acid sequence that is selected from SEQ ID NO: 47 or SEQ ID NO: 48.

Known are more than 1,000 characterized structures of MHC, CD1 and HFE. Any of the above structures may be used in the bispecific antibody according to the invention.

In some embodiments of the invention, the bivalent bispecific chimeric antibody comprises a modified variant of MHC or MHC-like protein, wherein the modified variant refers to a variant comprising substitutions for cysteine (C) to form a disulfide bridge between the chains of heterodimer produced from the first and second membrane-proximal domains of MHC or MHC-like protein.

In some embodiments of the invention, the bivalent bispecific chimeric antibody comprises a modified variant of MHC or MHC-like protein, wherein the modified variant refers to a variant including one or more substitutions at various positions of the membrane-proximal domains of MHC or MHC-like protein, leading to increased thermodynamic stability Tm by more than 1 degree Celsius as compared to wild type membrane-proximal domains of MHC or MHC-like protein, respectively.

In some embodiments of the invention, the bivalent bispecific chimeric antibody comprises a modified variant of MHC or MHC-like protein, wherein the modified variant refers to a variant including one or more substitutions at various positions of the membrane-proximal domains of MHC or MHC-like protein, leading to decreased amount of aggregates by more than 5% at concentration above 10 mg/ml as compared to wild type membrane-proximal domains of MHC or MHC-like protein, respectively.

In some embodiments of the invention, the bivalent bispecific chimeric antibody comprises a modified variant of MHC or MHC-like protein, wherein the modified variant refers to a variant including one or more substitutions at various positions of the membrane-proximal domains of MHC or MHC-like protein, leading to removed glycosylation sites as compared to wild type membrane-proximal domains of MHC or MHC-like protein, respectively.

In some embodiments of the invention, the bivalent bispecific chimeric antibody comprises the variable domain of a first light chain and the variable domain of a second light chain, which are identical.

In some embodiments of the invention, the bivalent bispecific chimeric antibody comprises an Fc fragment that belongs to IgG.

In some embodiments of the invention, the bivalent bispecific chimeric antibody comprises an Fc fragment selected from the group comprising: human IgG1, IgG2, or IgG4.

In some embodiments of the invention, the bivalent bispecific chimeric antibody includes an Fc fragment monomer, wherein substitutions are further introduced, leading to absent ADCC, CDC and/or ADCP properties in the bivalent bispecific antibody.

In some embodiments of the invention, the bivalent bispecific chimeric antibody includes an Fc fragment monomer, wherein LALA substitutions (L234A and L235A) are further introduced.

In some embodiments of the invention, the bivalent bispecific chimeric antibody includes an Fc fragment monomer, wherein substitutions are further introduced, leading to prolonged action of the antibody.

In some embodiments of the invention, the bivalent bispecific chimeric antibody includes an Fc fragment monomer, wherein YTE substitutions (M252Y, S254T and T256E) are further introduced.

In some embodiments of the invention, the bivalent bispecific chimeric antibody includes an Fc fragment monomer, wherein substitutions are further introduced, leading to enhanced ADCC, CDC and/or ADCP properties in the bivalent bispecific antibody.

In some embodiments of the invention, the bivalent bispecific chimeric antibody includes an Fc fragment monomer, wherein the substitution E345R is further introduced.

The above mutations in the Fc fragment are numbered according to EU numbering for amino acid chains of antibodies (Edelman, G.M., et al., Proc. Natl. Acad. Sci. USA 63 (1969), pp. 78-85; Kabat, E.A., et al., Sequences of Proteins of Immunological Interest, 5th ed., Public Health Service, National Institutes of Health, Bethesda, MD, (1991).

Mutations in Fc fragment are understood to mean modification(s) of the amino acid sequences of antibodies described in the present application. Amino acid sequence variants of antibody are prepared by introducing appropriate nucleotide changes into the antibody nucleic acid, or by peptide synthesis. Such modifications include, for example, deletions, and/or insertions and/or substitutions of residues within the amino acid sequences of antibody. Any combination of deletion, insertion, and substitution is made to arrive at the final construct, provided that the final construct possesses the desired characteristics.

A variant of modification of the amino acid sequences of antibodies using amino acid substitutions is the substitution of at least one amino acid residue in the antibody molecule with another residue.

Conservative substitutions are shown in Table A under "preferred substitutions".

| Table A | | |
|---|---|---|
| Initial residue | Exemplary substitutions | Preferred substitutions |
| Ala (A) | Val; Leu; Ile | Val |
| Arg(R) | Lys; Gln; Asn | Lys |
| Asn(N) | Gln; His; Asp, Lys; Arg | Gln |
| Asp (D) | Glu; Asn | Glu |
| Cys (C) | Ser; Ala | Ser |
| Gln(Q) | Asn; Glu | Asn |
| Glu (E) | Asp; Gln | Asp |
| Gly(G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe(F) | Trp; Leu; Val; Ile; Ala; Tyr | Tyr |
| Pro (P) | Ala | Ala |
| Ser(S) | Thr | Thr |
| Thr(T) | Val; Ser | Ser |
| Trp(W) | Tyr; Phe | Tyr |
| Tyr(Y) | Trp; Phe; Thr; Ser | Phe |
| Val (V) | Ile; Leu; Met; Phe; Ala; Norleucine | Leu |

The term antibody "effector function" refers to biological activities attributable to the Fc-region (native Fc-region sequence or Fc-region amino acid variants) of an antibody, which vary with the antibody isotype. Examples of antibody effector functions include: Cl_{q} binding and complement dependent cytotoxicity; Fc receptor binding; antibody-dependent cell-mediated cytotoxicity (ADCC); phagocytosis; down regulation of cell surface receptors (e.g., B-cell receptor, BCR), and B-cell activation.

"Antibody-dependent cellular cytotoxicity" or "ADCC" refers to a cell-mediated response, in which nonspecific cytotoxic cells that express Fc receptors (FcR) (for example, natural killer (NK) cells, neutrophils, and macrophages) recognize bound antibody on a target cell and subsequently cause lysis or phagocytosis of the target cell. The primary cells for mediating ADCC, NK cells, express FcyRJII only, whereas monocytes express FcyRI, FcyRII and FcyRIII. FcR expression on hematopoietic cells is summarized in Table 3 on page 464 of Ravetch and Kinet, Annu. Rev. Immunol 9: 457-92 (1991). To assess ADCC activity of a molecule of interest, an *in vitro* ADCC assay, such as that described in U.S. Patent Nos. 5,500,362 or 5,821,337 may be performed. Useful effector cells for such assays include peripheral blood mononuclear cells (PBMC) and Natural Killer (NK) cells. Alternatively, or additionally, ADCC activity of the molecule of interest may be assessed *in vivo,* e.g., in an animal model such as that disclosed in Clynes et al. PNAS (USA) 95: 652-656 (1998).

"Human effector cells" are leukocytes which express one or more FcRs and perform effector functions. Preferably, the cells express at least FcyRIII and perform ADCC effector function. Examples of human leukocytes which mediate ADCC include peripheral blood mononuclear cells (PBMC), natural killer (NK) cells, monocytes, cytotoxic T cells and neutrophils; with PBMCs and NK cells being preferred. The effector cells may be isolated from a native source thereof, e.g., from blood or PBMCs as described herein.

"Complement dependent cytotoxicity" and "CDC" refer to the ability of a molecule to lyse a target in the presence of complement. The complement activation pathway is initiated by the binding of the first component of the complement system (C1q) to a molecule {e.g., an antibody) complexed with a cognate antigen. To assess complement activation, a CDC assay, e.g., as described in Gazzano-Santoro et al., J. Immunol. Methods 202: 163 (1996).

In some embodiments of the invention, the bivalent bispecific chimeric antibody specifically binds to CD20 and CD3.

In some embodiments of the invention, the bivalent bispecific chimeric antibody specifically binds to BCMA and CD3.

In some embodiments of the invention, the bivalent bispecific chimeric antibody specifically binds to PD-L1 and CD47.

In some embodiments of the invention, the bivalent bispecific chimeric antibody specifically binds to coagulation factor 9 (FIX) and coagulation factor 10 (FX).

In some embodiments of the invention, the bivalent bispecific chimeric antibody specifically binds to GD2 and CD3.

In some embodiments of the invention, the bivalent bispecific chimeric antibody specifically binds to AXL and CD3.

In some embodiments of the invention, the bivalent bispecific chimeric antibody specifically binds to PD-L1 and TGF beta.

The application materials provide the following antibodies and antibody-like molecules: 01-001, 01-002, 01-003, 01-004, 01-005, 01-006, 01-007, 01-008, 01-009, 01-010, 01-011, 01-012, 02-004, 02-005, 02-006, 02-007, 02-008, 02-009, 03-001, 03-002, 03-003, 03-004, 03-005, 03-006, 03-007, 03-008.

01-001 is a model monospecific antibody-like molecule that includes a heavy chain based on VH and CH1, as well as a light chain based on VL and CD1b, wherein VH is the amino acid sequence of the heavy chain variable domain of antibody Prolgolimab (Prolgolimab_VH) with SEQ ID NO: 103, VL is the amino acid sequence of the light chain variable domain of the antibody Prolgolimab (Prolgolimab_VL) with SEQ ID NO: 104, and CD1b is the α3 domain of CD1b that has the amino acid sequence of SEQ ID NO: 109.

01-002 is a model monospecific antibody-like molecule that includes a heavy chain based on VH and b2M and a light chain based on VL and CK, wherein VH is the amino acid sequence of the heavy chain variable domain of antibody Prolgolimab (Prolgolimab_VH) with SEQ ID NO: 103, b2M is β2 microglobulin that has the amino acid sequence of SEQ ID NO: 46, and VL is the amino acid sequence of the light chain variable domain of antibody Prolgolimab (Prolgolimab_VL) with SEQ ID NO: 104.

01-003 is a model monospecific antibody-like molecule that includes a heavy chain based on VH and CH1, as well as a light chain based on VL and CD1b, wherein VH is the amino acid sequence of the heavy chain variable domain of antibody Ocrelizumab (Ocrelizumab VH) with SEQ ID NO: 105, VL is the amino acid sequence of the light chain variable domain of antibody Ocrelizumab (Ocrelizumab VH) with SEQ ID NO: 106, and CD1b is the α3 domain of CD1b that has the amino acid sequence of SEQ ID NO: 109.

01-004 is a model monospecific antibody-like molecule that includes a heavy chain based on VH and CH1 and a light chain based on VL and CK, wherein VH is the amino acid sequence of the heavy chain variable domain of antibody Prolgolimab (Prolgolimab_VH) with SEQ ID NO: 103, and VL is the amino acid sequence of the light chain variable domain of antibody Ocrelizumab (Ocrelizumab VL) with SEQ ID NO: 106.

01-005 is a model monospecific antibody-like molecule that includes a heavy chain based on VH and CH1, as well as a light chain based on VL and b2M, wherein VH is the amino acid sequence of the heavy chain variable domain of antibody Prolgolimab (Prolgolimab_VH) with SEQ ID NO: 103, b2M is β2 microglobulin that has the amino acid sequence of SEQ ID NO: 46, VL is the amino acid sequence of the light chain variable domain of antibody Ocrelizumab (Ocrelizumab VL) with SEQ ID NO: 106, and CD1b is the α3 domain of CD1b that has the amino acid sequence of SEQ ID NO: 109.

01-006 is a model monospecific antibody-like molecule that includes a heavy chain based on VH and CH1 and a light chain based on VL and CK, wherein VH is the amino acid sequence of the heavy chain variable domain of antibody Ocrelizumab (Ocrelizumab VH) with SEQ ID NO: 105, and VL is the amino acid sequence of the light chain variable domain of antibody Prolgolimab (Prolgolimab_VL) with SEQ ID NO: 104.

01-007 is a model monospecific antibody-like molecule that includes a heavy chain based on VH and CH1, as well as a light chain based on VL and b2M, wherein VH is the amino acid sequence of the heavy chain variable domain of antibody Ocrelizumab (Ocrelizumab VH) with SEQ ID NO: 105, b2M is β2 microglobulin that has the amino acid sequence of SEQ ID NO: 46, VL is the amino acid sequence of the light chain variable domain of antibody Prolgolimab (Prolgolimab_VL) with SEQ ID NO: 104, and CD1b is the α3 domain of CD1b that has the amino acid sequence of SEQ ID NO: 109.

01-008 is a model monospecific antibody-like molecule that includes a heavy chain based on VH and CH1, as well as a light chain based on VL and CD1b, wherein VH is the amino acid sequence of the heavy chain variable domain of antibody Prolgolimab (Prolgolimab_VH) with SEQ ID NO: 103, VL is the amino acid sequence of the light chain variable domain of antibody Ocrelizumab (Ocrelizumab VH) with SEQ ID NO: 106, and CD1b is the α3 domain of CD1b that has the amino acid sequence of SEQ ID NO: 109.

01-009 is a model monospecific antibody-like molecule that includes a heavy chain based on VH and b2M and a light chain based on VL and CK, wherein VH is the amino acid sequence of the heavy chain variable domain of antibody Prolgolimab (Prolgolimab_VH) with SEQ ID NO: 103, b2M is β2 microglobulin that has the amino acid sequence of SEQ ID NO: 46, and VL is the amino acid sequence of the light chain variable domain of antibody Ocrelizumab (Ocrelizumab VL) with SEQ ID NO: 106.

01-010 is a model monospecific antibody-like molecule that includes a heavy chain based on VH and CH1, as well as a light chain based on VL and CD1b, wherein VH is the amino acid sequence of the heavy chain variable domain of antibody Ocrelizumab (Ocrelizumab VH) with SEQ ID NO: 105, VL is the amino acid sequence of the light chain variable domain of the antibody Prolgolimab (Prolgolimab_VL) with SEQ ID NO: 104, and CD1b is the α3 domain of CD1b that has the amino acid sequence of SEQ ID NO: 109.

01-011 is a model monospecific antibody-like molecule that includes a heavy chain based on VH and CH1, as well as a light chain based on VL and b2M, wherein VH is the amino acid sequence of the heavy chain variable domain of antibody Prolgolimab (Prolgolimab_VH) with SEQ ID NO: 103, b2M is β2 microglobulin that has the amino acid sequence of SEQ ID NO: 46, VL is the amino acid sequence of the light chain variable domain of antibody Prolgolimab (Prolgolimab_VL) with SEQ ID NO: 104, and CD1b is the α3 domain of CD1b that has the amino acid sequence of SEQ ID NO: 109.

01-012 is a model monospecific antibody-like molecule that includes a heavy chain based on VH and CH1 and a light chain based on VL and CK, wherein VH is the amino acid sequence of the heavy chain variable domain of antibody Prolgolimab (Prolgolimab_VH) with SEQ ID NO: 103, and VL is the amino acid sequence of the light chain variable domain of antibody Prolgolimab (Prolgolimab_VL) with SEQ ID NO: 104.

02-004 is a bivalent bispecific chimeric antibody that specifically binds to PD1 and CD20 and comprises:
a) a first light chain and a first heavy chain of antibody specifically binding to PD1;
   wherein the first light chain comprises the light chain variable domain of antibody Prolgolimab (Prolgolimab_VL) with SEQ ID NO: 104 and a light chain constant domain;
   wherein the first heavy chain comprises the heavy chain variable domain of antibody Prolgolimab (Prolgolimab_VH) with SEQ ID NO: 103 and the heavy chain constant domains of the antibody that include a first (CH1) heavy chain constant domain and an Fc fragment monomer comprising second (CH2) and third (CH3) heavy chain constant domains, wherein CH3 includes amino acid substitutions to form Hole;
b) the second light chain and the second heavy chain of antibody specifically binding to CD20,
   wherein the second light chain comprises the light chain variable domain of antibody Ocrelizumab (Ocrelizumab VL) with SEQ ID NO: 106 and the α3 membrane-proximal domain of CD1b that has the amino acid sequence of SEQ ID NO: 109;
   wherein the second heavy chain comprises the heavy chain variable domain of antibody Ocrelizumab (Ocrelizumab VH) with SEQ ID NO: 105, a membrane-proximal domain of β2 microglobulin that has the amino acid sequence of SEQ ID NO: 46, and an Fc fragment monomer comprising second (CH2) and third (CH3) heavy chain constant domains, wherein CH3 includes amino acid substitutions to form Knob.
   02-004 is a bivalent bispecific chimeric antibody that specifically binds to PD1 and CD20 and comprises:
      a) a first light chain and a first heavy chain of antibody specifically binding to PD1;
         wherein the first light chain comprises the amino acid sequence of SEQ ID NO: 58 (Prolgolimab_VL_CK);
         wherein the first heavy chain comprises the amino acid sequence of SEQ ID NO: 57 (Prolgolimab_VH_HC_hole);
      b) the second light chain and the second heavy chain of antibody specifically binding to CD20,
         wherein the second light chain comprises the amino acid sequence of SEQ ID NO: 60 (Ocrelizumab_VL_CD1b);
         wherein the second heavy chain comprises the amino acid sequence of SEQ ID NO: 59 (Ocrelizumab_VH_b2m_Fc_knob).
   02-005 is a bivalent bispecific chimeric antibody that specifically binds to PD1 and CD20 and comprises:
      a) a first light chain and a first heavy chain of antibody specifically binding to CD20;
         wherein the first light chain comprises the light chain variable domain of antibody Ocrelizumab (Ocrelizumab VL) with SEQ ID NO: 106 and a light chain constant domain;
         wherein the first heavy chain comprises the heavy chain variable domain of antibody Ocrelizumab (Ocrelizumab VH) with SEQ ID NO: 105 and the heavy chain constant domains of the antibody that include a first (CH1) heavy chain constant domain and an Fc fragment monomer comprising second (CH2) and third (CH3) heavy chain constant domains, wherein CH3 includes amino acid substitutions to form Hole;
      b) a second light chain and a second heavy chain of antibody specifically binding to PD1,
         wherein the second light chain comprises the light chain variable domain of antibody Prolgolimab (Prolgolimab_VL) with SEQ ID NO: 104 and the α3 membrane-proximal domain of CD1b that has the amino acid sequence of SEQ ID NO: 109;
         wherein the second heavy chain comprises the heavy chain variable domain of antibody Prolgolimab (Prolgolimab_VH) with SEQ ID NO: 103, a membrane-proximal domain of β2 microglobulin that has the amino acid sequence of SEQ ID NO: 46, and an Fc fragment monomer comprising second (CH2) and third (CH3) heavy chain constant domains, wherein CH3 includes amino acid substitutions to form Knob.
   02-005 is a bivalent bispecific chimeric antibody that specifically binds to PD1 and CD20 and comprises:
      a) a first light chain and a first heavy chain of antibody specifically binding to CD20;
         wherein the first light chain comprises the amino acid sequence of SEQ ID NO: 62 (Ocrelizumab_VL_CK);
         wherein the first heavy chain comprises the amino acid sequence of SEQ ID NO: 61 (Ocrelizumab_VH_HC_hole);
      b) a second light chain and a second heavy chain of antibody specifically binding to PD1,
         wherein the second light chain comprises the amino acid sequence of SEQ ID NO: 64 (Prolgolimab_VL_CD1b);
         wherein the second heavy chain comprises the amino acid sequence of SEQ ID NO: 63 (Prolgolimab_VH_b2m_Fc_knob).
   02-006 is a bivalent bispecific chimeric antibody that specifically binds to PD1 and CSF1R and comprises:
      a) a first light chain and a first heavy chain of antibody specifically binding to PD1;
         wherein the first light chain comprises the light chain variable domain of antibody Prolgolimab (Prolgolimab_VL) with SEQ ID NO: 104 and a light chain constant domain;
         wherein the first heavy chain comprises the heavy chain variable domain of antibody Prolgolimab (Prolgolimab_VH) with SEQ ID NO: 103 and the heavy chain constant domains of the antibody that include a first (CH1) heavy chain constant domain and an Fc fragment monomer comprising second (CH2) and third (CH3) heavy chain constant domains, wherein CH3 includes amino acid substitutions to form Hole;
      b) a second light chain and a second heavy chain of antibody specifically binding to CSF1R,
         wherein the second light chain comprises the light chain variable domain of antibody to CSF1R with SEQ ID NO: 108 and the α3 membrane-proximal domain of CD1b that has the amino acid sequence of SEQ ID NO: 109;
         wherein the second heavy chain comprises the heavy chain variable domain of antibody to CSF1R with SEQ ID NO: 107, a membrane-proximal domain of β2 microglobulin that has the amino acid sequence of SEQ ID NO: 46, and an Fc fragment monomer comprising second (CH2) and third (CH3) heavy chain constant domains, wherein CH3 includes amino acid substitutions to form Knob.
   02-006 is a bivalent bispecific chimeric antibody that specifically binds to PD1 and CSF1R and comprises:
      a) a first light chain and a first heavy chain of antibody specifically binding to PD1;
         wherein the first light chain comprises the amino acid sequence of SEQ ID NO: 58 (Prolgolimab_VL_CK);
         wherein the first heavy chain comprises the amino acid sequence of SEQ ID NO: 57 (Prolgolimab_VH_HC_hole);
      b) a second light chain and a second heavy chain of antibody specifically binding to CSF1R,
         wherein the second light chain comprises the amino acid sequence of SEQ ID NO: 66 (Anti-CSF1R_VL_CD1b);
         wherein the second heavy chain comprises the amino acid sequence of SEQ ID NO: 65 (Anti-CSF1R_VH_b2m_Fc_knob).
   02-007 is a bivalent bispecific chimeric antibody that specifically binds to PD1 and CSF1R and comprises:
      a) a first light chain and a first heavy chain of antibody specifically binding to CSF1R;
         wherein the first light chain comprises the light chain variable domain of antibody to CSF1R with SEQ ID NO: 108 and a light chain constant domain;
         wherein the first heavy chain comprises the heavy chain variable domain of antibody to CSF1R with SEQ ID NO: 107 and the heavy chain constant domains of the antibody that include a first (CH1) heavy chain constant domain and an Fc fragment monomer comprising second (CH2) and third (CH3) heavy chain constant domains, wherein CH3 includes amino acid substitutions to form Hole;
      b) a second light chain and a second heavy chain of antibody specifically binding to PD1,
         wherein the second light chain comprises the light chain variable domain of antibody Prolgolimab (Prolgolimab_VL) with SEQ ID NO: 104 and the α3 membrane-proximal domain of CD1b that has the amino acid sequence of SEQ ID NO: 109;
         wherein the second heavy chain comprises the heavy chain variable domain of antibody Prolgolimab (Prolgolimab_VH) with SEQ ID NO: 103, a membrane-proximal domain of β2 microglobulin that has the amino acid sequence of SEQ ID NO: 46, and an Fc fragment monomer comprising second (CH2) and third (CH3) heavy chain constant domains, wherein CH3 includes amino acid substitutions to form Knob.
   02-007 is a bivalent bispecific chimeric antibody that specifically binds to PD1 and CSF1R and comprises:
      a) a first light chain and a first heavy chain of antibody specifically binding to CSF1R;
         wherein the first light chain comprises the amino acid sequence of SEQ ID NO: 68 (Anti-CSF1R_VL_CK);
         wherein the first heavy chain comprises the amino acid sequence of SEQ ID NO: 67 (Anti-CSF1R_VH_HC_hole);
      b) a second light chain and a second heavy chain of antibody specifically binding to PD1,
         wherein the second light chain comprises the amino acid sequence of SEQ ID NO: 64 (Prolgolimab_VL_CD1b);
         wherein the second heavy chain comprises the amino acid sequence of SEQ ID NO: 63 (Prolgolimab_VH_b2m_Fc_knob).
   02-008 is a bivalent bispecific chimeric antibody that specifically binds to CD20 and CSF1R and comprises:
      a) a first light chain and a first heavy chain of antibody specifically binding to CD20;
         wherein the first light chain comprises the light chain variable domain of antibody Ocrelizumab (Ocrelizumab VL) with SEQ ID NO: 106 and a light chain constant domain;
         wherein the first heavy chain comprises the heavy chain variable domain of antibody Ocrelizumab (Ocrelizumab VH) with SEQ ID NO: 105 and the heavy chain constant domains of the antibody that include a first (CH1) heavy chain constant domain and an Fc fragment monomer comprising second (CH2) and third (CH3) heavy chain constant domains, wherein CH3 includes amino acid substitutions to form Hole;
      b) a second light chain and a second heavy chain of antibody specifically binding to CSF1R,
         wherein the second light chain comprises the light chain variable domain of antibody to CSF1R with SEQ ID NO: 108 and the α3 membrane-proximal domain of CD1b that has the amino acid sequence of SEQ ID NO: 109;
         wherein the second heavy chain comprises the heavy chain variable domain of antibody to CSF1R with SEQ ID NO: 107, a membrane-proximal domain of β2 microglobulin that has the amino acid sequence of SEQ ID NO: 46, and an Fc fragment monomer comprising second (CH2) and third (CH3) heavy chain constant domains, wherein CH3 includes amino acid substitutions to form Knob.
   02-008 is a bivalent bispecific chimeric antibody that specifically binds to CD20 and CSF1R and comprises:
      a) a first light chain and a first heavy chain of antibody specifically binding to CD20;
         wherein the first light chain comprises the amino acid sequence of SEQ ID NO: 62 (Ocrelizumab_VL_CK);
         wherein the first heavy chain comprises the amino acid sequence of SEQ ID NO: 61 (Ocrelizumab_VH_HC_hole);
      b) a second light chain and a second heavy chain of antibody specifically binding to CSF1R,
         wherein the second light chain comprises the amino acid sequence of SEQ ID NO: 66 (Anti-CSF1R_VL_CD1b);
         wherein the second heavy chain comprises the amino acid sequence of SEQ ID NO: 65 (Anti-CSF1R_VH_b2m_Fc_knob).
   02-009 is a bivalent bispecific chimeric antibody that specifically binds to CD20 and CSF1R and comprises:
      a) a first light chain and a first heavy chain of antibody specifically binding to CSF1R;
         wherein the first light chain comprises the light chain variable domain of antibody to CSF1R with SEQ ID NO: 108 and a light chain constant domain;
         wherein the first heavy chain comprises the heavy chain variable domain of antibody to CSF1R with SEQ ID NO: 107 and the heavy chain constant domains of the antibody that include a first (CH1) heavy chain constant domain and an Fc fragment monomer comprising second (CH2) and third (CH3) heavy chain constant domains, wherein CH3 includes amino acid substitutions to form Hole;
      b) the second light chain and the second heavy chain of antibody specifically binding to CD20,
         wherein the second light chain comprises the light chain variable domain of antibody Ocrelizumab (Ocrelizumab VL) with SEQ ID NO: 106 and the α3 membrane-proximal domain of CD1b that has the amino acid sequence of SEQ ID NO: 109;
         wherein the second heavy chain comprises the heavy chain variable domain of antibody Ocrelizumab (Ocrelizumab VH) with SEQ ID NO: 105, a membrane-proximal domain of β2 microglobulin that has the amino acid sequence of SEQ ID NO: 46, and an Fc fragment monomer comprising second (CH2) and third (CH3) heavy chain constant domains, wherein CH3 includes amino acid substitutions to form Knob.
   02-009 is a bivalent bispecific chimeric antibody that specifically binds to CD20 and CSF1R and comprises:
      a) a first light chain and a first heavy chain of antibody specifically binding to CSF1R;
         wherein the first light chain comprises the amino acid sequence of SEQ ID NO: 68 (Anti-CSF1R_VL_CK);
         wherein the first heavy chain comprises the amino acid sequence of SEQ ID NO: 67 (Anti-CSF1R_VH_HC_hole);
      b) the second light chain and the second heavy chain of antibody specifically binding to CD20,
         wherein the second light chain comprises the amino acid sequence of SEQ ID NO: 60 (Ocrelizumab_VL_CD1b);
         wherein the second heavy chain comprises the amino acid sequence of SEQ ID NO: 59 (Ocrelizumab_VH_b2m_Fc_knob).
   03-001 is a bivalent monospecific chimeric antibody that specifically binds to PD1 and comprises:
      a light chain that comprises the light chain variable domain of antibody Prolgolimab (Prolgolimab_VL) with SEQ ID NO: 104 and the α3 membrane-proximal domain of CD1b with the mutation N57C (additional SS bridge inside the unit) and elongation by 3 amino acids GSC at the C terminus, which has the amino acid sequence of SEQ ID NO: 49;
      a heavy chain that comprises the heavy chain variable domain of antibody Prolgolimab (Prolgolimab_VH) with SEQ ID NO: 103, a membrane-proximal domain of β2 microglobulin with the mutation R12C, which has the amino acid sequence of SEQ ID NO: 47, and an Fc fragment monomer comprising second (CH2) and third (CH3) heavy chain constant domains.
   03-001 is a bivalent monospecific chimeric antibody that specifically binds to PD1 and comprises:
      a light chain that comprises the amino acid sequence of SEQ ID NO: 72 and
      a heavy chain that comprises the amino acid sequence of SEQ ID NO: 69.
   03-002 is a bivalent monospecific chimeric antibody that specifically binds to PD1 and comprises:
      a light chain that comprises the light chain variable domain of antibody Prolgolimab (Prolgolimab VL) with SEQ ID NO: 104 and the α3 membrane-proximal domain of CD1b with the mutation N57C (the SS bridge is inside the unit) and elongation by 2 GS amino acids at the C terminus (the SS bridge has been removed from the C-terminus of the unit and transferred inside the unit), which has the amino acid sequence of SEQ ID NO: 50;
      a heavy chain that comprises the heavy chain variable domain of antibody Prolgolimab (Prolgolimab_VH) with SEQ ID NO: 103, a membrane-proximal domain of β2 microglobulin with the mutation R12C and a hinge with the mutation C220A, which have the amino acid sequence of SEQ ID NO: 48 and an Fc fragment monomer comprising second (CH2) and third (CH3) heavy chain constant domains.
   03-002 is a bivalent monospecific chimeric antibody that specifically binds to PD1 and comprises:
      a light chain that comprises the amino acid sequence of SEQ ID NO: 73 and
      a heavy chain that comprises the amino acid sequence of SEQ ID NO: 71.
   03-003 is a bivalent monospecific chimeric antibody that specifically binds to PD1 and comprises:
      a light chain that comprises the light chain variable domain of antibody Prolgolimab (Prolgolimab_VL) with SEQ ID NO: 104 and the α3 membrane-proximal domain of CD1b with the mutation N59A (Substitution in the predicted N-glycosylation site on the light chain) and elongation by 3 amino acids GSC at the C terminus, which has the amino acid sequence of SEQ ID NO: 51;
      a heavy chain that comprises the heavy chain variable domain of antibody Prolgolimab (Prolgolimab_VH) with SEQ ID NO: 103, a membrane-proximal domain of β2 microglobulin that has the amino acid sequence of SEQ ID NO: 46, and an Fc fragment monomer comprising second (CH2) and third (CH3) heavy chain constant domains.
   03-003 is a bivalent monospecific chimeric antibody that specifically binds to PD1 and comprises:
      a light chain that comprises the amino acid sequence of SEQ ID NO: 74 and
      a heavy chain that comprises the amino acid sequence of SEQ ID NO: 70.
   03-004 is a bivalent monospecific chimeric antibody that specifically binds to PD1 and comprises:
      a light chain that comprises the light chain variable domain of antibody Prolgolimab (Prolgolimab_VL) with SEQ ID NO: 104 and the α3 membrane-proximal domain of CD1b with the mutation N59D (Substitution in the predicted N-glycosylation site on the light chain) and elongation by 3 amino acids GSC at the C terminus, which has the amino acid sequence of SEQ ID NO: 52;
      a heavy chain that comprises the heavy chain variable domain of antibody Prolgolimab (Prolgolimab_VH) with SEQ ID NO: 103, a membrane-proximal domain of β2 microglobulin that has the amino acid sequence of SEQ ID NO: 46, and an Fc fragment monomer comprising second (CH2) and third (CH3) heavy chain constant domains.
   03-004 is a bivalent monospecific chimeric antibody that specifically binds to PD1 and comprises:
      a light chain that comprises the amino acid sequence of SEQ ID NO: 75 and
      a heavy chain that comprises the amino acid sequence of SEQ ID NO: 70.
   03-005 is a bivalent monospecific chimeric antibody that specifically binds to PD1 and comprises:
      a light chain that comprises the light chain variable domain of antibody Prolgolimab (Prolgolimab_VL) with SEQ ID NO: 104 and the α3 membrane-proximal domain of CD1b with the mutations N57C, N59A and an elongation by 3 amino acids GSC at the C-terminus (a further SS bridge is inside the unit + the predicted N-glycosylation site has been removed) that has the amino acid sequence of SEQ ID NO: 53;
      a heavy chain that comprises the heavy chain variable domain of antibody Prolgolimab (Prolgolimab_VH) with SEQ ID NO: 103, a membrane-proximal domain of β2 microglobulin with the mutation R12C, which has the amino acid sequence of SEQ ID NO: 47, and an Fc fragment monomer comprising second (CH2) and third (CH3) heavy chain constant domains.
   03-005 is a bivalent monospecific chimeric antibody that specifically binds to PD1 and comprises:
      a light chain that comprises the amino acid sequence of SEQ ID NO: 76 and
      a heavy chain that comprises the amino acid sequence of SEQ ID NO: 69.
   03-006 is a bivalent monospecific chimeric antibody that specifically binds to PD1 and comprises:
      a light chain that comprises the light chain variable domain of antibody Prolgolimab (Prolgolimab_VL) with SEQ ID NO: 104 and the α3 membrane-proximal domain of CD1b with the mutations N57C, N59D and an elongation by 3 amino acids GSC at the C-terminus (a further SS bridge is inside the unit + the predicted N-glycosylation site has been removed) that has the amino acid sequence of SEQ ID NO: 54;
      a heavy chain that comprises the heavy chain variable domain of antibody Prolgolimab (Prolgolimab_VH) with SEQ ID NO: 103, a membrane-proximal domain of β2 microglobulin with the mutation R12C, which has the amino acid sequence of SEQ ID NO: 47, and an Fc fragment monomer comprising second (CH2) and third (CH3) heavy chain constant domains.
   03-006 is a bivalent monospecific chimeric antibody that specifically binds to PD1 and comprises:
      a light chain that comprises the amino acid sequence of SEQ ID NO: 77 and
      a heavy chain that comprises the amino acid sequence of SEQ ID NO: 69.
   03-007 is a bivalent monospecific chimeric antibody that specifically binds to PD1 and comprises:
      a light chain that comprises the light chain variable domain of antibody Prolgolimab (Prolgolimab_VL) with SEQ ID NO: 104 and the α3 membrane-proximal domain of CD1b with the mutations N57C, N59A and an elongation by 2 GS amino acids at the C terminus (the SS bridge has been transferred inside the unit + the predicted N-glycosylation site has been removed) that has the amino acid sequence of SEQ ID NO: 55;
      a heavy chain that comprises the heavy chain variable domain of antibody Prolgolimab (Prolgolimab_VH) with SEQ ID NO: 103, a membrane-proximal domain of β2 microglobulin with the mutation R12C and a hinge with the mutation C220A, which have the amino acid sequence of SEQ ID NO: 48 and an Fc fragment monomer comprising second (CH2) and third (CH3) heavy chain constant domains.
   03-007 is a bivalent monospecific chimeric antibody that specifically binds to PD1 and comprises:
      a light chain that comprises the amino acid sequence of SEQ ID NO: 78 and
      a heavy chain that comprises the amino acid sequence of SEQ ID NO: 71.
   03-008 is a bivalent monospecific chimeric antibody that specifically binds to PD1 and comprises:
      a light chain that comprises the light chain variable domain of antibody Prolgolimab (Prolgolimab_VL) with SEQ ID NO: 104 and the α3 membrane-proximal domain of CD1b with the mutations N57C, N59D and an elongation by 2 GS amino acids at the C terminus (the SS bridge has been transferred inside the unit + the predicted N-glycosylation site has been removed) that has the amino acid sequence of SEQ ID NO: 56;
      a heavy chain that comprises the heavy chain variable domain of antibody Prolgolimab (Prolgolimab_VH) with SEQ ID NO: 103, a membrane-proximal domain of β2 microglobulin with the mutation R12C and a hinge with the mutation C220A, which have the amino acid sequence of SEQ ID NO: 48 and an Fc fragment monomer comprising second (CH2) and third (CH3) heavy chain constant domains.
   03-008 is a bivalent monospecific chimeric antibody that specifically binds to PD1 and comprises:
      a light chain that comprises the amino acid sequence of SEQ ID NO: 79 and
      a heavy chain that comprises the amino acid sequence of SEQ ID NO: 71.

These antibodies are given for illustrative purposes to confirm the operability of the bivalent bispecific chimeric antibody format according to the invention, as well as to confirm surprising properties thereof. These antibodies should not be construed as somehow limiting the bivalent bispecific chimeric antibody according to the invention.

The yield parameters of the product with the correct heterodimeric assembly of two distinct heavy chains and the correct pairing between two distinct light chains and the corresponding heavy chains do not depend on the heavy and light chain variable fragments of the bispecific chimeric antibody and specificity thereof for antigens.

The bivalent bispecific chimeric antibodies according to the invention may be used to treat various diseases, in particular, oncological diseases, autoimmune diseases or diseases that are associated with blood clotting (coagulation) disorder.

### Nucleic acid

In one aspect, the present invention relates to an isolated nucleic acid that encodes any of the above bivalent bispecific chimeric antibodies or fragments thereof.

The terms "nucleic acid", "nucleic sequence", "nucleic acid sequence", "polynucleotide", "oligonucleotide", "polynucleotide sequence" and "nucleotide sequence", used interchangeably in the present description, mean a precise sequence of nucleotides, modified or not, determining a fragment or a region of a nucleic acid, containing unnatural nucleotides or not, and being either a double-strand DNA or RNA, a single-strand DNA or RNA, or transcription products of said DNAs.

It should also be included here that the present invention does not relate to nucleotide sequences in their natural chromosomal environment, i.e. in a natural state. The sequences of the present invention have been isolated and/or purified, i.e., they were sampled directly or indirectly, for example by copying, their environment having been at least partially modified. Thus, isolated nucleic acids obtained by recombinant genetics, by means, for example, of host cells, or obtained by chemical synthesis should also be mentioned here.

A reference to a nucleotide sequence encompasses the complement thereof unless otherwise specified. Thus, a reference to a nucleic acid having a particular sequence should be understood as one which encompasses the complementary strand thereof with the complementary sequence thereof.

In any of the above embodiments, the nucleic acid molecules may be isolated.

An "isolated" nucleic acid molecule is one which is identified and separated from at least one nucleic acid molecule-impurity, which the former is bound to in the natural source of antibody nucleic acid. An isolated nucleic acid molecule is different from the form or set in which it is found under natural conditions. Thus, an isolated nucleic acid molecule is different from a nucleic acid molecule that exists in cells under natural conditions.

In one aspect, the present invention relates to a nucleic acid molecule comprising a nucleotide sequence encoding an amino acid sequence selected from SEQ ID NO: 1-79 or SEQ ID NO: 109. A nucleic acid molecule can also comprise any combination of said nucleotide sequences.

In some embodiments, a nucleic acid is DNA.

In one variant, the present invention relates to a nucleic acid molecule comprising a nucleotide sequence that encodes the light or heavy chain amino acid sequence of the above bispecific antibody according to the invention, selected from:
a first light chain amino acid sequence that comprises a light chain variable domain and a light chain constant domain;
a first heavy chain amino acid sequence that comprises a heavy chain variable domain and heavy chain constant domains of antibody that include a first (CH1) heavy chain constant domain and an Fc fragment monomer comprising second (CH2) and third (CH3) heavy chain constant domains;
a second light chain amino acid sequence that comprises a light chain variable domain and a constant domain that is selected from the group:
   a first membrane-proximal domain of MHC (major histocompatibility complex) or
   a first membrane-proximal domain of MHC-like protein;
   a second heavy chain amino acid sequence that comprises a heavy chain variable domain and a constant domain that is selected from the group:
      a second membrane-proximal domain of MHC (major histocompatibility complex) or
      a second membrane-proximal domain of MHC-like protein,
      and an Fc fragment monomer comprising second (CH2) and third (CH3) heavy chain constant domains.

A nucleic acid molecule can also comprise any combination of the above nucleotide sequences.

This skilled in the art will appreciate that a peptide comprising an amino acid sequence of the light or heavy chain of the above bispecific antibody according to the invention, selected from:
a first light chain amino acid sequence that comprises a light chain variable domain and a light chain constant domain;
a first heavy chain amino acid sequence that comprises a heavy chain variable domain and heavy chain constant domains of antibody that include a first (CH1) heavy chain constant domain and an Fc fragment monomer comprising second (CH2) and third (CH3) heavy chain constant domains;
a second light chain amino acid sequence that comprises a light chain variable domain and a constant domain that is selected from the group:
   a first membrane-proximal domain of MHC (major histocompatibility complex) or
   a first membrane-proximal domain of MHC-like protein;
   a second heavy chain amino acid sequence that comprises a heavy chain variable domain and a constant domain that is selected from the group:
      a second membrane-proximal domain of MHC (major histocompatibility complex) or
      a second membrane-proximal domain of MHC-like protein,
      and an Fc fragment monomer comprising second (CH2) and third (CH3) heavy chain constant domains,
      may be encoded by a wide range of different DNA sequences, due to the degeneracy of genetic code. It is well within the skill of a person trained in the art to create these alternative DNA sequences encoding the same amino acid sequences. Such variant DNA sequences are within the scope of the present invention.

The nucleic acid molecule according to the present invention may be isolated from any source that produces the bispecific antibody according to the invention. In certain embodiments of the invention, the nucleic acid molecule of the invention may be synthesized, rather than isolated.

In some embodiments of the invention, the nucleic acid is a nucleic acid that encodes the amino acid sequence of the first heavy chain of candidate 02-004 and 02-006 (Prolgolimab VHHChole), and includes a nucleotide sequence with SEQ ID NO: 80.

In some embodiments of the invention, the nucleic acid is a nucleic acid that encodes the amino acid sequence of the first light chain of candidate 02-004 and 02-006 (Prolgolimab_VL_CK), and includes a nucleotide sequence with SEQ ID NO: 81.

In some embodiments of the invention, the nucleic acid is a nucleic acid that encodes the amino acid sequence of the second heavy chain of candidate 02-004 and 02-009 (Ocrelizumab_VH_b2m_Fcknob), and includes a nucleotide sequence with SEQ ID NO: 82.

In some embodiments of the invention, the nucleic acid is a nucleic acid that encodes the amino acid sequence of the second light chain of candidate 02-004 and 02-009 (Ocrelizumab_VL_CD1b), and includes a nucleotide sequence with SEQ ID NO: 83.

In some embodiments of the invention, the nucleic acid is a nucleic acid that encodes the amino acid sequence of the first heavy chain of candidate 02-005 and 02-008 (Ocrelizumab_VH_HC_hole), and includes a nucleotide sequence with SEQ ID NO: 84.

In some embodiments of the invention, the nucleic acid is a nucleic acid that encodes the amino acid sequence of the first light chain of 02-005 and 02-008 candidates (Ocrelizumab VL_CK), and includes a nucleotide sequence with SEQ ID NO: 85.

In some embodiments of the invention, the nucleic acid is a nucleic acid that encodes the amino acid sequence of the second heavy chain of 02-005 and 02-007 candidates (Prolgolimab_VH_b2m_Fc_knob), and includes a nucleotide sequence with SEQ ID NO: 86.

In some embodiments of the invention, the nucleic acid is a nucleic acid that encodes the amino acid sequence of the second light chain of 02-005 and 02-007 candidates (Prolgolimab_VL_CD1b), and includes a nucleotide sequence with SEQ ID NO: 87.

In some embodiments of the invention, the nucleic acid is a nucleic acid that encodes the amino acid sequence of the second heavy chain of 02-006 and 02-008 candidates (Anti-CSF1R_VH_b2m_Fcknob), and includes a nucleotide sequence with SEQ ID NO: 88.

In some embodiments of the invention, the nucleic acid is a nucleic acid that encodes the amino acid sequence of the second light chain of 02-006 and 02-008 candidates (Anti-CSF1R_VL_CD1b), and includes a nucleotide sequence with SEQ ID NO: 89.

In some embodiments of the invention, the nucleic acid is a nucleic acid that encodes the amino acid sequence of the first heavy chain of 02-007 and 02-009 candidates (Anti-CSFlR_VH_HC_hole), and includes a nucleotide sequence with SEQ ID NO: 90.

In some embodiments of the invention, the nucleic acid is a nucleic acid that encodes the amino acid sequence of the first light chain of 02-007 and 02-009 candidates (Anti-CSF1R_VL_CK), and includes a nucleotide sequence with SEQ ID NO: 91.

In some embodiments of the invention, the nucleic acid is a nucleic acid that encodes the amino acid sequence of the heavy chain of 03-001 and 03-005 candidates, which comprises a universal β2 microglobulin (β2M) with the mutation R12C and includes a nucleotide sequence with SEQ ID NO: 92.

In some embodiments of the invention, the nucleic acid is a nucleic acid that encodes the amino acid sequence of the heavy chain of 03-003 and 03-004 candidates, which comprises a universal β2 microglobulin (β2M) and includes a nucleotide sequence with SEQ ID NO: 93.

In some embodiments of the invention, the nucleic acid is a nucleic acid that encodes the amino acid sequence of the heavy chain of 03-002, 03-007 and 03-008 candidates, which comprises a universal β2 microglobulin (β2M) with the mutation R12C and a hinge with the mutation C220A and includes a nucleotide sequence with SEQ ID NO: 94.

In some embodiments of the invention, the nucleic acid is a nucleic acid that encodes the amino acid sequence of the light chain of 03-001 candidate, which comprises the α3 membrane-proximal domain of CD1b with the mutation N57C and an elongation by 3 amino acids GSC at the C-terminus, and includes a nucleotide sequence with SEQ ID NO: 95.

In some embodiments of the invention, the nucleic acid is a nucleic acid that encodes the amino acid sequence of the light chain of 03-002 candidate, which comprises the α3 membrane-proximal domain of CD1b with the mutation N57C and an elongation by 2 amino acids GS at the C-terminus, and includes a nucleotide sequence with SEQ ID NO: 96.

In some embodiments of the invention, the nucleic acid is a nucleic acid that encodes the amino acid sequence of the light chain of 03-003 candidate, which comprises the α3 membrane-proximal domain of CD1b with the mutation N59A and an elongation by 3 amino acids GSC at the C-terminus, and includes a nucleotide sequence with SEQ ID NO: 97.

In some embodiments of the invention, the nucleic acid is a nucleic acid that encodes the amino acid sequence of the light chain of 03-004 candidate, which comprises the α3 membrane-proximal domain of CD1b with the mutation N59D and an elongation by 3 amino acids GSC at the C-terminus, and includes a nucleotide sequence with SEQ ID NO: 98.

In some embodiments of the invention, the nucleic acid is a nucleic acid that encodes the amino acid sequence of the light chain of 03-005 candidate, which comprises the α3 membrane-proximal domain of CD1b with the mutations N57C, N59A and an elongation by 3 amino acids GSC at the C-terminus, and includes a nucleotide sequence with SEQ ID NO: 99.

In some embodiments of the invention, the nucleic acid is a nucleic acid that encodes the amino acid sequence of the light chain of 03-006 candidate, which comprises the α3 membrane-proximal domain of CD1b with the mutations N57C, N59D and an elongation by 3 amino acids GSC at the C-terminus, and includes a nucleotide sequence with SEQ ID NO: 100.

In some embodiments of the invention, the nucleic acid is a nucleic acid that encodes the amino acid sequence of the light chain of 03-007 candidate, which comprises the α3 membrane-proximal domain of CD1b with the mutations N57C, N59A and an elongation by 2 amino acids GS at the C-terminus, and includes a nucleotide sequence with SEQ ID NO: 101.

In some embodiments of the invention, the nucleic acid is a nucleic acid that encodes the amino acid sequence of the light chain of 03-008 candidate, which comprises the α3 membrane-proximal domain of CD1b with the mutations N57C, N59D and an elongation by 2 amino acids GS at the C-terminus, and includes a nucleotide sequence with SEQ ID NO: 102.

The nucleic acid molecules may be used to express the bivalent bispecific chimeric antibodies according to the invention.

### Expression vector

In one aspect, the present invention relates to an expression vector comprising the above isolated nucleic acid. The present invention relates to a vector suitable for the expression of any of nucleotide sequences described herein.

The term "vector" as used herein means a nucleic acid molecule capable of transporting another nucleic acid to which it has been linked. In some embodiments of the invention, the vector is a plasmid, i.e. a circular double stranded piece of DNA into which additional DNA segments may be ligated. In some embodiments of the invention, the vector is a viral vector, wherein additional DNA segments may be ligated into the viral genome. In some embodiments of the invention, vectors are capable of autonomous replication in a host cell into which they are introduced (e.g. bacterial vectors having a site of replication origin and episomal mammalian vectors). In further embodiments of the invention, vectors (e.g. non-episomal mammalian vectors) may be integrated into the genome of a host cell upon introduction into a host cell, and thereby are replicated along with the host gene. Moreover, certain vectors are capable of directing the expression of genes to which they are operably linked. Such vectors are referred to herein as "recombinant expression vectors" (or simply, "expression vectors").

The present invention relates to vectors comprising the above nucleic acid molecules that encode any of the above bivalent bispecific antibody or structural portions thereof selected from:
a first light chain amino acid sequence that comprises a light chain variable domain and a light chain constant domain;
a first heavy chain amino acid sequence that comprises a heavy chain variable domain and heavy chain constant domains of antibody that include a first (CH1) heavy chain constant domain and an Fc fragment monomer comprising second (CH2) and third (CH3) heavy chain constant domains;
a second light chain amino acid sequence that comprises a light chain variable domain and a constant domain that is selected from the group:
   a first membrane-proximal domain of MHC (major histocompatibility complex) or
   a first membrane-proximal domain of MHC-like protein;
   a second heavy chain amino acid sequence that comprises a heavy chain variable domain and a constant domain that is selected from the group:
      a second membrane-proximal domain of MHC (major histocompatibility complex) or
      a second membrane-proximal domain of MHC-like protein,
      and an Fc fragment monomer comprising second (CH2) and third (CH3) heavy chain constant domains,
      as described herein.

Expression vectors include plasmids, retroviruses, adenoviruses, adeno-associated viruses (AAVs), plant viruses, such as cauliflower mosaic virus, tobacco mosaic virus, cosmids, YACs, EBV derived episomes, and the like. DNA molecules may be ligated into a vector such that transcriptional and translational control sequences within the vector serve their intended function of regulating the transcription and translation of the DNA. An expression vector and expression control sequences may be chosen to be compatible with the expression host cell used. DNA molecules partially or fully encoding the sequences of first and second binding domains (for example, heavy and light chain sequences where a binding domain comprises a heavy and light chain sequence) can be introduced into individual vectors. In one embodiment, any combination of said DNA molecules is introduced into the same expression vector. DNA molecules may be introduced into an expression vector by standard methods (e.g. ligation of complementary restriction sites on an antibody gene fragment and vector, or blunt end ligation if no restriction sites are present).

In some embodiments of the invention, a suitable vector is one that includes restriction sites such that any VH or VL sequence can easily be inserted and expressed, as described above. Polyadenylation and transcription termination may occur at a native chromosomal site downstream of coding regions. A recombinant expression vector can also encode a signal peptide that facilitates secretion of an antibody chain from a host cell. An antibody chain gene may be cloned into a vector such that the signal peptide is linked in-frame to the amino terminus of an immunoglobulin chain. The signal peptide may be an immunoglobulin signal peptide or a heterologous signal peptide (i.e. a signal peptide from a non-immunoglobulin protein).

In some embodiments of the invention, in addition to antibody chain genes, the recombinant vector expression of the invention can carry regulatory sequences that control the expression of antibody chain genes in a host cell. It will be understood by those skilled in the art that the design of an expression vector, including the selection of regulatory sequences, may depend on such factors as the choice of a host cell to be transformed, the level of expression of a desired protein, and so forth. Preferred control sequences for an expression host cell in mammals include viral elements that ensure high levels of protein expression in mammalian cells, such as promoters and/or enhancers derived from a retroviral LTR, cytomegalovirus (CMV) (such as a CMV promoter/enhancer), simian virus 40 (SV40) (such as a SV40 promoter/enhancer), adenovirus, (e.g. the major late promoter adenovirus (AdMLP)), polyomavirus and strong mammalian promoters such as native immunoglobulin promoter or actin promoter. Methods for expressing polypeptides in bacterial cells or fungal cells, e.g. yeast cells, are also well known in the art.

In some embodiments of the invention, in addition to antibody chain genes and regulatory sequences, the recombinant expression vectors of the invention may carry additional sequences, such as sequences that regulate replication of a vector in host cells (e.g. origins of replication) and selectable marker genes. The selectable marker gene facilitates the selection of host cells into which a vector has been introduced.

In some embodiments of the invention, the vector may include an expression control sequence. The term "expression control sequence" as used in the present description refers to polynucleotide sequences that are necessary to effect the expression and processing of coding sequences to which they are ligated. Expression control sequences include appropriate transcription initiation, termination, promoter and enhancer sequences; efficient RNA processing signals such as splicing and polyadenylation signals; sequences that stabilize cytoplasmic mRNA; sequences that enhance translation efficiency (i.e., Kozak consensus sequence); sequences that enhance protein stability; and when desired, sequences that enhance protein secretion. The nature of such control sequences differs depending upon the host organism; in prokaryotes, such control sequences generally include the promoter of ribosome binding site, and transcription termination sequences; in eukaryotes, typically, such control sequences include promoters and transcription termination sequences. The term "control sequences" includes at least all components, the presence of which is essential for expression and processing, and can also include additional components, the presence of which is advantageous, for example, leader sequences and fusion partner sequences.

### Host cells and method for production thereof

In one aspect, the present invention relates to a method for producing a host cell for producing any of the above bivalent bispecific chimeric antibodies and includes transformation of a cell with the above expression vector.

In one aspect, the present invention relates to a host cell for producing any of the above bivalent bispecific chimeric antibodies that comprises any of the above nucleic acids.

The term "recombinant host cell" (or simply "host cell") as used herein refers to a cell into which a recombinant expression vector has been introduced. The present invention relates to host cells, which may include, for example, a vector according to the invention described above.

The present invention further relates to host cells that include, for example, a nucleotide sequence encoding the first heavy chain of the bivalent bispecific chimeric antibody according to the invention, a nucleotide sequence encoding the first light chain of the bivalent bispecific chimeric antibody according to the invention, a nucleotide sequence encoding the second heavy chain of the bivalent bispecific chimeric antibody according to the invention, or a nucleotide sequence encoding the second light chain of the bivalent bispecific chimeric antibody according to the invention or all of the above four sequences. It should be understood that "recombinant host cell" and "host cell" refer not only to a particular subject cell but to the progeny of such a cell as well. Since modifications may occur in succeeding generations due to either mutation or environmental influences, such progeny may not, in fact, be identical to a parental cell; however, such cells are still included within the scope of the term "host cell" as used herein.

Nucleic acid molecules comprising a nucleotide sequence that encodes the amino acid sequence of the light chain or heavy chain of the above bispecific antibody, selected from:
a first light chain amino acid sequence that comprises a light chain variable domain and a light chain constant domain;
a first heavy chain amino acid sequence that comprises a heavy chain variable domain and heavy chain constant domains of antibody that include a first (CH1) heavy chain constant domain and an Fc fragment monomer comprising second (CH2) and third (CH3) heavy chain constant domains;
a second light chain amino acid sequence that comprises a light chain variable domain and a constant domain that is selected from the group:
   a first membrane-proximal domain of MHC (major histocompatibility complex) or
   a first membrane-proximal domain of MHC-like protein;
   a second heavy chain amino acid sequence that comprises a heavy chain variable domain and a constant domain that is selected from the group:
      a second membrane-proximal domain of MHC (major histocompatibility complex) or
      a second membrane-proximal domain of MHC-like protein;
      and an Fc fragment monomer comprising second (CH2) and third (CH3) heavy chain constant domains,
      and vectors comprising these nucleic acid molecules may be used for transfecting a suitable mammalian or cell thereof, plant or cell thereof, bacterial or yeast host cell. Transformation may be carried out by any known technique of introducing polynucleotides into a host cell. Methods for introduction of heterologous polynucleotides into mammalian cells are well known in the art and include dextranmediated transfection, cationic polymer-nucleic acid complex transfection, calcium phosphate precipitation, polybrene-mediated transfection, protoplast fusion, encapsulation of the polynucleotide(s) in liposomes, and direct microinjection of DNA into nuclei. In addition, nucleic acid molecules may be introduced into mammalian cells by viral vectors.

Mammalian cell lines used as hosts for transformation are well known in the art and include a plurality of immortalized cell lines available. These include, e.g., Chinese hamster ovary (CHO) cells, NS0 cells, SP2 cells, HEK-293T cells, FreeStyle 293 cells (Invitrogen), NIH-3T3 cells, HeLa cells, baby hamster kidney (BHK) cells, African green monkey kidney cells (COS), human hepatocellular carcinoma cells (e.g., Hep G2), A549 cells, and a number of other cell lines. Cell lines are selected by determining which cell lines have high expression levels and provide for necessary characteristics of the protein produced. Other cell lines that may be used are insect cell lines, such as Sf9 or Sf21 cells. When the recombinant expression vectors encoding the above bispecific antibody or a portion thereof are introduced into mammalian host cells, the above bispecific antibody is produced by culturing the host cells for a period of time sufficient to express the above bispecific antibody or a portion thereof according to the invention in the host cells, or, more preferably, secrete the above bispecific antibody into the culture medium in which the host cells are cultured. The above bispecific antibody may be isolated from culture medium using standard protein purification techniques. Plant host cells include e.g. *Nicotiana, Arabidopsis,* duckweed, corn, wheat, potato, etc. Bacterial host cells include *Escherichia* and *Streptomyces* species. Yeast host cells include *Schizosaccharomyces pombe, Saccharomyces cerevisiae* and Pichia pastoris.

Furthermore, level of production of the bispecific antibody of the invention from a producing cell line may be enhanced using a number of known techniques. For example, the glutamine synthetase gene expression system (the GS system) is a common approach for enhancing expression under certain conditions. The GS system is discussed in whole or part in connection with EP Nos. 0216846, 0256055, 0323997 and 0338841.

It is likely that the bispecific chimeric antibody of the invention in different cell lines or host cells will have different glycosylation patterns from each other. However, the bispecific antibody disclosed herein is part of this invention, regardless of the state of glycosylation of the binding molecules and, in general, regardless of the presence or absence of post-translational modifications.

The above host cell does not refer to a host cell produced using human embryos.

The above host cell does not refer to a host cell produced by modifying the genetic integrity of human germline cells.

### Method of producing the antibody

In one aspect, the present invention relates to a method for producing any of the above bivalent bispecific chimeric antibodies that includes the steps of:
a) transforming a host cell
   - with expression vectors comprising nucleic acid molecules encoding the first light chain and the first heavy chain of the bivalent bispecific chimeric antibody according to the invention,
   - with expression vectors comprising nucleic acid molecules encoding the second light chain and the second heavy chain of the bivalent bispecific chimeric antibody according to the invention,
b) culturing the host cell under conditions suitable for synthesis of said bivalent bispecific antibody; and
c) isolating said bivalent bispecific antibody from cell culture.

The present invention relates to methods for producing the bivalent bispecific chimeric antibodies according to the present invention. One embodiment of the invention relates to a method for producing bivalent bispecific chimeric antibodies as defined herein, comprising producing a recombinant host cell capable of expressing the bivalent bispecific chimeric antibody, culturing said host cells under conditions suitable for expression of the bivalent bispecific chimeric antibodies, and isolating the resulting bivalent bispecific chimeric antibodies. The bivalent bispecific chimeric antibody produced by such expression in such recombinant host cells is referred to herein as "bivalent bispecific chimeric antibody".

### Examples

The following examples are provided for better understanding of the invention. These examples are for purposes of illustration only and are not to be construed as limiting the scope of the invention in any manner.

All publications, patents, and patent applications cited in this specification are incorporated herein by reference. Although the foregoing invention has been described in some detail by way of illustration and example for purposes of clarity of understanding, it will be readily apparent to those of ordinary skill in the art in light of the teachings of this invention that certain changes and modifications may be made thereto without departing from the spirit or scope of the appended embodiments.

### Materials and general methods

### Recombinant DNA techniques

Standard methods were used to manipulate DNA as described in Sambrook, J. et al, Molecular cloning: A laboratory manual; Cold Spring Harbor Laboratory Press, Cold Spring Harbor, New York, 1989. The molecular biological reagents were used according to the manufacturer protocols.

### Gene synthesis

Desired gene segments were prepared from oligonucleotides made by chemical synthesis. The gene segments of 300-4,000 bp long, which were flanked by singular restriction sites, were assembled by annealing and ligation of oligonucleotides including PCR amplification and subsequently cloned via the specified restriction sites. The DNA sequences of the subcloned gene fragments were confirmed by DNA sequencing.

### DNA sequence determination

DNA sequences were determined by Sanger sequencing.

### DNA and protein sequence analysis and sequence data management

Ylab2 (Biocad) software package was used for sequence creation, mapping, analysis, annotation and illustration.

### Expression vectors

Expression plasmid variants were applied for transient expression of the subject antibodies, antibody-like proteins and antigens in eukaryotic cells (e.g. CHO cells). Beside the expression cassette for a target protein, the (plasmids) vectors contained: an origin of replication which allows replication of said plasmid in *E. coli,* genes which confer resistance in *E. coli* to various antibiotics (e.g., to ampicillin and kanamycin).

The fusion genes comprising the described antibody chains as described above were generated by PCR and/or gene synthesis and assembled with known methods and techniques by connection of the according nucleic acid segments, e.g. using unique restriction sites in the corresponding vectors. The subcloned nucleic acid sequences were verified by DNA sequencing. The necessary amounts of plasmids for transient transfection were generated in *E.coli* cell cultures and isolated using known techniques.

### Generation and purification of recombinant antigens in suspension culture of mammalian cells

Recombinant proteins were produced in established cell line cells obtained from Chinese hamster ovary cells (CHO line). Suspension culture was conducted in flasks on orbital incubator shaker using serum-free media supplemented with 8 mM L-glutamine and 1 g/l pluronic 68. For transient expression, cells (2-2.2×10⁶ cells/ml) were transfected using linear polyethyleneimine. 9 days following transfection, culture liquid was separated from cells by filtration through a 0.5/0.22 µm deep-bed filter.

Histidine-tagged proteins were purified by metal chelate chromatography. The purified proteins were filtered through 0.22 µm and stored at -70°C.

The purity of the resulting protein solution was evaluated using SDS gel electrophoresis, electrophoresis was performed in denaturing 12% polyacrylamide gel supplemented with mercaptoethanol and in native 8% polyacrylamide gel.

### Production of antibodies and antibody-like proteins in suspension culture of mammalian cells

Control antibodies, bispecific chimeric antibodies according to the invention and antibody-like proteins were produced in cells of a permanent cell line obtained from Chinese hamster ovary cells (CHO line). Suspension culture was conducted in flasks on orbital incubator shaker using serum-free media supplemented with 8 mM L-glutamine and 1 g/l pluronic 68. For transient expression, cells (2-2.2×10⁶ cells/ml) were transfected using linear polyethyleneimine. DNA/PEI ratio was 1:3/1:10. 9 days following transfection, the culture fluid was separated from the cells by filtration through a 0.5/0.22 µm deep-bed filter, and the protein titer was then measured on ForteBio using the standard methodology. The clarified culture liquid was passed through a Protein A affinity sorbent column at 10-20 mg per ml of the sorbent, the column was equilibrated with phosphate-buffered saline (PBS, pH 7.4). The column was then washed with 5 column volumes of PBS to remove non-specifically binding components. Bound protein was eluted using 0.1 M glycine buffer (pH 3). The principal protein elution peak was collected and brought to pH 6.8 - 7.0 using 1 M Tris-HCl buffer (pH 8). All stages were conducted under 110 cm/h flow rate. Protein was then dialyzed into PBS (pH 7.4) by means of dialysis, filtered (0.22 µm), transferred into tubes and stored at -70°C.

The purity of the resulting protein solution was evaluated using SDS gel electrophoresis under reducing and non-reducing conditions, as well as using size-exclusion high-performance liquid chromatography (SE HPLC).

SE HPLC was performed on a TSK-Gel G3000SWXL column, 7.8x300 mm, particle size: 5 µm, pore size: 250Å, and a TSK-Gel Guard SWxl pre-column.

### Example 1. Selection of CH1/CK domain substitution in bispecific antibody

For the correct heterodimerization of heavy and light chains in the bispecific antibody, one of the two pairs of CH1/CK domains was substituted for structurally identical domains from other proteins (Fig. 1). Suitable substitution domains were selected in three steps:
1) pre-generation of a three-dimensional model of CH1/CK domains;
2) structural alignment of the three-dimensional models of CH1/CK domains to all objects of the Protein Data Bank (www.rcsb.org) except for antibodies and T-cell receptors;
3) selection of a suitable substitution following the results of structural alignment based on the RMSD metric and the number of amino acids that form identical secondary structure elements

The first step, the pre-generation of a three-dimensional model of CH1/CK domains, is the addition of the missing atoms in the CH1/CK domains from the PDBid 4nyl structure using the Prepwizard utility from Schrodinger Suite. At the second step, the resulting model was alternately aligned to all structures from the Protein Data Bank (except for antibodies and T-cell receptors) using the Protein Structure Alignment utility from Schrodinger Suite. The degree of similarity between an arbitrary structure and the CH1/CK domain model was evaluated using two metrics as follows: 1) RMSD that reflects a root mean square deviation for atoms between one structure and another structure (polypeptide chain backbone atoms were used for calculation), 2) the number of amino acids that form identical secondary protein structure elements following the results of alignment. Table 1 shows the best results of alignment using the RMSD metric.

**Table 1. PDB structures that demonstrated the best quality of alignment to the CH1/CK domains of human antibody according to the RMSD metric. PdbID is a structure identifier in the PDB database; RMSD is the root mean square deviation of the atoms of the structure from those of the CH1/CK domain model; structure name is a header from the PDB database**

| pdbID | Amino acids from identical secondary structures | RMSD, Å | Structure name | Protein type |
|---|---|---|---|---|
| 1ktd | 101 | 3.253 | CRYSTAL STRUCTURE OF CLASS II MHC MOLECULE IEK BOUND TO PIGEON CYTOCHROME C PEPTIDE | MHC Class II |
| 4mdj | 101 | 3.269 | Immune Receptor | MHC Class II |
| 3qxd | 101 | 3.526 | F54C HLA-DR1 bound with CLIP peptide | MHC Class II |
| 1d5x | 101 | 3.551 | X-RAY CRYSTAL STRUCTURE OF HLA-DR4 COMPLEXED WITH DIPEPTIDE MIMETIC AND SEB | MHC Class II |
| 5wl1 | 103 | 3.585 | Crystal Structure of Human CD1b in Complex | MHC-like protein CD1b |
| 1gzq | 102 | 3.594 | CD1b in complex with Phophatidylinositol | MHC-like protein CD1b |
| 6c15 | 103 | 3.607 | CDlc in complex with phosphatidylcholine | MHC-like protein CDlc |
| 5j1a | 108 | 3.639 | Antigen presenting molecule | MHC-like protein CDla |
| 4mx7 | 106 | 3.647 | Structure of mouse CDld in complex with dioleoyl-phosphatidic acid | MHC-like protein of CDld |
| 4i0p | 101 | 3.689 | HLA-DO in complex with HLA-DM | MHC Class II |
| 1bx2 | 105 | 3.690 | CRYSTAL STRUCTURE OF HLA-DR2 (DRA*0101,DRB1*1501) COMPLEXED WITH A PEPTIDE FROM HUMAN MYELIN BASIC PROTEIN | MHC Class II |
| 4e0r | 108 | 3.739 | Structure of the chicken MHC class I molecule BF2*0401 | MHC class I |
| 5tw5 | 101 | 3.794 | Structure of mouse CDld with bound glycosphingolipid JJ112 | MHC-like protein of CDld |
| 3gmo | 102 | 3.814 | Structure of mouse CDld in complex with C8PhF | MHC-like protein of CDld |
| 4f7c | 107 | 3.847 | Crystal structure of bovine CD1d with bound C12-di-sulfatide | MHC-like protein of CDld |
| 1gzp | 101 | 3.858 | CD1b in complex with GM2 ganglioside | MHC-like protein CD1b |
| 1a6z | 109 | 4.041 | HFE (HUMAN) HEMOCHROMATOSIS PROTEIN | MHC-like protein of HFE |
| 1s7q | 112 | 4.069 | Crystal structures of the murine class I major histocompatibility complex H-2Kb in complex with LCMV-derived gp33 index peptide and three of its escape variants | MHC class I |

The structures having the maximum structural similarity to the CH1/CK domains according to the RMSD metric belong to major histocompatibility complex class I and II (MHC) proteins, as well as to structural analogues thereof, CD1 and HFE. Based on the comparable values of the degree of structural similarity (RMSD and the number of amino acids from identical secondary structures), any of the above proteins may be selected for substitution. For further work involving CH1/CK domain substitutions and for illustrative purposes, we chose CD1b, in particular the membrane-proximal domains thereof.

### Example 2. Selection of location of disulfide bond between heavy and light chains in antibody molecule with substitution of CH1/CK for membrane-proximal domains of MHC or MHC-like proteins.

A human IgG isotype antibody molecule consists of two light and two heavy chains. Each of the two light chains of the antibody is connected to the heavy chain via an SS-bridge, a covalent bond between sulfur atoms in cysteine residues. This bond is formed (in the case of the IgG1 isotype) by the C-terminal cysteine residue of the light chain and the hinge region N-terminus-proximal cysteine residue of the heavy chain.

The membrane-proximal domains of MHC and MHC-like proteins are connected only by non-covalent bonds and do not form SS bridges therebetween; therefore, the substitution of CH1/CK domains for the membrane-proximal domains of MHC can potentially reduce the thermal stability of the antibody molecule and the yield of the target product during generation. To avoid this, a point cysteine substitution was required in the light chain of the chimeric antibody molecule, at a position suitable for the formation of an SS bridge with a cysteine residue proximal to the hinge region N-terminus.

The position for the introduction of a cysteine residue into the membrane-proximal domain of MHC or MHC-like protein was selected based on the structural alignment of the MHC or MHC-like protein to a full-length antibody molecule. As a result of alignment, the cysteine residue was introduced into the membrane-proximal domain of MHC or MHC-like protein located at the site of the initial CL (CK) domain of the antibody, to the position proximal to the hinge region cysteine residue forming an SS-bridge with a light chain in the native IgG1 isotype antibody molecule. By analogy with the light chain of the native antibody, the introduced cysteine residue is terminal. In order for the distance between the cysteine residues to be sufficient to establish a disulfide bond, the light chain was extended by two amino acids GS. Fig. 2 schematically shows the location of the SS-bridge between the heavy and light chains in the chimeric antibody with CH1-CK domains substituted for the membrane-proximal domains of the MHC-like protein CD1b.

### Example 3

### Production of genetic constructs to generate bivalent bispecific chimeric antibodies

To produce constructs encoding sequences of the first light and first heavy chains of the antibody specifically binding to the first antigen, PCR products comprising the genes of heavy and light chain variable domains of the antibodies were generated using primers comprising restriction sites. The heavy chain variable domain was cloned into the vector pSXn-HChole-NR_VH1 using Sall/Xbal restriction sites. The light chain variable domain was cloned into the vector pSXn-CL-BR_VL1 using SalI/XbaI restriction sites.

To produce constructs encoding the sequences of second light and second heavy chains of the antibody specifically binding to the second antigen, we synthesized constructs comprising the genes of heavy and light chain variable domains of the antibodies and the membrane-proximal domains of the human CD1 sequence (https://www.rcsb.org/structure/5wl1) including or free of various modifications.

The sequence was synthesized from oligonucleotides by PCR using primers comprising restriction sites. The heavy chain variable domain with the first membrane-proximal domain of the human CD1 sequence including or free of modifications was cloned into the vector pSX-FCknob-PR using SalI/XbaI restriction sites. The light chain variable domain with the second membrane-proximal domain of the human CD1 sequence including or free of modifications was cloned into the vector pSX-HR using Sal1/Xba1 restriction sites.

The above four vectors were combined at the transfection step to produce the bivalent bispecific chimeric antibodies according to the invention.

The required quantities of all of the above plasmids were generated in *E.Coli* cells and purified using Maxiprep Qiagen kit.

### Example 4. Effect of introduced disulfide bond on assembly of full-length chimeric antibody.

To test the operability of the introduced disulfide bond, we generated model monospecific chimeric antibodies. This format is an antibody consisting of two heavy and two light chains, wherein the heavy chain CH1 and the light chain CK domains have been substituted for β2 microglobulin and the α3 domain of the CD1b protein, respectively.

The sequences of VH (SEQ ID NO: 103) and VL (SEQ ID NO: 104) variable domains were obtained from the antibody Prolgolimab (CJSC Biocad). There was no disulfide bond between the heavy and light chains in the first variant of the sample. In the second variant there was added cysteine to the light chain C-terminus (as described in Example 2); the cysteine is considered to form a disulfide bond with the cysteine of the upper hinge region, thereby stabilizing the heavy and light chain heterodimer.

Fig. 3 shows the result of non-reducing SDS gel electrophoresis of the generated samples. Prolgolimab sample (classical IgG1) was applied on lane 3 as a control. The results show that the full-length molecule assembled only in the presence of a further disulfide bond (lane 2), whereas in the absence thereof we observed only a fragment which mobility corresponds to the heavy-chain dimer (lane 3).

We also generated a chimeric antibody with reverse orientation of dimerization units relative to heavy and light chains (the heavy chain CH1 domain and the light chain CK domain were substituted for the α3 domain of the CD1b protein and β2 microglobulin, respectively, see Fig. 1B). Fig. 3 (lane 4) shows the result of SDS gel electrophoresis for the sample under non-reducing conditions; the results confirm that this orientation also makes it possible to assemble the chimeric antibodies.

### Example 5. Verification of effect of dimerization unit based on MHC or MHC-like protein on correct pairing between heavy and light chains.

To prove the functioning of the dimerization unit based on MHC or MHC-like protein, in particular preventing incorrect pairing between light chains and inappropriate heavy chains, we conducted the following experiment involving monospecific molecules. We generated 4 groups of molecules as follows:
1. Molecules having a "correct" combination of VH and VL and an "incorrect" pair of constant domains.
2. Molecules having an "incorrect" combination of VH and VL and a "correct" pair of constant domains.
3. Molecules having an "incorrect" combination of VH and VL and an "incorrect" pair of constant domains.
4. Control molecules including a classical IgG1 format antibody and a chimeric antibody in which the constant domains have been substituted for the membrane-proximal domains of MHC or MHC-like proteins (in this case, β2 microglobulin and the α3 domain of the CD1b protein).

The "correct" combination of VH and VL means a pair of VH and VL obtained from a single antibody. The "incorrect" combination of VH and VL means VH and VL obtained from distinct antibodies.

The "correct" pair of constant domains means either a pair of CH1-CK or a pair of interacting domains from MHC-like proteins (in this case, β2 microglobulin and the α3 domain of the CD1b protein). The "incorrect" pair of constant domains means the CH1-CD1b or β2 microglobulin-CK pairs.

Fig. 4 shows the general scheme of the experiment. Variable domain sequences of antibody Prolgolimab were used as variable domains VH1 and VL1, the corresponding variable domain sequences of antibody Ocrelizumab (Genentech) were used as VH2 and VL2 variable domains.

Table 2 shows protein productivity data. The results show that candidates having the CH1 constant domain in the heavy chain thereof and the membrane-proximal domain of the CD1b protein in the light chain thereof demonstrated low productivity (antibodies 01-001, 01-003, 01-008, 01-010). This is consistent with the known fact that the correct folding and export from the endoplasmic reticulum (ER) requires the interaction between the CH1 domain and the CL domain (Feige MJ, Groscurth S, Marcinowski M, Shimizu Y, Kessler H, Hendershot LM, Buchner J. An unfolded CH1 domain controls the assembly and secretion of IgG antibodies. Mol Cell. 2009 Jun 12;34(5):569-79. doi: 10.1016/j.molcel.2009.04.028. PMID: 19524537; PMCID: PMC2908990). Antibodies 01-002 and 01-009 did not pass such quality control in the ER and demonstrated productivity above 200 mg/l; however, despite the high productivity levels, the mobility thereof during PAGE did not correspond to that of a full-length molecule (Fig. 5A, lanes 2, 5).

**Table 2. Productivity of model monospecific chimeric antibodies. VH is the heavy chain variable domain of the antibody, VL is the light chain variable domain of the antibody, CH1 is the first heavy chain constant domain of the antibody, CK is the light chain constant domain of the antibody, b2M is β2 microglobulin, CD1b is the α3 domain of the CD1b protein.**

| Antibody | Experimental group | Heavy chain (VH_CH1) | Light chain (VL_CK) | Productivity on day 9, mg/l |
|---|---|---|---|---|
| 01-001 | 1 | Prolgolimab_CH1 | Prolgolimab_CD1b | 13.6 |
| 01-002 | 1 | Prolgolimab_b2M | Prolgolimab_CK | 230.83 |
| 01-003 | 1 | Ocrelizumab_CH1 | Ocrelizumab_CD1b | 12.97 |
| 01-004 | 2 | Prolgolimab_CH1 | Ocrelizumab_CK | 221.7 |
| 01-005 | 2 | Prolgolimab_b2M | Ocrelizumab_CD1b | 214.8 |
| 01-006 | 2 | Ocrelizumab_CH1 | Prolgolimab_CK | 328.87 |
| 01-007 | 2 | Ocrelizumab_b2M | Prolgolimab_CD1b | 201.7 |
| 01-008 | 3 | Prolgolimab_CH1 | Ocrelizumab_CD1b | 7.31 |
| 01-009 | 3 | Prolgolimab_b2M | Ocrelizumab_CK | 200.47 |
| 01-010 | 3 | Ocrelizumab_CH1 | Prolgolimab_CD1b | 33.53 |
| 01-011 | Control | Prolgolimab_b2M | Prolgolimab_CD1b | 293.63 |
| 01-012 | Control | Prolgolimab_CH1 | Prolgolimab_CK | 284.03 |

Fig. 5 shows the results of SDS gel electrophoresis under non-reducing conditions. According to the results, the electrophoretic mobility of antibodies from group 1 (Fig. 5A lanes 1, 2, Fig. 5B lane 1) and group 3 (Fig. 5A lanes 4.5, Fig. 5B lane 1) was higher than that of a full-length molecule (Fig. 5B lane 2), indicating that the molecules failed to assemble. Despite the VH and VL sequences were obtained from distinct antibodies, the electrophoretic mobility of antibodies from group 2 (Fig. 5B lanes 2, 3, 4, 5 (the same as Fig. 5A lane 3)) corresponds to the mobility of a full-length molecule (Fig. 5B lane 2). As expected, antibodies from the control group assembled (Fig. 5B lane 2, Fig. 5A lane 6 (the same as Fig. 5B lane 6)

The resulting data indicate that the dimerization unit based on the membrane-proximal domains of MHC or MHC-like proteins prevents the formation of incorrect pairs between the heavy and light chains when the chains include constant domains of distinct nature.

### Example 6. Generation of bivalent bispecific chimeric antibodies comprising MHC-like dimerization unit.

To verify the universality of the subject approach as a platform solution for assembling bispecific molecules using any pair of antigen-binding fragments (hereinafter light and heavy chain variable fragments), we chose three random pairs of light and heavy chain variable fragments from known antibodies (see Table 3) and used them to generate 6 bispecific chimeric antibodies. Table 3 shows the results of productivity for the produced proteins. Fig. 6 shows the results of SDS gel electrophoresis of the generated samples under non-reducing and reducing conditions. For all six samples there is present a major band at about 150 kDa, corresponding to a full-length molecule. Table 3 also shows the results of the sample purity according to SE HPLC.

**Table 3. Productivity and purity of bispecific chimeric antibodies produced using dimerization units based on domains from the membrane-proximal domains of MHC-like proteins.**

| Sample | First pair of light and heavy chain variable fragments within_Fab | Second pair of light and heavy chain variable fragments and MHC-like dimerization unit | Productivity, day 9, mg/l | Purity by SE HPLC | | |
|---|---|---|---|---|---|---|
| | | | | Aggregates, % | Monomer, % | Fragments, % |
| 02-004 | Prolgolimab | Ocrelizumab | 188.6 | 14.22 | 72.94 | 12.84 |
| 02-005 | Ocrelizumab | Prolgolimab | 224.3 | 7.1 | 90.4 | 2.6 |
| 02-006 | Prolgolimab | Anti-CSF1R | 83.2 | 14.7 | 78.2 | 7.1 |
| 02-007 | Anti-CSF1R | Prolgolimab | 241.9 | 7.0 | 81.7 | 11.4 |
| 02-008 | Ocrelizumab | Anti-CSF1R | 141.0 | 9.0 | 87.1 | 3.9 |
| 02-009 | Anti-CSF1R | Ocrelizumab | 205.2 | 5.37 | 85.74 | 8.89 |

### Example 7 Determination of affinity of full-length bispecific chimeric antibodies on Forte Bio Octert RED 384

To verify that the generated bispecific chimeric antibodies have not lost the antigen-binding ability thereof, we conducted an affinity analysis on Forte Bio Octert RED 384. For antibodies 02-004 and 02-005, we measured affinity to the extracellular domain of the human PD-1 protein (hPD1ex-H6F) and to the biotinylated peptide [NH2]CEPANPSEKNSPSTQYCYSIQS[CH2CH2]biotin comprising in the amino acid sequence a fragment of the human CD20 sequence (hereinafter referred to as CD20 peptide); for antibodies 02-006 and 02-007, we measured affinity to the extracellular domain of the human PD-1 protein (hPD1ex-H6F) and to the extracellular domain of the human CSF1R protein (hCSF1R_His); for antibodies 02-008 and 02-009, we measured affinity to the extracellular domain of the human CSF1R protein (hCSF1R His) and to CD20 peptide.

The sequence of aa 20-512 of the human CSF1R protein with C-terminal His and FLAG tags, molecular weight 57.4 kDa, was used as the hCSF1R antigen. The sequence of aa 21-170 of the human PD-1 protein with C-terminal His and FLAG tags, molecular weight 20.6 kDa, was used as the hPD-1ex-H6F antigen (see Table 6).

Genetic sequences encoding antigens were synthesized *de novo,* cloned into an expression vector, generated in CHO cells and purified using affinity chromatography, as described in general examples.

The experiments were carried out using a kinetic buffer solution (hereinafter referred to as 1xKB) having the following formulation: 4.3 mM Na₂HPO₄; 136.9 mM NaCl, 1.5 mM KH₂PO₄; 2.7 mM KCl; the volume fraction of the added Tween 20 was 0.1%; the mass fraction of the added BSA (bovine serum albumin) was 0.1%; pH 7.4. Before the measurement, the ProA sensors were regenerated with a solution of 50 mM glycine and hydrochloric acid, pH 1.8 (5 seconds in the regenerating solution, 5 seconds in 1xKB, three repetitions).

In the case of the hPD1ex-H6F and hCSF1R His antigens, the Protein A (ProA) biosensors (ForteBio) were immersed in a solution with antibodies at a concentration of 10 µg/ml for 60 seconds to immobilize same. The baseline was recorded in 1xKB for 60 seconds. The sensors loaded with the antibody were then immersed into wells containing a solution of the target antigen (analyte) in a kinetic buffer for 90 seconds. Measurements were performed for solutions with hPD1ex-H6F analyte at concentrations of 2.50 µg/ml (121.4 nM), 1.25 µg/ml (60.7 nM), 0.625 µg/ml (30.35 nM). Measurements were performed for solutions with hCSF1R_His analyte at concentrations of 2.50 µg/ml (43.6 nM), 1.25 µg/ml (21.8 nM), 0.625 µg/ml (10.9 nM). Then we detected complex dissociation in 1xKB for 210 s for hPD1ex-H6F, 300 s for hCSF1R His.

The reference sensors went through all the steps as the sensors used to record analyte sensograms did, with the exception of the association step - at the association step, the sensors were immersed in 1xKB solution without analyte (the reference sensor signals were measured in parallel with the recording of the main sensograms). The reference signal was subtracted from the signal received on sensors interacting with the analyte during the processing of sensograms.

To check the nonspecific interaction between the analyte and the sensors, we used an antibody-free sensor (at the loading step, the sensor was immersed in 1xKB solution; all other steps are identical to those used for the sensor loaded with antibody).

In the case of the biotinylated CD20 peptide, the peptide at a concentration of 5 µg/ml was immobilized on the surface of SAX sensors (High Precision Streptavidin (SAX) biosensors, ForteBio) for 120 seconds. The baseline was recorded in a 1xKB solution for 120 seconds. The sensors containing the loaded peptide were immersed into wells containing the antibody solution (analyte) in 1xKB for 15 seconds. Measurements were carried out for solutions with analyte (antibodies in the case of CD20 peptide) at concentrations of 150 µg/ml, 75 µg/ml, 37.5 µg/ml. Complex dissociation was recorded in 1xKB for 120 seconds.

All measurements were carried out at 30 °C, orbital mixing speed was 1,000 revolutions per minute.

To obtain numerical values of kinetic constants (kₒₙ is the on/association rate constant, k_{dis} is the dissociation rate constant, KD is the equilibrium dissociation constant or affinity constant), the resulting sensograms were processed according to the 1:1 interaction model using Global Fit (selection of one set of kon, kdis, KD constants to analyze several sensograms of different concentrations) of the ForteBio Octet Data Analysis 9.0 software. The results are shown in Table 4.

**Table 4. Kinetic constants kₒₙ, k_{dis}, KD for interactions between antibodies 02-004 - 02-009 and the target antigens.**

| Antibody | Antigen | KD (M) | kₒₙ (M-1c-1) | k_{dis} (c-1) | R^2 |
|---|---|---|---|---|---|
| 02-004 | hPD1ex-H6F | 3,49E-09 | 2.16E+05 | 7.53E-04 | 0.9916 |
| | CD20 peptide | 1.27E-06 | 1.88E+05 | 2.38E-01 | 0.9781 |
| 02-005 | hPD1ex-H6F | 5.69E-09 | 1.78E+05 | 1.01E-03 | 0.9894 |
| | CD20 peptide | 1.12E-06 | 3.05E+05 | 3,42E-01 | 0.9789 |
| 02-006 | hPD1ex-H6F | 2.51E-09 | 2.75E+05 | 6.91E-04 | 0.9867 |
| | hCSF1R_His | 7.97E-10 | 3.59E+05 | 2.86E-04 | 0.9985 |
| 02-007 | hPD1ex-H6F | 4,49E-09 | 2,42E+05 | 1.09E-03 | 0.9873 |
| | hCSF1R_His | 8.31E-10 | 3.78E+05 | 3.14E-04 | 0.9986 |
| 02-008 | CD20 peptide | 8.92E-07 | 3.69E+05 | 3.29E-01 | 0.9696 |
| | hCSF1R_His | 5,49E-10 | 4.29E+05 | 2.36E-04 | 0.9982 |
| 02-009 | hCSF1R_His | 4.20E-10 | 5.00E+05 | 2.10E-04 | 0.9984 |
| | CD20 peptide | 8.20E-07 | 2.16E+05 | 1.77E-01 | 0.9780 |

Table 5 shows verification results for nonspecific interaction between the analyte and non-loaded sensors. There was no nonspecific interaction between the analyte and non-loaded sensors.

**Table 5. Results of verification of nonspecific interaction between the analyte and non-loaded sensors**

| Sensor type | Analyte | Concentration, µg/ml | Signal, nm |
|---|---|---|---|
| ProA | hPD1ex-H6F | 2.5 | -0.023 |
| ProA | hCSF1R_His | 2.5 | -0.0038 |
| SAX | 02-004 | 150 | 0.017 |
| SAX | 02-005 | 150 | 0.0048 |
| SAX | 02-008 | 150 | 0.0173 |
| SAX | 02-009 | 150 | 0.0112 |

Following the results, all bispecific chimeric antibodies 02-004 - 02-009 demonstrate specific binding to target antigens, and the numerical KD values have the same order of magnitude regardless of the position of the corresponding antigen-binding fragments (either within the Fab fragment or within the Fab-like fragment comprising β2 microglobulin and the α3 domain of CD1b protein, substituting the CK and CH1 domains).

### Example 8. Analysis of nonspecific binding of bispecific chimeric antibodies to non-target antigens

Experimental study of nonspecific binding of bispecific chimeric antibodies to non-target antigens was performed on Forte Bio Octert RED 384.

Protein A (ProA) biosensors (ForteBio) were immersed ino a solution containing antibodies at a concentration of 10 µg/ml for 150 seconds to immobilize same. The baseline was recorded in a 1xKB solution for 30 seconds. The antibody-loaded sensors were then immersed into wells containing non-target antigens at a concentration of 30 µg/ml for 150 seconds. Complex dissociation was then recorded for 30 seconds.

The reference sensors went through all the steps as the sensors used to record analyte sensograms did, with the exception of the association step - at the association step, the sensors were immersed in a kinetic buffer solution without analyte (the reference sensor signals were measured in parallel with the recording of the main sensograms). The reference signal was subtracted from the signal received on sensors interacting with the analyte during the processing of sensograms.

The antigen panel included: hAng2_H6F, hPD1ex-H6F, hCSF1R His, IL5R-His, hIL4R-His, hCD38-Avi, C-Avi-His-TEVs-HSA_hBCMA, Her3-H6F, hisOX40, Macaca CSF1R_C-His (see Table 6). Each studied antibody in the antigen panel has a specific (target) antigen, the results of interaction with the target antigen are given as a positive control.

Sensograms were processed using ForteBio Octet Data Analysisn 9.0 software. To report no non-specific interaction, the recorded signal level was checked at the end of the association step (the response parameter). Table 7 shows the results of verification of the binding of the bispecific chimeric antibodies. The experiment has revealed no interaction between antibodies 02-004 - 02-009 and non-target antigens.

**Table 6. Composition of antigens**

| Antigen | Protein name | UNIPROT ID | Amino acids |
|---|---|---|---|
| PD1ex-H6F | Programmed cell death protein 1 (Homo sapiens) | Q15116 | 21-170 |
| hCD38-Avi | ADP-ribosyl cyclase/cyclic ADP-ribose hydrolase 1 (Homo sapiens) | P28907 | 43-300 |
| Her3-H6F | Receptor tyrosine-protein kinase erbB-3 (Homo sapiens) | P21860 | 21-643 |
| IL4R-His | Interleukin-4 receptor subunit alpha (Homo sapiens) | P24394 | 27-227 |
| hAng-H6F | Angiopoietin-2 (Homo sapiens) | 015123 | 19-496 |
| His-OX40 | Tumor necrosis factor receptor superfamily member 4 (Homo sapiens) | P43489 | 26-210 |
| IL5R-His | Interleukin-5 receptor subunit alpha (Homo sapiens) | Q01344 | 22-342 |
| hCSF1R_His | Macrophage colony-stimulating factor 1 receptor (Homo sapiens) | P07333 | 20-512 |
| Macaca CSF1R_C-His | Macrophage colony-stimulating factor 1 receptor (Macaca mulatta) | F7BTH9 | 20-513 |
| C-Avi-His-TEVs-HSA_hBCMA | Tumor necrosis factor receptor superfamily member 17 (Homo sapiens) | Q02223 | 1-54 |

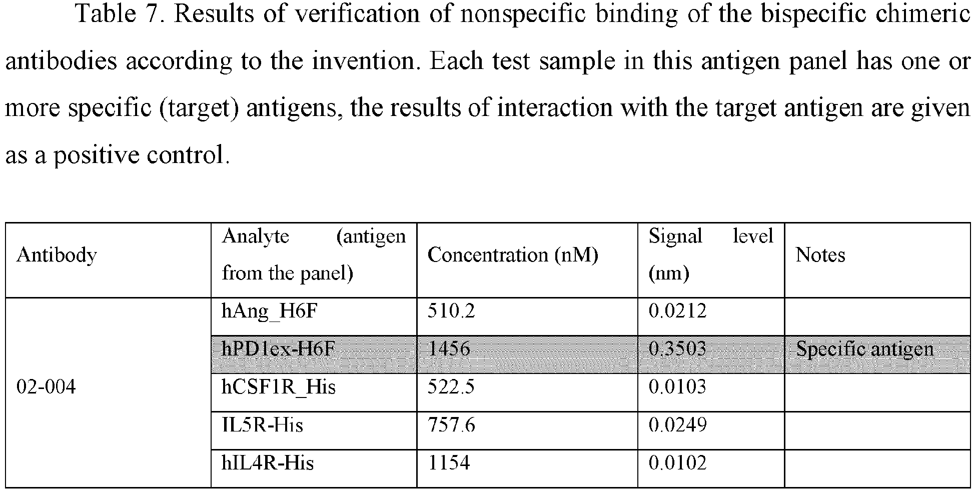

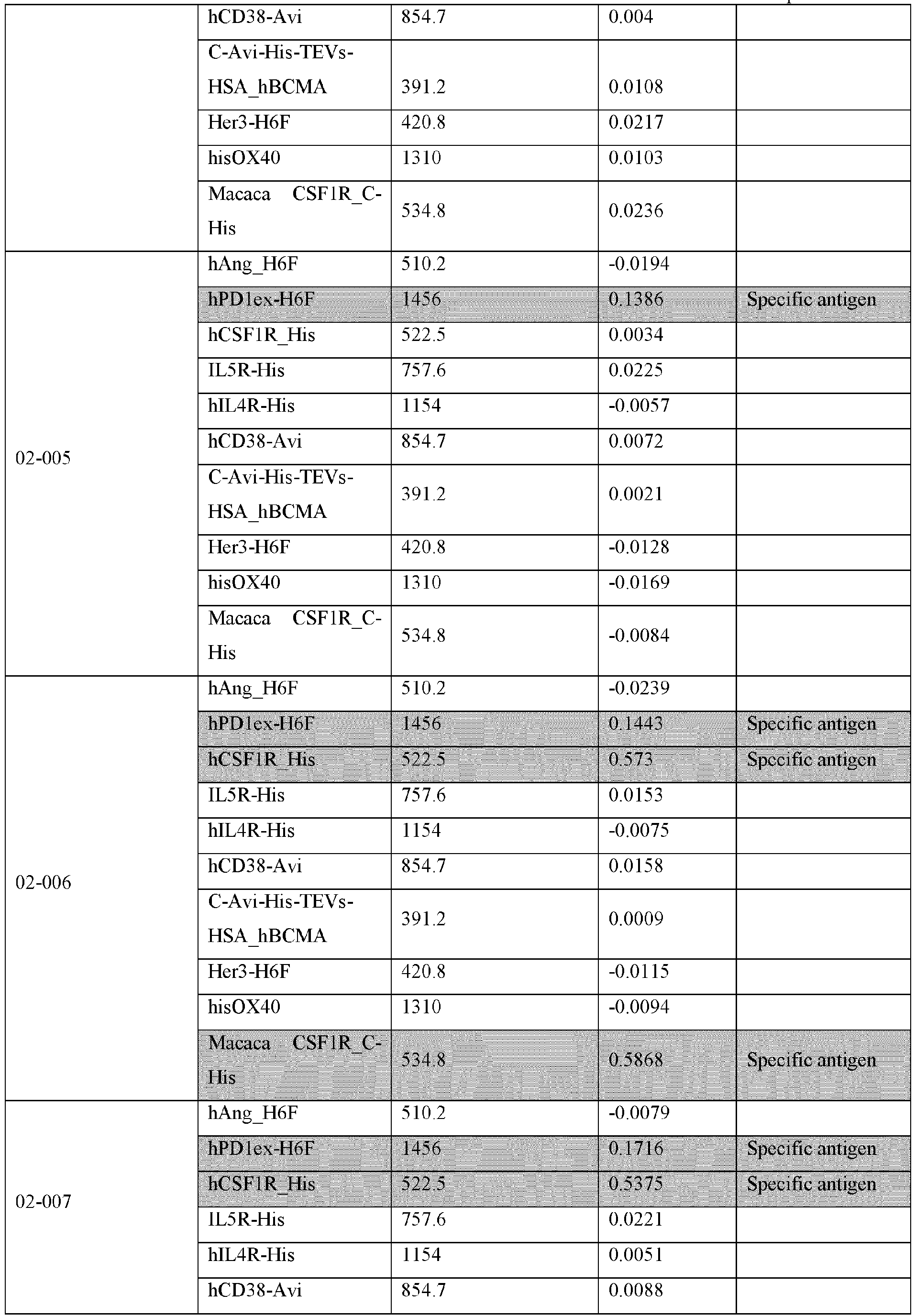

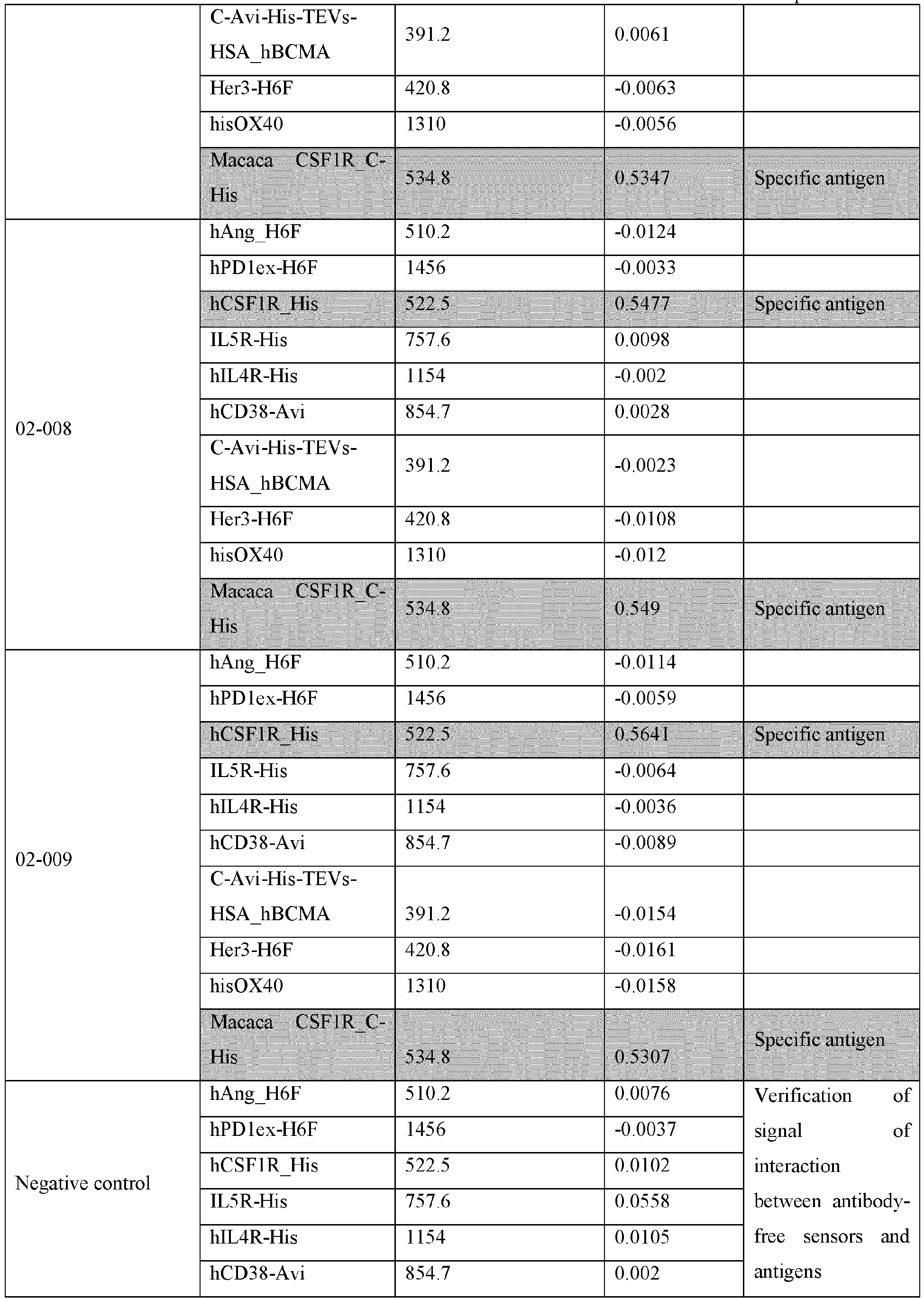

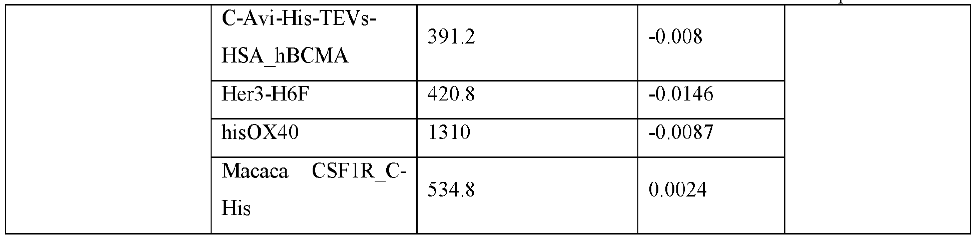

### Example 9 Analysis of simultaneous binding of bispecific chimeric antibodies according to the invention to two antigens.

Antibodies 02-006 and 02-007 were analysed for simultaneous binding to two distinct target antigens (hPD1ex-H6F and hCSF1R_His) on Forte Bio Octet RED 384. The experiment was carried out on AR2G sensors (Amine Reactive Second-Generation (AR2G) biosensors, ForteBio). The steps of the experiment are shown in Table 8.

**Table 8. Steps of experiment to verify simultaneous binding of bispecific chimeric antibodies according to the invention to two distinct antigens**

| No. | Experiment step | Step duration (s) | Notes |
|---|---|---|---|
| 1 | Baseline | 60 | Auxiliary step, equilibration in water, verification of the signal recorded on sensors |
| 2 | Activation | 300 | Aqueous solution of activators 20 mM EDC (1-(3-Dimethylaminopropyl)-3-ethylcarbodiimide hydrochloride) and 10 mM sNHS (N-hydroxysulfosuccinimide) |
| 3 | Loading | 900 | Loading of hPD1ex-H6F protein (15 µg/ml) in 10 mM sodium acetate buffer solution pH 5.0 |
| 4 | Quenching | 300 | 1 M ethanolamine pH 8.5. Quenching of unreacted active groups on sensor surface |
| 5 | Baseline 2 | 300 | Equilibration of sensors in kinetic buffer solution |
| 6 | Loading 2 | 300 | Loading of antibody onto sensors is due to interaction with sensor-immobilized hPD1ex-H6F |
| 7 | Baseline 3 | 15 | Equilibration of sensors in kinetic buffer solution, verification of dissociation rate of antibody from sensors |
| 8 | Association | 60 | Interaction with hCSF1R_His |
| 9 | Dissociation | 30 | Auxiliary step, confirms the absence of fast dissociation following binding to hCSF1R_His |

The sensors were activated in an aqueous solution containing 20 mM EDC (1-(3-dimethylaminopropyl)-3-ethylcarbodiimide hydrochloride) and 10 mM sNHS (N-*hydroxysulfosuccinimide*) for 300 s. The antigen (hPD1ex-H6F) was loaded onto the surface of biosensors in a 10 mM sodium-acetate buffer with pH 5.0 for 300 s. The concentration of protein to be loaded was 15 µg/ml. Unreacted active centers on the sensor surfaces were quenched in 1M aqueous solution of ethanolamine with pH 8.5 for 300 s. The baseline at the fifth step of the experiment and all subsequent steps were carried out in a kinetic buffer solution (1xKB) having the following formulation: 4.3 mM Na₂HPO₄; 136.9 mM NaCl, 1.5 mM KH₂PO₄; 2.7 mM KCl; the volume fraction of added Tween 20 was 0.1%; the mass fraction of added B SA was 0.1%; pH 7.4. After recording the baseline (step 5), antibodies were loaded onto sensors; the concentration of the antibody to be loaded was 30 µg/ml (step 6). The third baseline was then recorded (step 7). This step demonstrates the absence of fast signal decay, indicating a specific interaction between the loaded antibody and hPD1ex-H6F immobilized previously onto the sensors. At the next step of association (step 8), the sensors containing immobilized hPD1ex-H6F and bound antibodies were immersed into an hCSF 1R_His antigen solution at a concentration of 100 µg/ml. The signal amplification at this step is due to the presence of antibodies loaded onto he sensors, a FAB fragment to the hCSF1R His antigen.

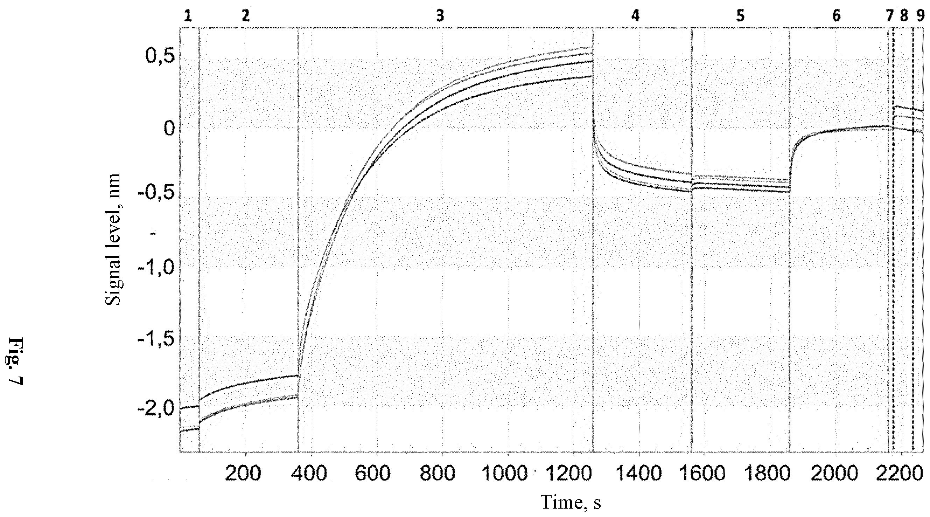

Sensograms were analysed using ForteBio Octet Data Analysisn 9.0 software. The main conclusions are shown in Table 9. Sensograms showing the steps (stages) of the experiment are shown in Fig. 7. Following the results, samples 02-006 and 02-007 demonstrate simultaneous binding to the hPD1ex-H6F and hCSF1R His antigens.

**Table 9. Results of experiment involving simultaneous interaction of antibodies 02-006 and 02-007 with two distinct antigens (hPD1ex-H6F and hCSF1R His).**

| Test antibody | Sensor-loaded proteins | Association | Signal level (nm) | Notes |
|---|---|---|---|---|
| 02-006 | Immobilized hPD1ex-H6F loaded with antibody 02-006 | hCSF1R_His (100 µg/ml) | 0.1375 | Demonstrates simultaneous interaction with two distinct antigens (hPD1ex-H6F and hCSF1R_His) |
| | | 1xKB | -0.0194 | |
| 02-007 | Immobilized hPD1ex-H6F loaded with antibody 02-007 | hCSF1R_His (100 µg/ml) | 0.0757 | Demonstrates simultaneous interaction with two distinct antigens (hPD1ex-H6F and hCSF1R_His) |
| | | 1xKB | -0.0125 | |
| Verification of nonspecific interaction between analyte (hCSF1R_His) and sensors loaded with hPD1ex-H6F | Immobilized hPD1ex-H6F not loaded with antibody | hCSF1R_His (100 µg/ml) | -0.0023 | This control demonstrates that sensors containing immobilized hPD1ex-H6F do not interact with hCSF1R_His when not loaded with antibodies |

Following the results, antibodies 02-006 and 02-007 demonstrate simultaneous binding to the hPD1ex-H6F and hCSF1R His antigens.

### Example 10. Analysis of molecular weights of bispecific chimeric antibodies according to the invention using reverse-phase ultra-high-performance liquid chromatography (RP UHPLC) with mass spectrometric detection

To confirm the correct assembly of molecules, we carried out an analysis of molecular weights of the full-length bispecific chimeric antibodies according to the invention. This method makes it possible to identify full-length bispecific chimeric antibodies according to the invention consisting of 4 distinct chains and to distinguish same from by-products formed from another set of chains. These data, combined with the data obtained in Example 5, suggest that the technological solution involving substituting a pair of constant CH1-CK domains for a pair of membrane-proximal domains from MHC or MHC-like proteins provides for the correct assembly of the bispecific chimeric antibodies according to the invention.

The analysis was carried out using the Agilent 1290 Infinity II UPLC-Agilent 6530 Q-Tof chromatography-mass spectrometric complex on the BioResolve Polyphenyl RP column (2.1 x 50 mm, particle diameter 2.7 µm; the BioResolve Polyphenyl RP precolumn, 2.1 x 5 mm, particle diameter 2.7 µm, was also used). Prior to the analysis, all molecules were treated with the PNGase F (Promega) enzyme to remove N-glycans. To this end, the enzyme was diluted with water to a concentration of 1 unit of activity in µl. A sample with the protein content of 120 µg was mixed with a buffer solution (100 mM ammonium bicarbonate pH 7.2), a solution of PNGase F was added at the enzyme:protein ratio of 1 unit : 50 µg, the mixture was incubated in a thermostat for 18 hours at (37.0 ± 0.1) °C.

For analysis, 10 µg of the test solution were selected in accordance with the measured protein concentrations and the concentration was adjusted with mobile phase A to 0.2 mg/ml. The sample input volume was 7 µl.

Prior to the analysis, the chromatographic system was equilibrated with a mobile phase at the initial ratio of mobile phases A: 95%, B: 5% (phase A contains 0.1% formic acid solution, 0.02% trifluoroacetic acid solution in water, phase B contains 0.1% formic acid solution, 30% acetonitrile in isopropyl alcohol) for at least 30 min until stable pressure was achieved. The mass spectrometer was calibrated using a calibration standard in accordance with the manufacturer's guidelines.

The sample was separated at a flow rate of 0.5 ml/min in mobile phase B concentration gradient (from 5% to 27% from 5 to 6 minutes following sample introduction, thereafter from 27% to 37% from 6 to 30 minutes) at a column temperature of (60 ± 1) °C, a chromatogram was obtained at a wavelength of 280 nm.

Data was processed in the PMi Intact software.

The names of the chains included into the bispecific chimeric antibodies are shown in Table 10.

**Table 10. Names of chains included into bispecific chimeric antibodies 02-004 - 02-009**

| Antibody | Name of chains included into antibody-like protein |
|---|---|
| 02-004 | Prolgolimab_VH_HC_hole |
| | Prolgolimab_VL_CK |
| | Ocrelizumab_VH_b2m_Fc_knob |
| | Ocrelizumab_VL_CD1b |
| 02-005 | Ocrelizumab_VH_HC_hole |
| | Ocrelizumab_VL_CK |
| | Prolgolimab_VH_b2m_Fc_knob |
| | Prolgolimab_VL_CD1b |
| 02-006 | Prolgolimab_VH_HC_hole |
| | Prolgolimab_VL_CK |
| | Anti-CSF1R_VH_b2m_Fc_knob |
| | Anti-CSF1R_VL_CD1b |
| 02-007 | Anti-CSF1R_VH_HC_hole |
| | Anti-CSF1R_VL_CK |
| | Prolgolimab_VH_b2m_Fc_knob |
| | Prolgolimab_VL_CD1b |
| 02-008 | Ocrelizumab_VH_HC_hole |
| | Ocrelizumab_VL_CK |
| | Anti-CSF1R_VH_b2m_Fc_knob |
| | Anti-CSF1R_VL_CD1b |
| 02-009 | Anti-CSF1R_VH_HC_hole |
| | Anti-CSF1R_VL_CK |
| | Ocrelizumab_VH_b2m_Fc_knob |
| | Ocrelizumab_VL_CD1b |

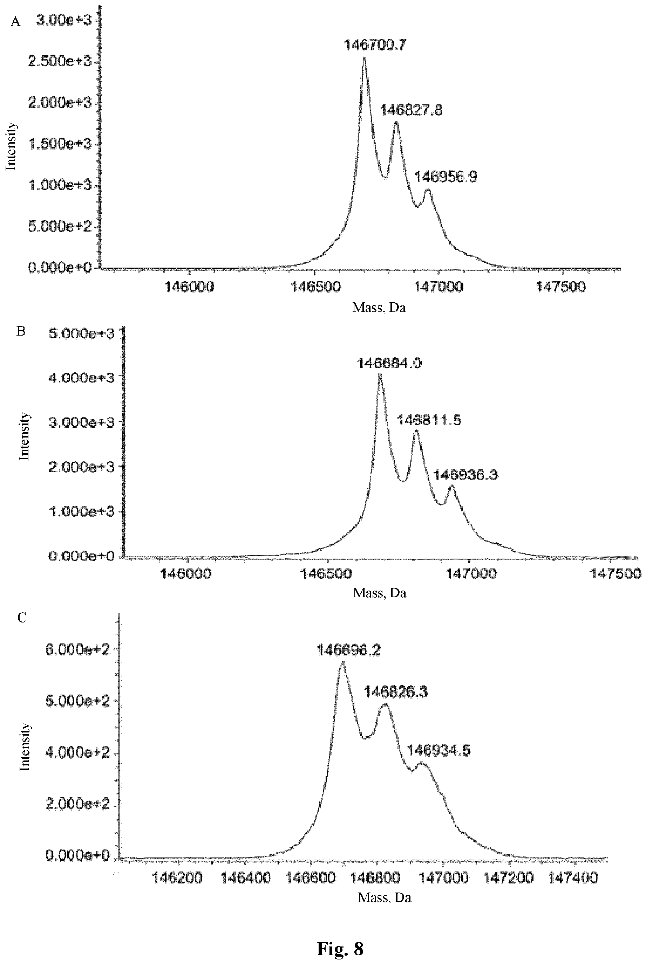

The results of the mass analysis for antibodies 02-004, 02-006, 02-009 (as the most representative ones) are shown in Table 11. Also, Fig. 8 shows images of the deconvoluted mass spectrum of peaks 4 to 6 of the total ion current chromatogram for antibody 02-004, the peaks for the rest of the antibodies looked similar.

For each antibody, the table shows the mass of the substance at the corresponding chromatogram peaks; the peaks were further annotated, resulting in identification of chains included into the fragments of a certain mass. The mass distribution was similar in all the samples: the major peak was at 146 kDa, and minor mass peaks of about 23 kDa (corresponding to the mass of a single light chain) and of about 74 kDa (corresponding to the heavy and light chain heterodimer). The major peaks were annotated based on the theoretical values of the average masses.

Peak 4 of antibody 02-004 was not annotated by software because the settings included the assumption that all N-terminal glutamines are in the pyro form (accordinly, the mass of pyroglutamine was 18 Da less than that of glutamine). Thus, the masses obtained in peak 4 correspond to the masses of molecules annotated in peak 5, where one of the N-terminal glutamines is in the non-pyro form.

Peaks 5 and 6 of the 02-004 antibody have 3 masses corresponding to a full-length molecule and two lysine-free forms of a full-length molecule (missing one C-terminal cysteine and two C-terminal lysines). In antibodies 02-006 and 02-009, the mass distribution was in a similar fashion.

**Table 11. Annotation of total ion current chromatogram peaks. The correspondence between antibodies and chain names is shown in Table 10. "-Lys(1)" indicates missing one of C-terminal lysines of heavy chains, "-Lys(2)" indicates missing both of C-terminal lysines of heavy chains.**

| Antibody | Peak, no. | Yield time, min | Relative intensity of peak mass, % | Peak annotation (chains that form a molecule) | Mass, Da | Relative peak area, % |
|---|---|---|---|---|---|---|
| 02-004 | 1 | 8.12793 | 100 | 23263 | 23262.5 | 0.452 |
| | 2 | 9.00068 | 34.9407 | 23432 | 23431.6 | 1.061 |
| | | | 100 | 23245 | 23245.1 | |
| | 3 | 14.1315 | 89.472 | 74260 | 74259.7 | 12.933 |
| | | | 91.3607 | Ocrelizumab_VH_b2m_Fc_knob, Ocrelizumab_VL_CD1b | 73961.5 | |
| | | | 95.6128 | 74138 | 74138.2 | |
| | | | 100 | 74088 | 74088.5 | |
| | 4 | 17.711 | 37.5684 | 146957 | 146957 | 26.771 |
| | | | 69.086 | 146828 | 146828 | |
| | | | 100 | 146701 | 146701 | |
| | 5 | 18.0457 | 39.3818 | Ocrelizumab_VL_CD1b, Ocrelizumab_VH_b2m_Fc_knob, Prolgolimab_VH_HC_hole, Prolgolimab_VL_CK | 146936 | 55.956 |
| | | | 69.116 | Ocrelizumab_VL_CD1b, Ocrelizumab_VH_b2m_Fc_knob, Prolgolimab_VH_HC_hole-Lys(1), Prolgolimab_VL_CK | 146812 | |
| | | | 100 | Ocrelizumab_VL_CD1b, Ocrelizumab_VH_b2m_Fc_knob, Prolgolimab_VH_HC_hole-Lys(2), Prolgolimab_VL_CK | 146684 | |
| | 6 | 19.5708 | 48.9742 | Ocrelizumab_VL_CD1b, Ocrelizumab_VH_b2m_Fc_knob, Prolgolimab_VH_HC_hole, Prolgolimab_VL_CK | 146935 | 2.828 |
| | | | 78.6579 | Ocrelizumab_VL_CD1b, Ocrelizumab_VH_b2m_Fc_knob, Prolgolimab_VH_HC_hole-Lys(1), Prolgolimab_VL_CK | 146826 | |
| | | | 100 | Ocrelizumab_VL_CD1b, Ocrelizumab VH b2m Fc knob. Prolgolimab_VH_HC_hole-Lys(2), Prolgolimab_VL_CK | 146696 | |
| 02-006 | 1 | 8.9566 | 42.2628 | 23432 | 23431.9 | 0.757 |
| | | | 100 | 23245 | 23245.5 | |
| | 2 | 15.5067 | 76.2204 | Anti-CSF1R_VL_CD1b, Anti-CSF1R_VH_b2m_Fc_knob | 73805.3 | 9.69 |
| | | | 96.3739 | 74117 | 74117.2 | |
| | | | 98.217 | 73931 | 73931.2 | |
| | | | 100 | 73986 | 73985.7 | |
| | 3 | 18.7862 | 47.5686 | Anti-CSF1R_VH_b2m_Fc_knob, Anti-CSF1R_VL_CD1b, Prolgolimab_VH_HC_hole, Prolgolimab_VL_CK, | 146786 | 89.553 |
| | | | 76.1565 | Anti-CSF1R_VH_b2m_Fc_knob, Anti-CSF1R_VL_CD1b, Prolgolimab_VH_HC_hole-Lys(1), Prolgolimab_VL_CK | 146661 | |
| | | | 100 | Anti-CSF1R_VH_b2m_Fc_knob, Anti-CSF1R_VL_CD1b, Prolgolimab_VH_HC_hole-Lys(2), Prolgolimab_VL_CK | 146534 | |
| 02-009 | 1 | 8.08387 | 45.7919 | Anti-CSF1R_VL_CK, Reference | 23035.6 | 2.969 |
| | | | 78.2384 | 23341 | 23341 | |
| | | | 100 | 23155 | 23154.7 | |
| | 2 | 14.1756 | 60.3569 | Ocrelizumab_VH_b2m_Fc_knob, Ocrelizumab_VL_CD1b(1) | 73962.3 | 14.71 |
| | | | 69.7348 | 74091 | 74091 | |
| | | | 97.9381 | 74134 | 74134.2 | |
| | | | 100 | 74259 | 74259.5 | |
| | 3 | 17.2699 | 32.2696 | Anti-CSF1R_VH_HC_hole, Anti-CSF1R_VL_CK, Ocrelizumab_VH_b2m_Fc_knob, Ocrelizumab_VL_CD1b | 146524 | 74.95 |
| | | | 63.7103 | Anti-CSF1R_VH_HC_hole, Anti-CSF1R_VL_CK, Ocrelizumab_VH_b2m_Fc_knob-Lys(1), Ocrelizumab_VL_CD1b | 146399 | |
| | | | 100 | Anti-CSF1R_VH_HC_hole, Anti-CSF1R_VL_CK, Ocrelizumab_VH_b2m_Fc_knob-Lys(2), Ocrelizumab_VL_CD1b | 146271 | |
| | 4 | 18.892 | 39.1159 | Anti-CSF1R_VH_HC_hole, Anti-CSF1R_VL_CK, Ocrelizumab_VH_b2m_Fc_knob, Ocrelizumab_VL_CD1b | 146526 | 7.371 |
| | | | 69.8928 | Anti-CSF1R_VH_HC_hole, Anti-CSF1R_VL_CK, Ocrelizumab_VH_b2m_Fc_knob-Lys(1), Ocrelizumab_VL_CD1b | 146404 | |
| | | | 100 | Anti-CSF1R_VH_HC_hole, Anti-CSF1R_VL_CK, Ocrelizumab_VH_b2m_Fc_knob-Lys(2), Ocrelizumab_VL_CD1b | 146275 | |

To sum up so far, the results demonstrate that the mass of full-length bispecific chimeric antibodies corresponds to that of a molecule consisting of four distinct chains. Also, the preparations include low molecular weight fragments but they are minor.

### Example 11. Confirmation of correct assembly of bispecific chimeric antibodies according to the invention using proteolysis and mass analysis of the resulting fragments

To directly verify the correct pairing between light and heavy chains, the bispecific chimeric antibodies according to the invention were cleaved by the GingisKHAN protease (Genovis) whose the recognition site is located in the antibody hinge region (...KSCDK/THTCPPCP...). Such proteolysis results in the breaking down of the full-length antibody into an Fc fragment, a Fab fragment, and a Fab-like fragment comprising β2-microglobulin and the α3 domain of the CDlb protein substituting the CK and CH1 domains. Fragments of the bispecific molecule were analyzed following proteolysis using vertical electrophoresis under non-reducing conditions; also, the resulting fragments were subjected to mass spectrometry.

The proteolysis reaction mixture contained 40 µg of the bispecific antibody and 40 units of the GingisKHAN enzyme (at the ratio of 1 enzyme unit : 1 µg of protein) in a buffer composed of 100 mM Tris-HCl, pH 8.0, 1 mM cysteine in a volume of 60 µl. The reaction was carried out in a thermostat at (37.0 ± 0.1) °C for 1 hour and stopped by adding iodoacetamide to a concentration of 10 mM. For electrophoresis, a buffer containing SDS (up to a concentration of 1% SDS) was added to the resulting samples, and SDS gel electrophoresis was performed under non-reducing conditions.

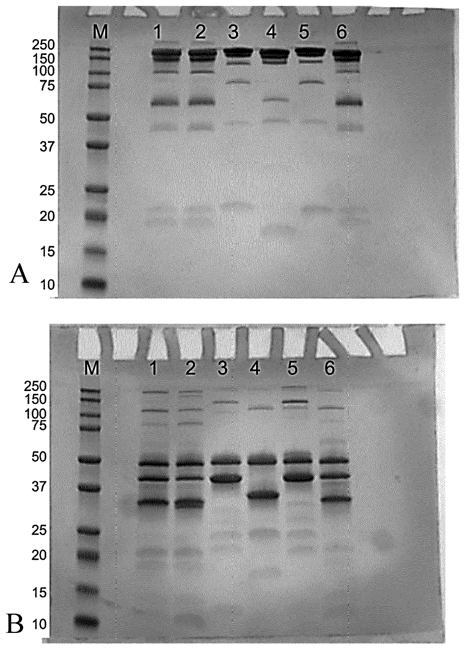

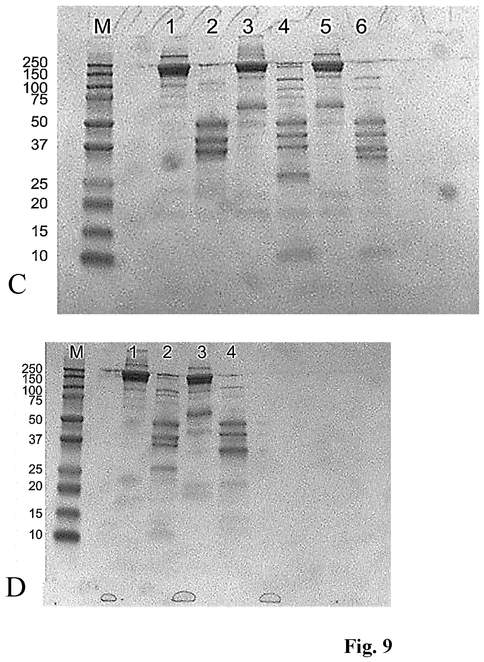

Fig. 9 shows the results of SDS gel electrophoresis. Following treatment of the bispecific chimeric antibodies (02-004 - 02-009) with the GingisKHAN protease, 3 major fragments were formed, which were observed in electrophoregram in 40-50 kDa region and showed distinct mobilities in polyacrylamide gel. Monospecific molecules containing (01-011) and free of a dimerization unit based on the membrane-proximal domains of the MHC-like protein CDlb (Prolgolimab, Ocrelizumab) were also treated with the GingisKHAN protease (Fig. 9B, lanes 3, 4, 5). As a result of comparing the electrophoretic mobilities formed as a result of proteolysis of fragments, it was concluded that the fragment showing the lowest mobility level corresponds to the Fc fragment, with medium mobility to a Fab fragment, and the fragment showing the highest mobility level to a Fab-like fragment comprising β2 microglobulin and the α3 domain of the CDlb protein substituting the CK and CH1 domains. In the case of antibodies 02-006 - 02-009, the electrophoregram showed a further fragment formed as a result of nonspecific cleavage of the variable domain of the anti-CSF1R fragment. In order to determine the exact masses of the fragments formed as a result of proteolysis, we conducted mass spectrometry analysis.

Prior to the mass spectrometry analysis, the samples, immediately following adding iodoacetamide, were transferred to 50 mM ammonium bicarbonate, pH (7.6 ± 0.2), on Zeba (MWCO 7 kDa) columns, according to the manufacturer's guidelines; thereafter, PNGase F at the ratio of 1 enzyme unit: 50 µg of protein was added to the samples and the samples were incubated in a thermostat for 18 hours at (37.0 ± 0.1) °C.

Mass analysis of the resulting fragments was carried out according to the methodology described in Example 10.

Results of RP UHPLC with mass spectrometric detection are shown in Table 12.

Antibodies 02-006 and 02-008 showed fragments of 11,424 Da and 37,035 Da, in total 48,459 Da corresponding to the mass of a Fab-like fragment comprising β2 microglobulin and the α3 domain of the CDlb protein substituting the CK and CH1 domains and variable fragments of light and heavy chains to CSF1R. Antibodies 02-007 and 02-009 showed fragments of 11,424 Da and 36,062 Da, in total 47,486 Da corresponding to the mass of a Fab fragment with variable fragments of light and heavy chains to CSF1R, which is consistent with the data obtained following SDS gel electrophoresis of these samples and confirms the presence of a putative further proteolysis site in the variable domain to CSF1R (Fig. 9).

**Table 12. Annotation of total ion current chromatogram peaks. Table 10 shows the correspondence between samples and chain names. Npart is the N-terminal section of the corresponding chain formed following cleavage of a full-length antibody by the GingisKHAN protease, Cpart is the C-terminal section of the corresponding chain formed following cleavage of a full-length bispecific chimeric antibody by the GingisKHAN protease.**

| Antibod y | Pea k, no. | Yiel d time, min | Peak mass fract ion | Peak annotation | Mass , Da | Relat ive peak area, UV 280n m % |
|---|---|---|---|---|---|---|
| 02-004 GingisK HAN | 1 | 7.72 388 | 1.00 | Prolgolimab_VH_HC_hole-Cpart, Ocrelizumab_VH_b2m_Fc_knob-Cpart (Fc fragment) | 5024 2.5 | 16.48 |
| | 2 | 13.1 124 | 0.04 | 37128 | 3712 8.3 | 2.47 |
| | | | 0.10 | 47583 | 4758 3.1 | |
| | | | 0.13 | 36023 | 3602 2.6 | |
| | | | 0.74 | Ocrelizumab_VL_CD1b, Ocrelizumab_VH_b2m_Fc_knob-Npart (Fab-like fragment comprising β2 microglobulin and the α3 domain of CD1b protein substituting CK and CH1 domains (hereinafter Fab-like fragment)) | 4861 6.5 | |
| | 3 | 13.3 272 | 1.00 | Ocrelizumab_VH_b2m_Fc_knob-Npart, Ocrelizumab_VL_CD1b(Fab-like fragment) | 4859 9.5 | 3.44 |
| | 4 | 13.5 261 | 1.00 | Ocrelizumab_VL_CD1b, Ocrelizumab_VH_b2m_Fc_knob-Npart(Fab-like fragment) | 4861 5 | 9.04 |
| | 5 | 13.8 08 | 1.00 | Ocrelizumab_VH_b2m_Fc_knob-Npart, Ocrelizumab_VL_CD1b(Fab-like fragment) | 4859 8.5 | 36,41 |
| | 6 | 14.2 39 | 1.00 | Ocrelizumab_VH_b2m_Fc_knob-Npart, Ocrelizumab_VL_CD1b(Fab-like fragment) | 4847 0,4 | 7.95 |
| | 7 | 16.2 781 | 0.22 | 47918.1 (non-pyro form of Fab fragment) | 4791 8.1 | 1.71 |
| | | | 0.78 | Ocrelizumab_VL_CD1b, Ocrelizumab_VH_b2m_Fc_knob-Npart(Fab-like fragment) | 4860 0.4 | |
| | 8 | 17.2 727 | 1.00 | Prolgolimab_VH_HC_hole-Npart, Prolgolimab_VL_CK(Fab fragment) | 4790 0.5 | 8.55 |
| | 9 | 17.5 877 | 1.00 | Prolgolimab_VH_HC_hole-Npart, Prolgolimab_VL_CK (Fab fragment) | 4788 3.1 | 12.6 |
| | 10 | 18,4 497 | 1.00 | 47865 | 4786 5.5 | 1.36 |
| 02-005 GingisK HAN | 1 | 7.72 805 | 1.00 | Ocrelizumab_VH_HC_hole_C-part, Prolgolimab_VH_b2m_Fc_knob_C-part (Fc fragment) | 5024 2.5 | 15.24 |
| | 2 | 13.0 666 | 1.00 | Ocrelizumab_VH_HC_hole_N-part, Ocrelizumab_VL_CK (Fab fragment) | 4762 5.9 | 4.52 |
| | 3 | 13.6 131 | 1.00 | Ocrelizumab_VH_HC_hole_N-part, Ocrelizumab_VL_CK (Fab fragment) | 4762 5 | 32.12 |
| | 4 | 14.6 078 | 0,46 | Ocrelizumab_VH_HC_hole_N-part, Ocrelizumab_VL_CK (Fab fragment) | 4762 5,4 | 5.98 |
| | | | 0.54 | 47607 | 4760 6.8 | |
| | 5 | 16.0 17 | 0.12 | Ocrelizumab_VH_HC_hole_N-part, Ocrelizumab_VL_CK (Fab fragment) | 4762 5.5 | 9.65 |
| | | | 0.88 | 48874 | 4887 4.2 | |
| | 6 | 16,4 977 | 0.10 | Ocrelizumab_VH_HC_hole_N-part, Ocrelizumab_VL_CK (Fab fragment) | 4874 6.5 | 27.74 |
| | | | 0.90 | Prolgolimab_VL_CD1b, Prolgolimab_VH_b2m_Fc_knob_N-part (Fab-like fragment) | 4885 6.7 | |
| | 7 | 16.9 122 | 0,46 | Prolgolimab_VL_CD1b, Prolgolimab_VH_b2m_Fc_knob_N-part (Fab-like fragment) | 4872 8.5 | 4.75 |
| | | | 0.54 | Ocrelizumab_VH_HC_hole_N-part, Ocrelizumab _VL_CK (Fab fragment) | 4762 6,4 | |
| 02-006 GingisK HAN | 1 | 7.67 755 | 1.00 | Prolgolimab_VH_HC_hole_C-part, Anti-CSF1R_VH_b2m_Fc_knob_C-part(Fc fragment) | 5024 2.5 | 15.11 |
| | 2 | 7.92 622 | 0.13 | Prolgolimab_VH_HC_hole_C-part, Anti-CSF1R_VH_b2m_Fc_knob_C-part(Fc fragment) | 5024 2.5 | 7.1 |
| | | | 0.87 | 11424 | 1142 4.5 | |
| | 3 | 11.5 402 | 1.00 | 37035 | 3703 5 | 4.02 |
| | 4 | 15.2 867 | 1.00 | 48459 | 4845 8.9 | 5.16 |
| | 5 | 15.4 857 | 1.00 | Anti-CSF1R_VH_b2m_Fc_knob_N-part, Anti-CSF1R_VL_CD1b(Fab-like fragment) | 4844 2.1 | 24.04 |
| | 6 | 17.0 937 | 1.00 | Prolgolimab_VH_HC_hole_N-part, Prolgolimab_VL_CK(Fab fragment) | 4790 0.5 | 10.97 |
| | 7 | 17.5 579 | 1.00 | Prolgolimab_VH_HC_hole_N-part, Prolgolimab_VL_CK(Fab fragment) | 4788 3 | 27.76 |
| | 8 | 18.4 697 | 1.00 | 47865.2 | 4786 5.2 | 5.84 |
| 02-007 GingisK HAN | 1 | 7.70 328 | 1.00 | Anti-CSF1R_VH_HC_hole_C-part, Prolgolimab_VH_b2m_Fc_knob_C-part (Fc fragment) | 5024 2 | 9.48 |
| | 2 | 7.93 537 | 1.00 | 11425 | 1142 4.5 | 6.03 |
| | 3 | 11.0 188 | 1.00 | 36062 | 3606 1.5 | 3.63 |
| | 4 | 15.6 825 | 0.38 | 47486 | 4748 5.9 | 6.14 |
| | | | 0.62 | 48891 | 4889 0.5 | |
| | 5 | 16.1 911 | 0.27 | 48873.9 | 4887 3.9 | 33.27 |
| | | | 0.73 | Anti-CSF1R_VH_HC_hole_N-part, Anti-CSF1R_VL_CK(Fab fragment) | 4746 8.6 | |
| | 6 | 16.5 06 | 0.10 | Anti-CSF1R_VH_HC_hole_N-part, Anti-CSF1R_VL_CK(Fab fragment) | 4746 9.1 | 27.71 |
| | | | 0.90 | Prolgolimab_VL_CD1b, Prolgolimab_VH_b2m_Fc_knob_N-part(Fab-like fragment) | 4885 6.5 | |
| | 7 | 16.9 205 | 0.12 | 47595.5 | 4759 5.5 | 13.74 |
| | | | 0.38 | Prolgolimab_VL_CD1b, Prolgolimab_VH_b2m_Fc_knob_N-par(Fab-like fragment) | 4885 7.2 | |
| | | | 0.50 | 47451 | 4745 0.7 | |
| 02-008 GingisK HAN | 1 | 7.66 267 | 1.00 | Ocrelizumab_VH_HC_hole_C-part, Anti-CSF1R_VH_b2m_Fc_knob_C-part(Fc fragment) | 5024 2.1 | 20.18 |
| | 2 | 7.92 792 | 0.32 | Ocrelizumab_VH_HC_hole_C-part , Anti-CSF1R_VH_b2m_Fc_knob_C-part(Fc fragment) | 5024 2.6 | 4.85 |
| | | | 0.68 | 11425 | 1142 4.5 | |
| | 3 | 11.6 082 | 1.00 | 37035 | 3703 5 | 1.25 |
| | 4 | 13.1 002 | 1.00 | Ocrelizumab_VH_HC_hole_N-part, Ocrelizumab_VL_CK(Fab fragment) | 4762 6 | 5.25 |
| | 5 | 13.6 472 | 1.00 | Ocrelizumab_VH_HC_hole_N-part, Ocrelizumab_VL_CK(Fab fragment) | 4762 5 | 35.7 |
| | 6 | 14.5 756 | 1.00 | 47607 | 4760 7 | 4.54 |
| | 7 | 15.2 719 | 0.13 | Ocrelizumab_VH_HC_hole_N-part, Ocrelizumab_VL_CK(Fab fragment) | 4762 5.6 | 4.6 |
| | | | 0.87 | 48458.9 (non-pyro form from mass 48442) | 4845 8.9 | |
| | 8 | 15,4 873 | 1.00 | Anti-CSF1R_VH_b2m_Fc_knob_N-part, Anti-CSF1R_VL_CD1b(Fab-like fragment) | 4844 2.1 | 22.27 |
| | 9 | 16.9 462 | 0.09 | Anti-CSF1R_VH_b2m_Fc_knob_N-part, Anti-CSF1R_VL_CD1b(Fab-like fragment) | 4844 3 | 1.37 |
| | | | 0.91 | Ocrelizumab_VH_HC_hole_N-part, Ocrelizumab_VL_CK(Fab fragment) | 4762 6.6 | |
| 02-009 GingisK han | 1 | 7.90 917 | | Anti-CSF1R_VH_HC_hole-Cpart, Ocrelizumab_VH_b2m_Fc_knob-Cpart(Fc fragment) | 5024 2 | 16.75 |
| | 2 | 8.37 167 | | 11425 | 1142 4.6 | 10.79 |
| | 3 | 11.5 2 | | 36079 | 3607 8.6 | 1.12 |
| | 4 | 11.9 383 | | 36062 | 3606 1.6 | 7.69 |
| | 5 | 13.2 667 | | 36043 | 3604 3.5 | 0.9 |
| | 6 | 14.2 3 | | non-pyro form from mass 48600 | 4861 6.1 | 1.52 |
| | | | | 48655 | 4865 5.1 | |
| | 7 | 14.5 133 | | Ocrelizumab_VH_b2m_Fc_knob-Npart, Ocrelizumab_VL_CD1b(Fab-like fragment) | 4860 0 | 3.73 |
| | 8 | 14.8 117 | | non-pyro form from mass 48600 | 4861 5.9 | 4.26 |
| | 9 | 15.0 45 | | Ocrelizumab_VH_b2m_Fc_knob-Npart, Ocrelizumab_VL_CD1b(Fab-like fragment) | 4859 8.4 | 31.1 |
| | 10 | 15.5 35 | | Ocrelizumab_VH_b2m_Fc_knob-Npart, Ocrelizumab_VL_CD1b(Fab-like fragment) | 4859 9.1 | 5.94 |
| | | | | 48470 | 4847 0 | |
| | 11 | 16.2 95 | | Anti-CSF1R_VL_CK,Anti-CSF1R_VH_HC_hole-Npart, Anti-CSF1R_VH_HC_hole-Cpart, Ocrelizumab_VH_b2m_Fc_knob-Cpart(antibody with a single Fab fragment, ineffective proteolysis) | 9781 1.8 | 4.23 |
| | | | | 36062 | 3606 2 | |
| | | | | 97693 | 9769 2.9 | |
| | | | | Ocrelizumab_VH_b2m_Fc_knob-Npart, Ocrelizumab_VL_CD1b(Fab-like fragment) | 4859 8.9 | |
| | 12 | 17.5 6 | | Anti-CSF1R_VH_HC_hole-Npart, Anti-CSF1R_VL_CK(Fab fragment) | 4746 8.9 | 9.99 |
| | 13 | 18.5 283 | | 47451 | 4745 1 | 1.96 |

The masses 97811.8 Da and 97693 Da in the case of sample 02-009 correspond to a molecule with one truncated Fab-like fragment.

The results show that all the bispecific chimeric antibodies according to the invention broke down following proteolysis into fragments corresponding in mass to an Fc fragment, Fab fragment and Fab-like fragment comprising β2 microglobulin and the α3 domain of the CDlb protein substituting the CK and CH1 domains, indicating that the technological solution based on the substitution of the pair of constant domains of CH1-CK for the pair of membrane-proximal domains from MHC or MHC-like proteins provides for the correct assembly of the bispecific chimeric antibodies according to the invention.

### Example 12. Verification of effect of amino acid substitutions in dimerization unit based on the membrane-proximal domains of MHC-like protein on the thermal stability of chimeric antibodies.

We further tested various substitutions in the dimerization unit and the effect thereof on the purity and stability of the resulting chimeric antibodies. The testing was conducted for the monospecific format is an antibody consisting of two heavy and two light chains, wherein the heavy chain CH1 and the light chain CK domains have been substituted for β2 microglobulin and the α3 domain of the CDlb protein, respectively (forward orientation of the dimerization unit). The sequences of the VH and VL variable domains were obtained from the antibody Prolgolimab (CJSC Biocad). Mutations indicated in Table 13 were introduced into the sequences of β2 microglobulin and the α3 domain of the CDlb protein. The sequences of the α3 domain of the CDlb protein further comprises C-terminal elongations indicated in Table 13. Table 13 shows the numbering of the positions where mutations were introduced from the beginning of the dimerization unit, and further shows the correspondence of the positions numbered according to the EU numbering of antibody chain amino acids (Edelman G.M. et al., Proc. Natl. Acad. Sci. USA 63 (1969), pp. 78-85; Kabat, E.A., et al., Sequences of Proteins of Immunological Interest, 5th ed., Public Health Service, National Institutes of Health, Bethesda, MD, (1991). Below shown is the numbering of the positions where substitutions were introduced, from the beginning of the dimerization unit; the position of substitutions in the hinge region is according to the EU numbering.

**Table 13. Description of mutant variants of dimerization unit based on β2 microglobulin and the α3 domain of CDlb protein.**

| Antibody | Heavy chain mutations (β2 microglobulin).Numbering from unit beginning | Heavy chain mutations EU numbering | Mutations and C-terminal elongation in light chain (CD1b) | Light chain mutations EU numbering | Notes |
|---|---|---|---|---|---|
| 03-001 | R12C | R129C | N57C and GSC elongation at C-terminus | N164C and GSC elongation at C-terminus | molecule with 2 S-S bridges in dimerization unit |
| 03-002 | R12C | R129C, C220A | N57C and elongation GS at the C-terminus | N164C and elongation GS at the C-terminus | molecule with 1 S-S bridge within dimerization unit |
| 03-003 | | | N59A and elongation GSC at the C-terminus | N166A and elongation GSC at the C-terminus | Molecule with removed potential glycosylation site in light chain |
| 03-004 | | | N59D and elongation GSC at the C-terminus | N166D and elongation GSC at the C-terminus | Molecule with removed potential glycosylation site in light chain |
| 03-005 | R12C | R129C | N57C, N59A and elongation GSC at the C-terminus | N164C, N166A and elongation GSC at the C-terminus | a molecule with 2 S-S bridges in dimerization unit and removed potential glycosylation site in light chain |
| 03-006 | R12C | R129C | N57C, N59D and elongation GSC at the C-terminus | N164C, N166D and elongation GSC at the C-terminus | a molecule with 2 S-S bridges in dimerization unit and removed potential glycosylation site in light chain |
| 03-007 | R12C | R129C, C220A | N57C, N59A and elongation GS at the C-terminus | N164C, N166A and elongation GS at the C-terminus | a molecule with 1 S-S bridge inside dimerization unit and removed potential glycosylation site in light chain |
| 03-008 | R12C | R129C, C220A | N57C, N59D and elongation GS at the C-terminus | N164C, N166D and elongation GS at the C-terminus | a molecule with 1 S-S bridge inside dimerization unit and removed potential glycosylation site in light chain |

Fig. 10 shows the result of non-reducing SDS gel electrophoresis of the generated antibodies. Samples of Prolgolimab (classical IgG1) and 01-011 (sample with an initial dimerization unit based on the membrane-proximal domains of MHC-like protein, see Example 5) were applied as controls onto lanes 1 and 2 (Fig. 10A and 10B), respectively. The results show that the mobility of the major fragment of all the modified samples corresponds to a full-length molecule (see Fig. 10 A lanes 3-8, Fig. 10 B lanes 3 - 4), thus indicating that the modifications that have been introduced do not effect the assembly of a full-length molecule. SE HPLC (Table 14) shows that some modifications lead to decreased monomer content and increased number of low-molecular fragments (antibodies 03-001, 03-003, 03-005, 03-006, 03-007); some of them however demonstrated increased monomer percentage (03-002, 03-004, 03-008). Further, we analyzed the aggregation temperature by dynamic light scattering of the resulting antibodies; the results are also shown in Table 14.

**Table 14. Productivity, purity and aggregation temperature of monospecific chimeric antibodies comprising modified dimerization units based on membrane-proximal domains of MHC-like proteins.**

| Antibody | Productivity, day 9, mg/l | Purity by SE HPLC | | | Aggregation temperature, °C |
|---|---|---|---|---|---|
| | | Aggregates, % | Monomer, % | Fragments, % | |
| Prolgolimab | 252.4 | 3.5 | 94.7 | 1.8 | 66.34 |
| 01-011 | 302.6 | 1.7 | 80.6 | 17.7 | 55.23 |
| 03-001 | 192.7 | 4.2 | 61.5 | 34.3 | 59.09 |
| 03-002 | 310.8 | 3.9 | 82.9 | 13.1 | 58.16 |
| 03-003 | 229.6 | 3.3 | 62.2 | 34.4 | 42.14 |
| 03-004 | 189.9 | 5.0 | 84.3 | 10.8 | 61.13 |
| 03-005 | 175.0 | 2.7 | 69.7 | 27.6 | 58.66 |
| 03-006 | 182.2 | 2.0 | 71.7 | 26.3 | 58.91 |
| 03-007 | 297.5 | 5.8 | 78.4 | 15.9 | 52.07 |
| 03-008 | 199.8 | 3.4 | 88.6 | 8.0 | 58.07 |

The results show that the antibody variants that demonstrated increased relative monomer content according to SE HPLC further demonstrated an increased aggregation temperature as compared to a non-modified variant of the dimerization unit based on MHC-like proteins.

## Claims

1. A bivalent bispecific chimeric antibody, wherein said antibody comprises:
a) a first light chain and a first heavy chain of antibody specifically binding to a first antigen;
wherein the first light chain comprises a light chain variable domain and a light chain constant domain;
wherein the first heavy chain comprises a heavy chain variable domain and heavy chain constant domains of antibody that include a first (CH1) heavy chain constant domain and an Fc fragment monomer comprising second (CH2) and third (CH3) heavy chain constant domains;
b) a second light chain and a second heavy chain of antibody specifically binding to a second antigen,
wherein the second light chain comprises a light chain variable domain and a constant domain that is selected from the group:
a first membrane-proximal domain of MHC (major histocompatibility complex) or
a first membrane-proximal domain of MHC-like protein;
wherein the second heavy chain comprises a heavy chain variable domain, a constant domain that is selected from the group:
a second membrane-proximal domain of MHC (major histocompatibility complex) or
a second membrane-proximal domain of MHC-like protein;
and an Fc fragment monomer comprising second (CH2) and third (CH3) heavy chain constant domains;
wherein the first membrane-proximal domain of MHC or MHC-like protein and the second membrane-proximal domain of MHC or MHC-like protein form a heterodimer therebetween, which is stabilized by a disulfide bond;
wherein the CH3 domain of one heavy chain and the CH3 domain of another heavy chain contact each other with surfaces thereof, which are modified to form a bivalent bispecific chimeric antibody, said modifications in the heavy chain CH3 domains being substitutions to facilitate heterodimerization.

2. The bivalent bispecific chimeric antibody according to claim 1, wherein the first membrane-proximal domain of MHC may be selected from a group including:
a first membrane-proximal domain of MHC class I (major histocompatibility complex class I),
a first membrane-proximal domain of MHC class II (major histocompatibility complex class II),
a modified variant of a first membrane-proximal domain of MHC class I or
a modified variant of a first membrane-proximal domain of MHC class II.

3. The bivalent bispecific chimeric antibody according to claim 1, wherein the second membrane-proximal domain of MHC may be selected from a group including:
a second membrane-proximal domain of MHC class I (major histocompatibility complex class I),
a second membrane-proximal domain of MHC class II (major histocompatibility complex class II),
a modified variant of a second membrane-proximal domain of MHC class I or
a modified variant of a second membrane-proximal domain of MHC class II.

4. The bivalent bispecific chimeric antibody according to any one of claims 2 to 3, wherein the MHC class II is selected from the group: HLA-DM, HLA-DO, HLA-DP, HLA-DQ or HLA-DR.

5. The bivalent bispecific chimeric antibody according to any one of claims 2 to 3, wherein the MHC class I is selected from the group: HLA-A, HLA-B, HLA-C, HLA-E, HLA-F or HLA-G.

6. The bivalent bispecific chimeric antibody according to claim 1, wherein the first membrane-proximal domain of MHC-like protein may be selected from a group including:
a first membrane-proximal domain of CD1 (cluster of differentiation 1),
a first membrane-proximal domain of HFE (hemochromatosis protein),
a modified variant of a first membrane-proximal domain of CD1 or
a modified variant of a first membrane-proximal domain of HFE.

7. The bivalent bispecific chimeric antibody according to claim 1, wherein the second membrane-proximal domain of MHC-like protein may be selected from a group including:
a second membrane-proximal domain of CD1 (cluster of differentiation 1),
a second membrane-proximal domain of HFE (hemochromatosis protein),
a modified variant of a second membrane-proximal domain of CD1 or
a modified variant of a second membrane-proximal domain of HFE.

8. The bivalent bispecific chimeric antibody according to any one of claims 6 to 7, wherein the CD1 is selected from the group: CD1a, CD1b, CD1c, CD1d or CD1e.

9. The bivalent bispecific chimeric antibody according to claim 1, wherein the variable fragment of the second light chain (VL) is separated from the first membrane-proximal domain of MHC or MHC-like protein by a linker of 1 to 25 amino acids long and/or the variable fragment of the second heavy chain (VH) is separated from the second membrane-proximal domain of MHC or MHC-like protein by a linker of 1 to 25 amino acids long.

10. The bivalent bispecific chimeric antibody according to claim 1, wherein
a) the CH3 domain of one heavy chain is modified so that on the surface of the CH3 domain of one heavy chain contacting the surface of the CH3 domain of another heavy chain in the bivalent bispecific antibody, the amino acid residue is substituted for an amino acid residue that has a larger side chain volume, leading to formation of a knob on the surface of the CH3 domain of one heavy chain that can fit into a hole on the surface of the CH3 domain of another heavy chain,
and
b) the CH3 domain of another heavy chain is modified so that on the surface of the CH3 domain of the second heavy chain contacting the surface of the CH3 domain of the first heavy chain in the bivalent bispecific antibody, the amino acid residue is substituted for an amino acid residue that has a smaller side chain volume, leading to formation of a hole on the surface of the CH3 domain of the second heavy chain that can accept a knob on the interface of the CH3 domain of the first heavy chain;
wherein said amino acid residue that has a larger side chain volume is selected from a group including arginine (R), phenylalanine (F), tyrosine (Y), tryptophan (W),
and wherein said amino acid residue that has a smaller side chain volume is selected from a group including alanine (A), serine (S), threonine (T), valine (V).

11. The bivalent bispecific chimeric antibody according to claim 1, wherein the constant domain of the first light chain of antibody is selected from CK or CL.

12. The bivalent bispecific chimeric antibody according to claim 1, wherein the CH3 domains of antibody are further modified by introduction of cysteine (C) as an amino acid into the corresponding positions of each CH3 domain so that a disulfide bridge may form between the both CH3 domains.

13. The bivalent bispecific chimeric antibody according to claim 10, wherein the CH3 domain of one heavy chain is modified to form Knob, and the CH3 domain of another heavy chain is modified to form Hole, or vice versa.

14. The bivalent bispecific chimeric antibody according to claim 13, wherein the CH3 domain of one heavy chain has amino acid substitutions S354C/T366W, and the CH3 domain of another heavy chain has amino acid substitutions Y349C/T366S/L368A/Y407V; or
the CH3 domain of one heavy chain has amino acid substitutions Y349C/T366S/L368A/Y407, and the CH3 domain of another heavy chain has amino acid substitutions S354C/T366W.

15. The bivalent bispecific chimeric antibody according to claim 1, wherein the first membrane-proximal domain of MHC and the second membrane-proximal domain of MHC are the α2 domain of MHC II and the β2 domain of MHC II, respectively, which form a heterodimer therebetween.

16. The bivalent bispecific chimeric antibody according to claim 15, wherein the α2 domain of MHC II has an amino acid sequence that is selected from the group: SEQ ID NO: 30, SEQ ID NO: 32, SEQ ID NO: 34, SEQ ID NO: 36 or SEQ ID NO: 38, and the β2 domain of MHC II has the amino acid sequence that is selected from the group: SEQ ID NO: 31, SEQ ID NO: 33, SEQ ID NO: 35, SEQ ID NO: 37 or SEQ ID NO: 39.

17. The bivalent bispecific chimeric antibody according to claim 1, wherein the first membrane-proximal domain of MHC and the second membrane-proximal domain of MHC are the β2 domain of MHC II and the α2 domain of MHC II, respectively, which form a heterodimer therebetween.

18. The bivalent bispecific chimeric antibody according to claim 17, wherein the β2 domain of MHC II has an amino acid sequence that is selected from the group: SEQ ID NO: 31, SEQ ID NO: 33, SEQ ID NO: 35, SEQ ID NO: 37 or SEQ ID NO: 39, and the α2 domain of MHC II has the amino acid sequence that is selected from the group: SEQ ID NO: 30, SEQ ID NO: 32, SEQ ID NO: 34, SEQ ID NO: 36 or SEQ ID NO: 38.

19. The bivalent bispecific chimeric antibody according to claim 1, wherein the first membrane-proximal domain of MHC and the second membrane-proximal domain of MHC are a modified variant of the α2 domain of MHC II and a modified variant of the β2 domain of MHC II, respectively, which form a heterodimer therebetween; or
wherein the first membrane-proximal domain of MHC and the second membrane-proximal domain of MHC are a modified variant of the β2 domain of MHC II and a modified variant of the α2 domain of MHC II, respectively, which form a heterodimer therebetween; or
wherein the first membrane-proximal domain of MHC and the second membrane-proximal domain of MHC are the α3 domain of MHC I and β2 microglobulin (β2M), respectively, which form a heterodimer therebetween.

20. The bivalent bispecific chimeric antibody according to claim 19, wherein the α3 domain of MHC I has an amino acid sequence selected from the group: SEQ ID NO: 1-29, and β2 microglobulin (β2M) has the amino acid sequence of SEQ ID NO: 46.

21. The bivalent bispecific chimeric antibody according to claim 1, wherein the first membrane-proximal domain of MHC and the second membrane-proximal domain of MHC are β2 microglobulin (β2M) and the α3 domain of MHC I, respectively, which form a heterodimer therebetween.

22. The bivalent bispecific chimeric antibody according to claim 21, wherein the β2 microglobulin (β2M) has the amino acid sequence of SEQ ID NO: 46, and the α3 domain of MHC I has an amino acid sequence selected from the group: SEQ ID NO: 1-29.

23. The bivalent bispecific chimeric antibody according to claim 1, wherein the first membrane-proximal domain of MHC and the second membrane-proximal domain of MHC are a modified variant of the α3 domain of MHC I and a modified variant of β2 microglobulin (β2M), respectively, which form a heterodimer therebetween; or
wherein the first membrane-proximal domain of MHC and the second membrane-proximal domain of MHC are a modified variant of β2 microglobulin (β2M) and a modified variant of the α3 domain of MHC I, respectively, which form a heterodimer therebetween.

24. The bivalent bispecific chimeric antibody according to claim 23, wherein the modified variant of β2 microglobulin (β2M) has an amino acid sequence selected from SEQ ID NO: 47 or SEQ ID NO: 48.

25. The bivalent bispecific chimeric antibody according to claim 1, wherein the first membrane-proximal domain of MHC-like protein and the second membrane-proximal domain of MHC-like protein are the α3 domain of CD1 and β2 microglobulin (β2M), respectively, which form a heterodimer therebetween.

26. The bivalent bispecific chimeric antibody according to claim 25, wherein the α3 domain of CD1 has an amino acid sequence selected from the group: SEQ ID NO: 40-44, and the β2 microglobulin (β2M) has the amino acid sequence of SEQ ID NO: 46.

27. The bivalent bispecific chimeric antibody according to claim 1, wherein the first membrane-proximal domain of MHC-like protein and the second membrane-proximal domain of MHC-like protein are β2 microglobulin (β2M) and the α3 domain of CD1, respectively, which form a heterodimer therebetween.

28. The bivalent bispecific chimeric antibody according to claim 27, wherein the β2 microglobulin (β2M) has the amino acid sequence of SEQ ID NO: 46, and the α3 domain of CD1 has an amino acid sequence selected from the group: SEQ ID NO: 40-44.

29. The bivalent bispecific chimeric antibody according to claim 1, wherein the first membrane-proximal domain of MHC-like protein and the second membrane-proximal domain of MHC-like protein are a modified variant of the α3 domain of CD1 and a modified variant of β2 microglobulin (β2M), respectively, which form a heterodimer therebetween.

30. The bivalent bispecific chimeric antibody according to claim 29, wherein the modified variant of the α3 domain of CD1 has an amino acid sequence selected from the group: SEQ ID NO: 49-56, and the modified variant of β2 microglobulin (β2M) has an amino acid sequence selected from SEQ ID NO: 47 or SEQ ID NO: 48.

31. The bivalent bispecific chimeric antibody according to claim 1, wherein the first membrane-proximal domain of MHC-like protein and the second membrane-proximal domain of MHC-like protein are a modified variant of β2 microglobulin (β2M) and a modified variant of the α3 domain of CD1, respectively, which form a heterodimer therebetween.

32. The bivalent bispecific chimeric antibody according to claim 31, wherein the modified variant of β2 microglobulin (β2M) has an amino acid sequence selected from SEQ ID NO: 47 or SEQ ID NO: 48, and the modified variant of the α3 domain of CD1 has an amino acid sequence selected from the group: SEQ ID NO: 49-56 or SEQ ID NO: 109.

33. The bivalent bispecific chimeric antibody according to claim 1, wherein the first membrane-proximal domain of MHC-like protein and the second membrane-proximal domain of MHC-like protein are the α3 domain of HFE and β2 microglobulin (β2M), respectively, which form a heterodimer therebetween.

34. The bivalent bispecific chimeric antibody according to claim 33, wherein the α3 domain of HFE has the amino acid sequence of SEQ ID NO: 45, and the β2 microglobulin (β2M) has the amino acid sequence of SEQ ID NO: 46.

35. The bivalent bispecific chimeric antibody according to claim 1, wherein the first membrane-proximal domain of MHC-like protein and the second membrane-proximal domain of MHC-like protein are β2 microglobulin (β2M) and the α3 domain of HFE, respectively, which form a heterodimer therebetween.

36. The bivalent bispecific chimeric antibody according to claim 35, wherein the β2 microglobulin (β2M) has the amino acid sequence of SEQ ID NO: 46, and the α3 domain of HFE has the amino acid sequence of SEQ ID NO: 45.

37. The bivalent bispecific chimeric antibody according to claim 1, wherein the first membrane-proximal domain of MHC-like protein and the second membrane-proximal domain of MHC-like protein are a modified variant of the α3 domain of HFE and a modified variant of β2 microglobulin (β2M), respectively, which form a heterodimer therebetween; or
wherein the first membrane-proximal domain of MHC-like protein and the second membrane-proximal domain of MHC-like protein are a modified variant of β2 microglobulin (β2M) and a modified variant of the α3 domain of HFE, respectively, which form a heterodimer therebetween.

38. The bivalent bispecific chimeric antibody according to claim 37, wherein the modified variant of β2 microglobulin (β2M) has an amino acid sequence selected from SEQ ID NO: 47 or SEQ ID NO: 48.

39. The bivalent bispecific chimeric antibody according to any one of claims 2 to 3, 6 to 7, 19, 23, 29, 31 and 37, wherein the modified variant refers to a variant that includes substitutions for cysteine (C) to form a disulfide bridge between the chains of heterodimer produced from the first and second membrane-proximal domains of MHC or MHC-like protein; or
wherein the modified variant refers to a variant including one or more substitutions at various positions of the membrane-proximal domains of MHC or MHC-like protein, resulting in increased thermodynamic stability Tm by more than 1 degree Celsius as compared to wild type membrane-proximal domains of MHC or MHC-like protein, respectively; or
wherein the modified variant refers to a variant including one or more substitutions at various positions of the membrane-proximal domains of MHC or MHC-like protein, leading to decreased amount of aggregates by more than 5% at concentration above 10 mg/ml as compared to wild type membrane-proximal domains of MHC or MHC-like protein, respectively; or
wherein the modified variant refers to a variant including one or more substitutions at various positions of the membrane-proximal domains of MHC or MHC-like protein, leading to removed glycosylation sites as compared to wild type membrane-proximal domains of MHC or MHC-like protein, respectively.

40. The bivalent bispecific chimeric antibody according to claim 1, wherein the variable domain of the first light chain and the variable domain of the second light chain are identical.

41. The bivalent bispecific chimeric antibody according to claim 1, wherein the Fc fragment belongs to IgG.

42. The bivalent bispecific chimeric antibody according to claim 41, wherein the Fc fragment isotype is selected from the group: human IgG1, IgG2, or IgG4.

43. The bivalent bispecific chimeric antibody according to claim 1, wherein substitutions are further introduced in the Fc fragment monomer, leading to absent ADCC, CDC and/or ADCP properties in the bivalent bispecific antibody.

44. The bivalent bispecific chimeric antibody according to claim 43, wherein LALA substitutions (L234A and L235A) are further introduced in the Fc fragment monomer.

45. The bivalent bispecific chimeric antibody according to claim 1, wherein substitutions leading to prolonged antibody activity are further introduced in the Fc fragment monomer.

46. The bivalent bispecific chimeric antibody according to claim 45, wherein YTE substitutions (M252Y, S254T and T256E) are further introduced in the Fc fragment monomer.

47. The bivalent bispecific chimeric antibody according to claim 43, wherein the substitution E345R is further introduced in the Fc fragment monomer.

48. The bivalent bispecific chimeric antibody according to claim 1, which specifically binds to CD20 and CD3; or
which specifically binds to BCMA and CD3; or
which specifically binds to PD-L1 and CD47; or
which specifically binds to coagulation factor 9 (FIX) and coagulation factor 10 (FX); or
which specifically binds to GD2 and CD3; or
which specifically binds to AXL and CD3; or
which specifically binds to PD-L1 and TGF beta.

49. The bivalent bispecific chimeric antibody according to claim 1, wherein the first membrane-proximal domain of MHC or MHC-like protein and the second membrane-proximal domain of MHC or MHC-like protein form a heterodimer therebetween, which is stabilized by a disulfide bond by means of a mutation or mutations in the first and/or second membrane-proximal domain to form an S-S bond (disulfide cysteine bridge) between the first and second membrane-proximal domains of MHC or MHC-like protein.

50. The bivalent bispecific chimeric antibody according to claim 1, wherein the first membrane-proximal domain of MHC or MHC-like protein and the second membrane-proximal domain of MHC or MHC-like protein form a heterodimer therebetween, which is stabilized by a disulfide bond by means of elongation of the first membrane-proximal domain of MHC or MHC-like protein by one or more (1 to 10) amino acids at the C-terminus and with terminal Cys at the C-terminus to form an S-S bond (disulfide bond, cysteine bridge) between the first membrane-proximal domain of MHC or MHC-like protein and the hinge.

51. The bivalent bispecific chimeric antibody according to claim 50, wherein the elongation of the first membrane-proximal domain of MHC or MHC-like protein is GSC.

52. An isolated nucleic acid, which encodes the bivalent bispecific chimeric antibody according to any one of claims 1 to 51.

53. The isolated nucleic acid according to claim 52, wherein the nucleic acid is DNA.

54. An expression vector comprising the nucleic acid according to any one of claims 52 to 53.

55. A method for producing a host cell for producing a bivalent bispecific chimeric antibody according to any one of claims 1 to 51, comprising transforming the cell with the vector according to claim 54.

56. A host cell for producing the bivalent bispecific chimeric antibody according to any one of claims 1 to 51, comprising the nucleic acid according to any one of claims 52 to 53.

57. A method for producing the bivalent bispecific chimeric antibody according to claims 1 to 51, wherein the method comprises the steps of:
a) transforming the host cell
- with expression vectors comprising nucleic acid molecules encoding the first light chain and the first heavy chain of the bispecific chimeric antibody,
- with expression vectors comprising nucleic acid molecules encoding the second light chain and the second heavy chain of the bispecific chimeric antibody,
b) culturing the host cell under conditions suitable for synthesis of said bivalent bispecific chimeric antibody; and
c) isolating said bivalent bispecific antibody from cell culture.
